# EUROPEAN PATENT SPECIFICATION

(11) **EP 3 160 500 B1**
(45) Date of publication and mention of the grant of the patent: **21.08.2019**
(21) Application number: 15733404.6
(22) Date of filing: 25.06.2015
(51) Int. Cl.: A61K 39/08, C07K 14/33

(54) **CLOSTRIDIUM DIFFICILE IMMUNOGENIC COMPOSITION**
CLOSTRIDIUM DIFFICILE IMMUNOGENE ZUSAMMENSETZUNG
COMPOSITION IMMUNOGÈNE CONTRE LE CLOSTRIDIUM DIFFICILE

(30) Priority: 25.06.2014 GB 201411306; 26.06.2014 GB 201411371
(43) Date of publication of application: 03.05.2017
(73) Proprietor: GlaxoSmithKline Biologicals S.A., 1330 Rixensart (BE)
(72) Inventor: CASTADO, Cindy, B-1330 Rixensart (BE)
(74) Representative: Johnston, Caroline Louise
(86) International application number: PCT/EP2015/064324
(87) International publication number: WO 2015/197737

(56) References cited:
- WO-A1-2013/112867
- WO-A1-2014/096393
- WO-A2-2005/004791
- T. NEUMEYER ET AL: "Clostridium botulinum C2 Toxin: IDENTIFICATION OF THE BINDING SITE FOR CHLOROQUINE AND RELATED COMPOUNDS AND INFLUENCE OF THE BINDING SITE ON PROPERTIES OF THE C2II CHANNEL", JOURNAL OF BIOLOGICAL CHEMISTRY, vol. 283, no. 7, 11 December 2007 (2007-12-11), pages 3904-3914, XP55207643, ISSN: 0021-9258, DOI: 10.1074/jbc.M709807200
- SUNDRIYAL A ET AL: "Expression, purification and cell cytotoxicity of actin-modifying binary toxin from Clostridium difficile", PROTEIN EXPRESSION AND PURIFICATION, ACADEMIC PRESS, SAN DIEGO, CA, vol. 74, no. 1, 1 November 2010 (2010-11-01), pages 42-48, XP027226634, ISSN: 1046-5928, DOI: 10.1016/J.PEP.2010.04.014 [retrieved on 2010-04-28] cited in the application
- KANOKPORN SRISUCHARITPANIT ET AL: "Expression and purification of the active soluble form of binary toxin for structural analysis", PROTEIN EXPRESSION AND PURIFICATION, vol. 82, no. 2, 20 February 2012 (2012-02-20), pages 368-372, XP028477061, ISSN: 1046-5928, DOI: 10.1016/J.PEP.2012.02.009 [retrieved on 2012-02-20]

## Description

### Background

*Clostridium difficile (C. difficile)* is the most important cause of nosocomial intestinal infections and is the major cause of pseudomembranous colitis in humans (Bartlett et al Am. J. Clin. Nutr. 11 suppl:2521-6 (1980)). The overall associated mortality rate for individuals infected with C. *difficile* was calculated to be 5.99% within 3 months of diagnosis, with higher mortality associated with advanced age, being 13.5% in patients over 80 years (Karas et al Journal of Infection 561:1-9 (2010)).The current treatment for *C*. *difficile* infection is the administration of antibiotics (metronidazole and vancomycin); however there has been evidence of strains which are resistant to these antibiotics (Shah et al., Expert Rev. Anti Infect. Ther. 8(5), 555-564 (2010)). Accordingly there is a need for immunogenic compositions capable of inducing antibodies to, and /or a protective immune response to, *C*. *difficile.*

The enterotoxicity of *C*. *difficile* is primarily due to the action of two toxins, toxin A and toxin B. These are both potent cytotoxins (Lyerly et al Current Microbiology 21:29-32 (1990).
It has been demonstrated that fragments of toxin A, in particular fragments of the C-terminal domain, can lead to a protective immune response in hamsters (Lyerly et al Current Microbiology 21:29-32 (1990)), WO96/12802 and WO00/61762.

Some strains, but not all, also express *Clostridium difficile* binary toxin (CDT). Similar to many other binary toxins, CDT is composed of two components - an enzymatically active component ("binary toxin A" or "CDTa") and a catalytically inert transport and binding component ("binary toxin B" or "CDTb"). The catalytically inert component facilitates translocation of the CDTa into the target cell.

CDTa has an ADP-ribosylating activity, which transfers the ADP-ribose moiety of NAD/NADPH to monomeric actin (G-actin) in the target cell and thus preventing its polymerization to F-actin and resulting in disruption of the cytoskeleton and eventual cell death (Sundriyal et al, Protein expression and Purification 74 (2010) 42-48).

WO2013/112867 (Merck) describes vaccines against *Clostridium difficile* comprising recombinant *C*. *difficile* Toxin A, Toxin B and CDTa proteins, all comprising specifically defined mutations relative to the native toxin sequences that are described as substantially reducing or eliminating toxicity, in combination with binary toxin B (CDTb).

WO2014/096393 describes immunogenic compositions comprising CDTb. WO2005/004791 A2 describes the pore forming binary toxin B of *B.anthracis.* Neumeyer et al (2007) Journal of Biological Chemistry vol.283, No.7, pg 3904-3914 describes the identification of the binding site for chloroquine and related compounds and influence of the binding site on properties of the C2II channel.

The present inventors have found that CDTb proteins comprising mutations designed to modify the pore forming ability of CDTb or modify the heptamerisation ability of CDTb have improved characteristics.

### Summary of Invention

In a fourth aspect of the invention there is provided an immunogenic composition comprising a fusion protein comprising (i) a full length CDTa protein and (ii) a CDTb protein wherein the CDTb protein is a full length CDTb protein with the prodomain removed.

In a first aspect of the disclosure there is provided an immunogenic composition comprising an isolated *Clostridium difficile* CDTb protein wherein the isolated *Clostridium difficile* CDTb protein has been mutated to modify pore forming ability.

In a second aspect of the disclosure there is provided an immunogenic composition comprising an isolated *Clostridium difficile* CDTb protein which is a truncated CDTb protein with the signal peptide and the prodomain removed and also the receptor binding domain removed and/or the CDTa binding domain removed.

In a third aspect of the disclosure there is provided an immunogenic composition comprising an isolated *Clostridium difficile* CDTb protein wherein the isolated *Clostridium difficile* CDTb protein has been mutated to modify heptamerisation ability.

In a fifth aspect the present invention provides a vaccine comprising the immunogenic composition of the fourth aspect and a pharmaceutically acceptable excipient.

In a sixth aspect the present invention provides the immunogenic composition of the fourth aspect or the vaccine of the fifth aspect, for use in the treatment or prevention of disease e.g. C. *difficile* disease.

In a seventh aspect the present invention provides the use of an immunogenic composition of the fourth aspect or the vaccine of the fifth aspect in the preparation of a medicament for the prevention or treatment of disease e.g. C. *difficile* disease.

In an eighth aspect the present disclosure provides a method of preventing or treating C. *difficile* disease comprising administering an immunogenic composition of any one of the first four aspects or the vaccine of the fifth aspect to a mammalian subject.

In a ninth aspect the present disclosure provides novel polypeptides and nucleotides as defined herein.

In a further aspect, the present disclosure provides a method for the treatment or prevention of a condition or disease caused wholly or in part by C. *difficile.* The method comprises administering to a subject in need thereof a therapeutically effective amount of the proteins as described herein, the immunogenic composition of the invention or the vaccines of the invention.

### BRIEF DESCRIPTION OF THE DRAWINGS

Figure 1 - Cytotoxicity of CDTb candidates alone on HT29 cells: data from Example 7. C37, C123, C124, C126 and C128 referred to in Figure 1 are the CDTb proteins as described in Examples 2, 4 and 6, the sequences of which are set out in the sequence summary (Table A).
Figure 2 - Cytotoxicity of CDTb candidates mixed with CDTa full on HT29 cells: data from Example 7. C37, C123, C124, C126 and C128 referred to in Figure 1 are the CDTb proteins as described in Examples 2, 4 and 6, the sequences of which are set out in the sequence summary (Table A). "CDTa full" is the CDTa protein C34 the sequence of which is set out in the sequence summary (Table A).
Figure 3: Dynamic light scattering measurements of hydrodynamic radius for KO pore forming CDTb constructs C123, C126 and of KO heptamerisation CDTb construct C128. Average radius gives an indication of the homogeneity of the sample, while peak 1 radius is an estimation of the hydrodynamic radius of the protein of interest.
Figure 4: Graph showing anti-CDTb immunogenicity in mice immunised with C. *difficile* CDTa or C. *difficile* CDTb, in both cases formulated with adjuvant
Figure 5: Graph showing anti-CDTa immunogenicity in mice immunised with C. *difficile* CDTa or C. *difficile* CDTb, in both cases formulated with adjuvant
Figure 6: Cytotoxicity inhibition titres in HCT116 cells from mice immunised with C. *difficile* CDTa or C. *difficile* CDTb, in both cases formulated with adjuvant
Figure 7: Cytotoxicity inhibition titres in HT29 cells from mice immunised with C. *difficile* CDTa or C. *difficile* CDTb, in both cases formulated with adjuvant
Figure 8: Cytotoxicity of CDTb candidates alone (Figure 8a) or mixed with CDTa full (Figure 8b) on HT29 cells: data from Example 7. C37, C123, C124, C126 and C128 are the CDTb proteins as described in Examples 2, 4 and 6, the sequences of which are set out in the sequence summary (Table A). "CDTa full" is the CDTa protein C34 the sequence of which is set out in the sequence summary (Table A). C149, C152, C164, C166, C116, C117 are the CDTb proteins as described in Examples 4 and 5.
Figure 9: Cytotoxicity of CDTb candidates alone (Figure 9a) or mixed with CDTa full (Figure 9b) on HCT116 cells (as described in Example 12)
Figure 10: Cytotoxicity of CDTa-CDTb fusion proteins mixed with full activated CDTa on HT29 cells (as described in Example 13)
Figure 11 Cytotoxicity of CDTa-CDTb fusion proteins mixed with full CDTb on HT29 cells (as described in Example 13)
Figure 12: Graph showing anti-CDTa immunogenicity in mice immunised with CDTb proteins or CDTa-CDTb fusions (see Example 14)
Figure 13: Graph showing anti-CDTb immunogenicity in mice immunised with CDTb proteins or CDTa-CDTb fusions (see Example 14)
Figure 14: Cytotoxicity inhibition titres in HT29 and HCT116 cells, mice immunised with CDTb proteins or CDTa-CDTb fusions (see Example 15)

### Detailed Description

The *Clostridium difficile* binary toxin comprises two different proteins, CDTa and CDTb. CDTa comprises two domains, the C-terminal domain is responsible for the ADP ribosyltransferase activity whilst the N-terminal domain is responsible for interacting with CDTb.

During infection CDTb is activated by proteolytic cleavage by a chymotrypsin-like protease to produce a CDTb protein lacking the prodomain (referred to as CDTb"). Note that CDTb" also lacks the CDTb signal sequence. A CDTb protein lacking the signal sequence but not lacking the prodomain is referred to as CDTb'. After proteolytic activation the CDTb oligomerises and binds to CDTa to form the complete C. *difficile* binary toxin (CDT). The binding of the binary toxin to the cell receptors leads to receptor-mediated endocytosis. As the endosome acidifies the CDTb binding domain undergoes conformational changes that allow the CDTb oligomer to form a pore, the pore formation triggers translocation of the ADP-ribosyltransferase domain (CDTa) into the target cell.

In one aspect the present disclosure provides an immunogenic composition comprising an isolated *Clostridium difficile* CDTb protein which has been mutated to modify pore forming ability.
The term "CDTb protein which has been mutated to modify pore forming ability" refers to a CDTb protein which has been mutated to modify the ability of the CDTb protein to form a pore.

In one embodiment of the disclosure, the isolated *Clostridium difficile* CDTb protein has been mutated to reduce pore forming ability.

Pore forming ability may be analysed according to the methods described in The J. of Biol. Chem. 2001, vol. 276 : 8371-8376, PNAS 2004, vol. 101 : 16756-16761, or The J. of Biol. Chem. 2008, vol. 283 : 3904-3914

In this aspect the isolated *Clostridium difficile* CDTb protein may have been mutated to avoid the formation of the beta barrel structure which is composed of beta strands shared by each CDTb monomer contained in the heptamer which forms upon oligomerisation of CDTb. In this aspect the CDTb protein may be a truncated CDTb protein with the signal peptide removed. In this aspect the CDTb protein may be a truncated CDTb protein with the signal peptide removed and the prodomain removed.

The term "truncated CDTb protein with the signal peptide removed" refers to a CDTb protein with substantially all of the signal peptide removed (therefore which does not comprise amino acids corresponding to substantially all of the signal peptide. For example, the term "truncated CDTb protein with the signal peptide removed" refers to a CDTb protein with at least 25 of the amino acids of the signal peptide removed. There may be a few amino acids of the signal peptide remaining. For example 2, 5, 10, 15 or 20 amino acids of the signal peptide may remain.

The term "truncated CDTb protein with the signal peptide removed.and the prodomain removed" refers to a CDTb protein with substantially all of the signal peptide and the prodomain removed (therefore which does not comprise amino acids corresponding to substantially all of the signal peptide and substantially all of the prodomain). For example, the term "truncated CDTb protein with the signal peptide removed.and the prodomain removed" refers to a CDTb protein with all of the signal peptide removed and at least 85 of the amino acids of the prodomain removed.There may be a few amino acids of the signal peptide and/or prodomain remaining. For example 2, 5, 10, 15 or 20 amino acids of the signal peptide may remain. For example 2, 5, 10, 15 or 20 amino acids of the prodomain may remain.The signal peptide of CDTb corresponds to amino acids 1-48 (encompassing amino acids 1-42) of SEQ ID NO: 3 or their equivalents in a binary toxin protein isolated from a different strain of C. *difficile,* for example amino acids 1-42 of the amino acid sequence of CDTb from strain CD196 (Perelle, M. et al Infect. Immun., 65 (1997), pp. 1402-1407). The prodomain of CDTb corresponds to amino acids 48-211 (encompassing amino acids 48-166) of SEQ ID NO:3 or their equivalents in a binary toxin protein isolated from a different strain of C. *difficile.*

In one embodiment of the disclosure, the isolated *Clostridium difficile* CDTb protein comprises a mutation at position 455 of SEQ ID NO: 3 or its equivalent in a different strain of C. *difficile.* In another embodiment of this aspect, the isolated *Clostridium difficile* CDTb protein comprises a mutation at position 426 of SEQ ID NO: 3 or its equivalent in a different strain of C. *difficile.* In another embodiment of this aspect, the isolated *Clostridium difficile* CDTb protein comprises a mutation at position 453 of SEQ ID NO: 3 or its equivalent in a different strain of C. *difficile.* In another embodiment of this aspect, the isolated *Clostridium difficile* CDTb protein comprises a mutation at position 426 and a mutation at position 453 of SEQ ID NO:3 or its equivalent in a different strain of C. *difficile.*

In one embodiment of the disclosure, the isolated *Clostridium difficile* CDTb protein comprises at least one mutation selected from the group consisting of F455R, F455G, E426A and D453A. "F455R" means that the phenylalanine (F) at position 455 of the CDTb sequence in SEQ ID NO: 3 is mutated to arginine (R). "F455G" means that the phenylalanine (F) at position 455 of the CDTb sequence in SEQ ID NO: 3 is mutated to glycine (G). "E426A" means that the glutamate (E) at position 426 of the CDTb sequence in SEQ ID NO: 3 is mutated to alanine (A). "D453A" means that the aspartate (D) at position 453 of the CDTb sequence in SEQ ID NO: 3 is mutated to alanine (A). In one embodiment of this aspect, the isolated *Clostridium difficile* CDTb protein comprises the mutation F455R or its equivalent in a different strain of C. *difficile.* In one embodiment of this aspect, the isolated *Clostridium difficile* CDTb protein comprises the mutation F455G or its equivalent in a different strain of *C*. *difficile.* In one embodiment of this aspect, the isolated *Clostridium difficile* CDTb protein comprises the mutations E426A and D453A or their equivalents in a different strain of *C*. *difficile.*

In one embodiment of the disclosure the isolated *Clostridium difficile* CDTb protein is or comprises
(i) SEQ ID NO: 10 or SEQ ID NO: 11 or SEQ ID NO: 25 or SEQ ID NO: 26; or
(ii) a variant of CDTb having at least 80%, 85%, 88%, 90%, 92%, 95%, 98%, 99%, 100% sequence identity to SEQ ID NO: 10 or SEQ ID NO: 11 or SEQ ID NO: 25 or SEQ ID NO: 26; or
(iii) a fragment of CDTb having at least 30, 50, 80, 100, 120, 150, 200, 250 or 300 contiguous amino acids of SEQ ID NO: 10 or SEQ ID NO: 11 or SEQ ID NO: 25 or SEQ ID NO: 26.

In one embodiment of the disclosure the isolated *Clostridium difficile* CDTb protein is or comprises
(i) SEQ ID NO: 43 or SEQ ID NO: 44; or
(ii) a variant of CDTb having at least 80%, 85%, 88%, 90%, 92%, 95%, 98%, 99%, 100% sequence identity to SEQ ID NO: 43 or SEQ ID NO: 44; or
(iii) a fragment of CDTb having at least 30, 50, 80, 100, 120, 150, 200, 250 or 300 contiguous amino acids of SEQ ID NO: 43 or SEQ ID NO: 44.

In one such aspect there is provided an immunogenic composition wherein the isolated *Clostridium difficile* CDTb protein is a variant of CDTb having at least 80%, 85%, 88%, 90%, 92%, 95%, 98%, 99%, 100% sequence identity to SEQ ID NO: 10 or SEQ ID NO: 11 or SEQ ID NO: 25 or SEQ ID NO: 26.

In one such aspect there is provided an immunogenic composition wherein the isolated *Clostridium difficile* CDTb protein is a variant of CDTb having at least 80%, 85%, 88%, 90%, 92%, 95%, 98%, 99%, 100% sequence identity to SEQ ID NO: 43 or SEQ ID NO: 44.

In another aspect there is provided an immunogenic composition wherein the isolated *Clostridium difficile* CDTb protein is a fragment of CDTb having at least 30, 50, 80, 100, 120, 150, 200, 250, 300, 350, 400, 450, 500, 550, 600, 650, 700, 750, 800 or 850 contiguous amino acids of SEQ ID NO: 10 or SEQ ID NO: 11 or SEQ ID NO: 25 or SEQ ID NO: 26.

In another aspect there is provided an immunogenic composition wherein the isolated *Clostridium difficile* CDTb protein is a fragment of CDTb having at least 30, 50, 80, 100, 120, 150, 200, 250, 300, 350, 400, 450, 500, 550, 600 or 650 contiguous amino acids of SEQ ID NO: 43 or SEQ ID NO: 44.

The present disclosure also provides an immunogenic composition comprising an isolated *Clostridium difficile* CDTb protein which is a truncated CDTb protein with the signal peptide and the prodomain removed and also the receptor binding domain removed and/or the CDTa binding domain removed.

The term 'truncated CDTb protein with the signal peptide and the prodomain removed and also the receptor binding domain removed and/or the CDTa binding domain removed' means a truncated CDTb protein with the signal peptide and the prodomain removed, wherein also the receptor binding domain and/or the CDTa binding domain have been removed. It refers to SEQ ID NO: 3 or a fragment or variant thereof with substantially all of the signal peptide and the prodomain removed (therefore which does not comprise amino acids corresponding to substantially all of the signal peptide and substantially all of the prodomain) and also substantially all of the receptor binding domain removed and/or substantially all of the CDTa binding domain removed (therefore which does not comprise amino acids corresponding to substantially all of the receptor binding domain and/or which does not comprise amino acids corresponding to substantially all of the CDTa binding domain). For example, the term "truncated CDTb protein with the signal peptide removed and the prodomain removed" refers to a CDTb protein with all of the signal peptide removed and at least 85 of the amino acids of the prodomain removed.There may be a few amino acids of the signal peptide, prodomain, receptor binding domain or CDTa binding domain remaining. For example 2, 5, 10, 15 or 20 amino acids of the signal peptide may remain. For example 2, 5, 10, 15 or 20 amino acids of the prodomain may remain. For example 2, 5, 10, 15 or 20 amino acids of the receptor binding domain may remain. For example 2, 5, 10, 15 or 20 amino acids of the CDTa binding domain may remain.

The signal peptide of CDTb corresponds to amino acids 1-48 (encompassing amino acids 1-42) of SEQ ID NO: 3 or their equivalents in a binary toxin protein isolated from a different strain of C. *difficile,* for example amino acids 1-42 of the amino acid sequence of CDTb from strain CD196 (Perelle, M. et al Infect. Immun., 65 (1997), pp. 1402-1407).

The prodomain of CDTb corresponds to amino acids 48-211 (encompassing amino acids 48-166) of SEQ ID NO:3 or their equivalents in a binary toxin protein isolated from a different strain of *C*. *difficile.*

In one embodiment, the receptor binding domain of CDTb corresponds to amino acids 620-876 of SEQ ID NO:3, or their equivalents in a binary toxin protein isolated from a different strain of *C*. *difficile.* In another embodiment, the receptor binding domain corresponds to amino acids 636-876 of SEQ ID NO:3 or their equivalents in a binary toxin protein isolated from a different strain of *C*. *difficile.*

The CDTa binding domain of CDTb corresponds to amino acids 212-296 of SEQ ID NO: 3 or their equivalents in a binary toxin protein isolated from a different strain of *C*. *difficile.*

In one embodiment of the disclosure the immunogenic composition comprises an isolated *Clostridium difficile* CDTb protein which is a truncated CDTb protein with the signal peptide and the prodomain removed and also either the receptor binding domain removed or the CDTa binding domain removed.

In another embodiment of the disclosure the immunogenic composition comprises an isolated *Clostridium difficile* CDTb protein which is a truncated CDTb protein with the signal peptide and the prodomain removed and also the receptor binding domain removed and the CDTa binding domain removed.

Suitably the isolated *Clostridium difficile* CDTb protein is or comprises
(i) SEQ ID NO: 8 or SEQ ID NO: 9 or SEQ ID NO: 37;
(ii) a variant of CDTb having at least 80%, 85%, 88%, 90%, 92%, 95%, 98%, 99%, 100% sequence identity to SEQ ID NO: 8 or SEQ ID NO: 9 or SEQ ID NO: 37; or
(iii) a fragment of CDTb having at least 30, 50, 80, 100, 120, 150, 200, 250, 300, 350, 400, 450, 500, 550, 600, 650, 700, 750 or 800 contiguous amino acids of SEQ ID NO: 8 or SEQ ID NO: 9 or SEQ ID NO: 37.

Suitably the isolated *Clostridium difficile* CDTb protein is or comprises
(i) SEQ ID NO: 8 or SEQ ID NO: 9 or SEQ ID NO: 37;
(ii) a variant of CDTb having at least 80%, 85%, 88%, 90%, 92%, 95%, 98%, 99%, 100% sequence identity to SEQ ID NO: 8 or SEQ ID NO: 9 or SEQ ID NO: 37; or
(iii) a fragment of CDTb having at least 30, 50, 80, 100, 120, 150, 200, 250, 300, 350 or 400 contiguous amino acids of SEQ ID NO: 8 or at least 30, 50, 80, 100, 120, 150, 200, 250, 300, 350, 400, 450, 500 or, 550 contiguous amino acids of SEQ ID NO: 9 or at least 30, 50, 80, 100, 120, 150, 200, 250 or 300, contiguous amino acids of SEQ ID NO: 37

In one such aspect of the disclosure there is provided an immunogenic composition wherein the isolated *Clostridium difficile* CDTb protein is a variant of CDTb having at least 80%, 85%, 88%, 90%, 92%, 95%, 98%, 99%, 100% sequence identity to SEQ ID NO: 8 or SEQ ID NO: 9 or SEQ ID NO: 37.

In another aspect of the disclosure there is provided an immunogenic composition wherein the isolated *Clostridium difficile* CDTb protein is a fragment of CDTb having at least 30, 50, 80, 100, 120, 150, 200, 250, 300, 350, 400, 450, 500, 550, contiguous amino acids of SEQ ID NO: 8 or SEQ ID NO: 9 or SEQ ID NO: 37.

In a third aspect of the disclosure there is provided an immunogenic composition comprising an isolated *Clostridium difficile* CDTb protein wherein the isolated *Clostridium difficile* CDTb protein has been mutated to modify heptamerisation ability.

CDTb protein which has been mutated to modify heptamerisation ability means that the CDTb protein has been mutated to modify its ability to self-assemble into a heptameric fold. This heptamerisation is allowed by the sharing of beta-strands coming from each CDTb monomer in order to form a beta-barrel structure. In this aspect the CDTb protein may be a truncated CDTb protein with the signal peptide removed. In this aspect the CDTb protein may be a truncated CDTb protein with the signal peptide removed and the prodomain removed.

In one embodiment of the disclosure, the isolated *Clostridium difficile* CDTb protein has been mutated to reduce heptamerisation ability.

The ability of CDTb protein to heptamerise may be measured using a technique such as dynamic light scattering (DLS). DLS can be used to evaluate hydrodynamic radius in solution of CDTb purified proteins, in addition to providing information about homogeneity and to detect the presence of high molecular weight aggregates (such as heptamers) within a protein sample. This is based on the calculation of the diffusion coefficient of the different species that are obtained by measuring the light scattering fluctuation, which depends on protein molecular size and shape, and on the other minor constituents of the sample.

Based on a deep analysis of a 3D structural model obtained for CDTb, a loop in the oligomerisation domain may potentially be involved in the heptamerisation of CDTb. In this loop four amino acids have been identified having potential electrostatic interactions with amino acids contained in other monomers of heptameric CDTb. These residues are Asp537, Glu539, Asp540 and Lys541 of SEQ ID NO: 3.

In one embodiment of the disclosure the isolated *Clostridium difficile* CDTb protein comprises at least one mutation and/or truncation in the oligomerisation domain which modifies heptamerisation ability.

The oligomerisation domain corresponds to amino acids 297-619 of SEQ ID NO:3, or their equivalents in a CDTb protein isolated from a different strain of *C*. *difficile.*

In one embodiment of the disclosure the isolated *Clostridium difficile* CDTb protein comprises at least one mutation and/or truncation to the region of CDTb comprising amino acids 533-542 of SEQ ID NO: 3 or to the equivalent region in a different strain of *C*. *difficile.*

In one embodiment of the disclosure, the isolated Clostridium difficile CDTb protein comprises at least one mutation at Asp537, Glu539, Asp540 and Lys541 of SEQ ID NO: 3 or their equivalents in a different strain of *C*. *difficile.* In one embodiment of the disclosure, suitably any one or more of Asp537, Glu539, Asp540 and/or Lys541 is/are mutated to Gly or another small residue.

In another embodiment of the disclosure, the oligomerisation domain is truncated. For example, amino acids 533-542 of SEQ ID NO:3, or their equivalents in a CDTb protein isolated from a different strain of *C*. *difficile,* may be deleted and optionally replaced by other residues, e.g. by 3, 4, 5 or more Gly and/or Ala residues. In one embodiment of the disclosure amino acids 533-542 of SEQ ID NO:3, or their equivalents in a CDTb protein isolated from a different strain of *C*. *difficile,* may be deleted and optionally replaced by 3 Gly residues or 3 Ala residues.

In one embodiment of the disclosure, the isolated *Clostridium difficile* CDTb protein comprises mutations in four amino acids. For example, the CDTb protein comprises the mutation D537G-E539G-D540G-K541G or their equivalents in a different strain of *C*. *difficile.* "D537G" means that the aspartate at position 537 of the CDTb sequence in SEQ ID NO:3 is mutated to glycine. "E539G" means that the glutamate at position 539 of the CDTb sequence in SEQ ID NO:3 is mutated to glycine. "D540G" means that the aspartate at position 540 of the CDTb sequence in SEQ ID NO:3 is mutated to glycine. "K541G" means that the lysine at position 541 of the CDTb sequence in SEQ ID NO:3 is mutated to glycine.

In another embodiment of this aspect, the isolated *Clostridium difficile* CDTb protein comprises a mutation in which amino acids 533 to 542 of SEQ ID NO:3, or their equivalents in a CDTb protein isolated from a different strain of *C*. *difficile,* are replaced by 3 Gly residues.

In one embodiment of the disclosure the isolated *Clostridium difficile* CDTb protein is or comprises
(i) SEQ ID NO: 12 or SEQ ID NO: 13; or
(ii) a variant of CDTb having at least 80%, 85%, 88%, 90%, 92%, 95%, 98%, 99%, 100% sequence identity to SEQ ID NO: 12 or SEQ ID NO: 13; or
(iii) a fragment of CDTb having at least 30, 50, 80, 100, 120, 150, 200, 250, 300, 350, 400, 450, 500, 550, 600, 650, 700, 750, 800 or 850 contiguous amino acids of SEQ ID NO: 12 or SEQ ID NO: 13.

In one such aspect there is provided an immunogenic composition wherein the isolated *Clostridium difficile* CDTb protein is a variant of CDTb having at least 80%, 85%, 88%, 90%, 92%, 95%, 98%, 99%, 100% sequence identity to SEQ ID NO: 12 or SEQ ID NO: 13.

In another aspect there is provided an immunogenic composition wherein the isolated *Clostridium difficile* CDTb protein is a fragment of CDTb having at least 30, 50, 80, 100, 120, 150, 200, 250, 300, 350, 400, 450, 500, 550, 600, 650, 700, 750, 800 or 850 contiguous amino acids of SEQ ID NO: 12 or SEQ ID NO: 13.

The CDTb and CDTa proteins vary in amino acid sequence between different strains, for this reason the amino acid numbering may differ between strains. For this reason the term 'equivalents in a different strain' refers to amino acids which correspond to those of a reference strain (e.g., C. *difficile* R20291 from which SEQ ID NO:1 and SEQ ID NO:3 are derived), but which are found in a toxin from a different strain and which may thus be numbered differently. A region of 'equivalent' amino acids may be determined by aligning the sequences of the toxins from the different strains. Example binary toxin producing strains of C. *difficile* include CD196, CCUG 20309, R8637, IS81, IS93, IS51, IS58, R6786, R7605, R10456 and R5989. Unless otherwise indicated, the amino acids numbers provided throughout refer to those of reference strain R20291, as set forth in SEQ ID NO: 1 (CDTa) and SEQ ID NO:3 (CDTb).

In one embodiment of the disclosure the isolated *Clostridium difficile* CDTb protein is a monomer of CDTb. In a further embodiment the isolated *Clostridium difficile* CDTb protein is a multimer of CDTb. In a further embodiment the isolated *Clostridium difficile* CDTb protein is a heptamer of CDTb.

In one embodiment of the disclosure, the immunogenic composition does not comprise/does not further comprise an isolated *Clostridium difficile* CDTa protein.

The present disclosure provides an immunogenic composition comprising an isolated *Clostridium difficile* CDTb protein as the sole C. difficile antigen. As used herein the term "as the sole C. difficile antigen" means that the immunogenic composition does not also comprise another antigen from C. difficile e.g. the immunogenic composition does not also comprise a toxin A, toxin B or CDTa protein.

In one embodiment of the disclosure, the immunogenic composition comprises/further comprises an isolated *Clostridium difficile* CDTa protein.

Suitably the isolated *Clostridium difficile* CDTa protein is a truncated CDTa protein. "A truncated CDTa protein" as used herein means a CDTa protein that does not achieve its full length or its proper form, and thus is missing some of the amino acid residues that are present in full length CDTa of SEQ ID NO: 1 or an equivalent in a different strain, and which cannot perform the function for which it was intended because its structure is incapable of doing so, e.g. ADP ribosyltransferase activity and/or interacting with CDTb. A suitable assay for ADP ribosyltransferase activity is described in PLOS pathogens 2009, vol 5(10) : e1000626.

Suitably the isolated *Clostridium difficile* CDTa protein is a truncated CDTa protein which does not comprise the C-terminal domain. The term 'truncated CDTa protein which does not comprise the C-terminal domain' refers to a fragment or variant of SEQ ID NO:1 which does not comprise a substantial portion of the C-terminal domain, there may be a few amino acids of the C-terminal domain remaining, for example, 2, 5, 10, 15, 20, 25, 30, 35 or 50 amino acids of the C-terminal domain may remain. The C-terminal domain corresponds to amino acids 269-463 of SEQ ID NO:1 or their equivalents in a CDTa protein isolated from a different strain of C. *difficile.* In this embodiment of the disclosure the truncated *Clostridium difficile* CDTa protein suitably is or comprises
(i) SEQ ID NO: 18 or SEQ ID NO: 19;
(i) a variant of CDTa having at least 80%, 85%, 88%, 90%, 92%, 95%, 98%, 99%, 100% sequence identity to SEQ ID NO: 18 or SEQ ID NO: 19; or
(iii) a fragment of CDTa having at least 30, 50, 80, 100, 120, 150, or 190 contiguous amino acids of SEQ ID NO: 18 or SEQ ID NO: 19.

In one embodiment of the disclosure the truncated CDTa protein which does not comprise the C-terminal domain is a variant of CDTa having at least 80%, 85%, 88%, 90%, 92%, 95%, 98%, 99%, 100% sequence identity to SEQ ID NO: 20. In a further embodiment of the disclosure the truncated CDTa protein which does not comprise the C-terminal domain is a variant of CDTa having at least 30, 50, 80, 100, 120, 150, or 190 contiguous amino acids of SEQ ID NO: 20.

In a further embodiment of any of the aspects of the disclosure, the isolated *Clostridium difficile* CDTa protein suitably contains a mutation which reduces its ADP-ribosyltransferase activity. For example the isolated *Clostridium difficile* CDTa protein has a mutation from glutamate to another amino acid at position 428 of SEQ ID NO: 1 or an equivalent mutation in another strain of *C*. *difficile.* The term 'has a mutation at position 428' refers to CDTa proteins which have a mutation at this exact location but also to a CDTa protein which is isolated from a different strain and which has a mutation at an equivalent position. The CDTa protein varies in amino acid sequence between different strains, for this reason the amino acid numbering may differ between strains, thus a CDTa protein from a different strain may have a corresponding glutamate which is not number 428 in sequence. In one embodiment the isolated *Clostridium difficile* CDTa protein has a mutation from glutamate to glutamine at position 428 of SEQ ID NO: 1.

In a further embodiment of any of the aspects of the disclosure, the isolated *Clostridium difficile* CDTa protein suitably has a mutation from glutamate to a different amino acid at position 430 of SEQ ID NO:1 or an equivalent mutation in another strain of C. *difficile.* The term 'has a mutation at position 430' refers to proteins which have this exact location but also to a CDTa protein which is isolated from a different strain and which has a mutation at an equivalent position. In one embodiment the isolated *Clostridium difficile* CDTa protein has a mutation from glutamate to glutamine at position 430 of SEQ ID NO: 1.

In a further embodiment of any of the aspects of the disclosure, the isolated *Clostridium difficile* CDTa protein suitably is or comprises
(i) SEQ ID NO: 21; SEQ ID NO: 22; SEQ ID NO: 23; or SEQ ID NO: 24; or
(ii) a variant of CDTa having at least 80%, 85%, 88%, 90%, 92%, 95%, 98%, 99%, 100% sequence identity to SEQ ID NO: 21; SEQ ID NO: 22; SEQ ID NO: 23; or SEQ ID NO: 24; or
(iii) a fragment of CDTa having at least 30, 50, 80, 100, 120, 150, 200, 250, 300, 350 or 400 contiguous amino acids of SEQ ID NO: 21; SEQ ID NO: 22; SEQ ID NO: 23; or SEQ ID NO: 24.

In a further embodiment of any of the aspects of the disclosure, the isolated *Clostridium difficile* CDTa protein suitably is or comprises
(i) SEQ ID NO: 22; or
(ii) a variant of CDTa having at least 80%, 85%, 88%, 90%, 92%, 95%, 98%, 99%, 100% sequence identity to SEQ ID NO: 22; or
(iii) a fragment of CDTa having at least 30, 50, 80, 100, 120, 150, 200, 250, 300, 350 or 400 contiguous amino acids of SEQ NO: 22.

### Fusion proteins comprising a CDTa protein and a CDTb protein

In another aspect, the invention provides immunogenic compositions comprising a fusion protein comprising a full length CDTa protein and a CDTb protein wherein the CDTb protein is a full length CDTb protein with the prodomain removed. In this aspect "full length CDTa" means the full length CDTa of SEQ ID NO. 36 or the equivalent in another strain of C. *difficile,* or a variant of CDTa having at least 80%, 85%, 88%, 90%, 92%, 95%, 98%, 99%, 100% sequence identity to SEQ ID NO: 36

"Fusion polypeptide" or "fusion protein" refers to a protein having at least two heterologous polypeptides (e.g. at least two *Clostridium difficile* polypeptides) covalently linked, either directly or via an amino acid linker. It may also refer to a protein having at least two heterologous polypeptides linked non-covalently. The polypeptides forming the fusion protein are typically linked C-terminus to N-terminus, although they can also be linked C-terminus to C-terminus, N-terminus to N-terminus, or N-terminus to C-terminus. The polypeptides of the fusion protein can be in any order. This term also refers to conservatively modified variants, polymorphic variants, alleles, mutants, immunogenic fragments, and interspecies homologs of the antigens that make up the fusion protein.

The term "fused" refers to the linkage e.g. covalent linkage between two polypeptides in a fusion protein. The polypeptides are typically joined via a peptide bond, either directly to each other or via an amino acid linker. Optionally, the peptides can be joined via non-peptide covalent linkages known to those of skill in the art.

A peptide linker sequence may be employed to separate the first and second polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46 (1985); Murphy et al., Proc. Natl. Acad. Sci. USA 83:8258-8262 (1986); U.S. Patent No. 4,935,233 and U.S. Patent No. 4,751,180. The linker sequence may generally be from 1 to about 50 amino acids in length for example 1, 5, 10, 15, 20, 25, 30, 35 or 40 amino acids in length. Linker sequences are not required when the first and second polypeptides have non-essential N-terminal amino acid regions that can be used to separate the functional domains and prevent steric interference.

In this aspect the fusion protein may suitably comprise a linker. An example of a suitable linker comprises or consists of six consecutive Gly residues.

In this aspect the CDTa protein is suitably first in the fusion. This means that the CDTa protein is placed in the N-terminal of the fusion protein relative to the position of the CDTb protein. This improves the processabilty of the CDTa-CDTb fusion.

In one embodiment the CDTa protein has a mutation from cysteine to another amino acid at position 45 of SEQ ID NO: 1 or the equivalent amino acid in another strain of C. *difficile.* The term 'has a mutation at position 45' refers to CDTa proteins which have a mutation at this exact location of SEQ ID NO: 1 but also to a CDTa protein which is isolated from a different strain and which has a mutation at an equivalent position. In one embodiment the CDTa protein has a mutation from cysteine to tyrosine at position 45. This mutation is made in order to prevent/avoid the formation of a disulfide bond between two CDTa proteins.

In one embodiment, the full length CDTa protein comprises one or more mutations which reduce its ADP-ribosyltransferase activity.

For example, the CDTa protein may have any of the mutation sets disclosed in WO2013/112867. For example, the CDTa protein may comprise at least two mutations selected from the following mutations at positions 2, 67, 69, 253, 255, 258, 302, 307, 342, 345, 356, 359, 382, 385 and 387 (corresponding to positions 3, 68, 70, 254, 256, 259, 303, 308, 343, 346, 357, 360, 383, 386 and 388 of the sequence of SEQ ID NO: 36 of the present application:) C2A, Y67A, Y69A, Y253A, R255A, Y258A, R302A, Q307E, N342A, S345F, F356A, R359A, Y382A, E385Q, E385A, E387Q, and E387D as disclosed in WO2013/112867. "C2A" means that the cysteine at position 2 of the CDTb protein sequence disclosed in WO2013/112867 (corresponding to position 3 of SEQ ID NO:36 of the present application) is mutated to alanine, and the other mutations listed in this group should be understood similarly. In some embodiments, the CDTa mutations are selected from: C2A, R302A, S345F, E385Q, E385A, E387Q, and E387D. In embodiments of this aspect of the invention, the CDTa protein comprises a set of mutations selected from the group consisting of: (a) S345F, E385Q and E387Q; (b) R302A, E385A, E387D; (c) C2A, S345F, E385Q and E387Q; (d) R302A, S345F, E385Q and E387Q; (e) Y67A, Y69A, and R255A; (f) R359A, Y67A, and Q307E; (g) Y258A, F356A, S345F; and (h) N342A, Y253A and Y382A.

In one embodiment, the CDTa protein comprises one mutation which reduces its ADP-ribosyltransferase activity.

The term "CDTa protein comprises one mutation which reduces its ADP-ribosyltransferase activity" refers to a CDTa protein which comprises a mutation at a single position which alone is intended to reduce its ADP ribosyltransferase activity. The CDTa protein may optionally comprise other mutations not specifically intended to reduce its ADP ribosyltransferase activity. Mutations specifically intended to reduce ADP ribosyltransferase activity include E428Q and E430Q (amino acid numbering with reference to SEQ ID NO:1) or C2A, Y67A, Y69A, Y253A, R255A, Y258A, R302A, Q307E, N342A, S345F, F356A, R359A, Y382A, E385Q, E385A, E387Q, and E387D as disclosed in WO2013/112867 (amino acid numbering corresponding to positions 3, 68, 70, 254, 256, 259, 303, 308, 343, 346, 357, 360, 383, 386 and 388 with reference to SEQ ID NO 36).

For example the CDTa protein has a mutation from glutamate to another amino acid at position 428 of SEQ ID NO: 1 or the equivalent amino acid in another strain of *C*. *difficile.* The term 'has a mutation at position 428' refers to CDTa proteins which have a mutation at this exact location of SEQ ID NO: 1 but also to a CDTa protein which is isolated from a different strain and which has a mutation at an equivalent position. The CDTa protein varies in amino acid sequence between different strains, for this reason the amino acid numbering may differ between strains, thus a CDTa protein from a different strain may have a corresponding glutamate which is not number 428 in sequence. In one embodiment the CDTa protein has a mutation from glutamate to glutamine at position 428. In a further embodiment, the CDTa protein suitably has a mutation from glutamate to a different amino acid at position 430 of SEQ ID NO: 1 or the equivalent amino acid in another strain of *C*. *difficile* The term 'has a mutation at position 430' refers to proteins which have this exact location but also to a CDTa protein which is isolated from a different strain and which has a mutation at an equivalent position. In one embodiment the CDTa protein has a mutation from glutamate to glutamine at position 430.

In one embodiment, the full length CDTa protein suitably is or comprises
(i) SEQ ID NO: 39; or
(ii)a variant CDTa having at least 80%, 85%, 88%, 90%, 92%, 95%, 98%, 99%, 100% sequence identity to SEQ ID NO: 39; or
(iii) a fragment of CDTa having at least 30, 50, 80, 100, 120, 150, 200, 250, 300, 350 or 400 contiguous amino acids of SEQ ID NO: 39.

In another embodiment, the full length CDTa protein suitably is or comprises
(i) a sequence of amino acids as set forth in SEQ ID NO: 40, 42 or 67 in WO2013/112867; or
(ii) a variant of CDTa having at least 80%, 85%, 88%, 90%, 92%, 95%, 98%, 99%, 100% sequence identity to any of the sequences in (i); or
(iii) a fragment of CDTa having at least 30, 50, 80, 100, 120, 150, 200, 250, 300, 350 or 400 contiguous amino acids of any of the sequences in (i).

In one embodiment, the CDTb protein is a full length CDTb protein. In this aspect "full length CDTb" means the full length CDTb of SEQ ID NO. 7, ie CDTb with the prodomain removed.

In this embodiment of this aspect, the fusion protein comprising a full length CDTa protein and a CDTb protein suitably is or comprises
(i) SEQ ID NO: 14; or
(ii)a variant fusion protein having at least 80%, 85%, 88%, 90%, 92%, 95%, 98%, 99%, 100% sequence identity to SEQ ID NO: 14; or
(iii) a fragment having at least 30, 50, 80, 100, 120, 150, 200, 250, 300, 350, 400, 450, 500, 550, 600, 650, 700, 750, 800 or 850 contiguous amino acids of SEQ ID NO: 14.

In another embodiment of the disclosure, the CDTb protein is a truncated CDTb protein with the CDTa binding domain removed. The term 'with the CDTa binding domain removed' refers to a fragment or variant of SEQ ID NO: 3, or its equivalent in another strain of C. *difficile,* with substantially all of the CDTa binding domain removed (therefore which does not comprise amino acids corresponding to substantially all of the CDTa binding domain). For example, the term "truncated CDTb protein with the CDTa binding domain removed" refers to a CDTb protein with at least 65 of the amino acids of the CDTa binding domain removed.There may be a few amino acids of the CDTa binding domain remaining. For example 2, 5, 10, 15 or 20 amino acids of the CDTa binding domain may remain.

The CDTa binding domain of CDTb corresponds to amino acids 212 to 295 of SEQ ID NO: 3 or their equivalents in a binary toxin protein isolated from a different strain of *C*. *difficile.*

In this embodiment, the truncated CDTb protein with the CDTa binding domain removed suitably is or comprises
(i) SEQ ID NO: 9; or
(ii)a variant fusion protein having at least 80%, 85%, 88%, 90%, 92%, 95%, 98%, 99%, 100% sequence identity to SEQ ID NO: 9; or
(iii) a fragment having at least 30, 50, 80, 100, 120, 150, 200, 250, 300, 350 or 400 contiguous amino acids of SEQ ID NO: 9.

In this embodiment, the fusion protein comprising a full length CDTa protein and a truncated CDTb protein with the CDTa binding domain removed suitably is or comprises
(i) SEQ ID NO: 15; or
(ii) a variant fusion protein having at least 80%, 85%, 88%, 90%, 92%, 95%, 98%, 99%, 100% sequence identity to SEQ ID NO: 15; or
(iii) a fragment having at least 30, 50, 80, 100, 120, 150, 200, 250, 300, 350, 400, 450, 500, 550, 600, 650, 700, 750, 800 or 850contiguous amino acids of SEQ ID NO: 15.

In another embodiment of the disclosure the CDTb protein is a truncated CDTb protein comprising the receptor binding domain. The term 'truncated CDTb protein comprising the receptor binding domain' refers to a fragment or variant of SEQ ID NO:3, or its equivalents in another strain of *C*. *difficile,* with substantially all but the receptor binding domain removed (therefore which does not comprise amino acids corresponding to substantially all of the protein except for the receptor binding domain), there may be a few amino acids in addition to the receptor binding domain remaining, for example 2, 5, 10, 15 or 20 amino acids except for/in addition to the receptor binding domain. In one version, the receptor binding domain corresponds to amino acids 620-876 of SEQ ID NO:3, or their equivalents in a binary toxin protein isolated from a different strain of *C*. *difficile.* In another version, the receptor binding domain corresponds to amino acids 636-876 of SEQ ID NO:3 or their equivalents in a binary toxin protein isolated from a different strain of *C*. *difficile.*

In this embodiment the CDTb protein suitably is or comprises
(i) SEQ ID NO: 27 or SEQ ID NO: 28; or
(i) a variant of CDTb having at least 80%, 85%, 88%, 90%, 92%, 95%, 98%, 99%, 100% sequence identity to SEQ ID NO: 27 or SEQ ID NO: 28; or
(iii) a fragment of CDTb having at least 30, 50, 80, 100, 120, 150 or 200 contiguous amino acids of SEQ ID NO: 27 or SEQ ID NO: 28.

In this embodiment, the fusion protein comprising a full length CDTa protein and a truncated CDTb protein comprising the receptor binding domain suitably is or comprises
(i) SEQ ID NO: 16 or SEQ ID NO: 17; or
(ii)a variant fusion protein having at least 80%, 85%, 88%, 90%, 92%, 95%, 98%, 99%, 100% sequence identity to SEQ ID NO: 16 or SEQ ID NO: 17; or
(iii) a fragment having at least 30, 50, 80, 100, 120, 150, 200, 250, 300, 350 or 400 contiguous amino acids of SEQ ID NO: 16 or SEQ ID NO: 17.

In another aspect the invention provides novel polypeptides and nucleotides as defined herein.

In one embodiment, the invention provides a polypeptide comprising an amino acid sequence represented by any of SEQ ID NOs 8, 9, 10, 11, 12, 13, 14, 15, 16, 17, 25, 26, 37, 43, 44, 49, 50, 51, 52, 53, 54, 55, 56, 57, 58, 66, 67 or 71.
In one embodiment of any of the aspects of the invention, the immunogenic composition elicits antibodies that neutralize CDTa or CDTb or both. In a further embodiment the composition elicits antibodies that neutralize binary toxin. Whether a composition elicits antibodies against a toxin can be measured by immunising mice with the immunogenic composition, collecting sera and analysing the anti-toxin titres of the sera using by ELISA. The sera should be compared to a reference sample obtained from mice which have not been immunised. The composition of the invention elicits antibodies that neutralise CDTa if the sera against the polypeptide gives an ELISA readout more than 10%, 20%, 30%, 50%, 70%, 80%, 90%, or 100% higher than the reference sample.

In a further embodiment the immunogenic compositions of the invention elicits a protective immune response in a mammalian host against strains of *C*. *Difficile.* In one embodiment the mammalian host is selected from the group consisting of mouse, rabbit, guinea pig, non-human primate, monkey and human. In one embodiment the mammalian host is a mouse. In a further embodiment the mammalian host is a human.

Whether an immunogenic composition elicits a protective immune response in a mammalian host against strains of *C*. *Difficile* can be determined using a challenge assay. In such an assay the mammalian host is vaccinated with the immunogenic composition and challenged by exposure to *C*. *Difficile,* the time which the mammal survives after challenge is compared with the time which a reference mammal that has not been immunised with the immunogenic composition survives. An immunogenic composition elicits a protective immune response if a mammal immunised with the immunogenic composition survives at least 10%, 20%, 30%, 50%, 80%, 80%, 90%, or 100% longer than a reference mammal which has not been immunised after challenge with C. *Difficile.*

### Toxin A and Toxin B

In one embodiment of any of the aspects of the invention, the immunogenic compositions of the invention further comprise an isolated *Clostridium difficile* toxin A protein and/or an isolated C. *difficile* toxin B protein.

The term 'isolated *Clostridium difficile* toxin A protein' refers to a protein with the amino acid sequence of SEQ ID NO: 29, or a fragment or variant of SEQ ID NO: 29. In one embodiment the isolated *Clostridium difficile* toxin A protein is a fragment comprising 50, 100, 150, 200, 250, 300, 500, 750, 1000, 1250, 1500, 1750, 2000 or 2500 contiguous amino acids of SEQ ID NO: 29. In one embodiment the isolated *Clostridium difficile* toxin A protein is a variant comprising 80%, 85%, 90%, 92%, 95%, 98%, 99% or 100% identity to SEQ ID NO: 29.

The term 'isolated *Clostridium difficile* toxin B protein' refers to a protein with the amino acid sequence of SEQ ID NO: 30, or a fragment or variant of SEQ ID NO: 30. In one embodiment the isolated *Clostridium difficile* toxin B protein is a fragment comprising 50, 100, 150, 200, 250, 300, 500, 750, 1000, 1250, 1500, 1750 or 2000 SEQ ID NO: 30. In one embodiment the isolated *Clostridium difficile* toxin B protein is a variant comprising 80%, 85%, 90%, 92%, 95%, 98%, 99% or 100% identity to SEQ ID NO: 30.

In one embodiment the isolated *Clostridium difficile* toxin A protein comprises a repeating domain fragment. The term 'toxin A repeating domain' refers to the C-terminal domain of the toxin A protein from *C*. *difficile,* comprising repeated sequences. The toxin A repeating domain refers to amino acids 1832-2710 of toxin A from strain VPI10463 (ATCC43255) and their equivalents in a different strain The sequence of amino acids 1832-2710 from strain VPI10463 (ATCC43255) corresponds to amino acids 1832-2710 of SEQ ID NO: 29.

In a further embodiment the isolated *Clostridium difficile* toxin A protein comprises a fragment of the toxin A N-terminal domain. The toxin A N-terminal domain refers to amino acids 1-1831 of toxin A from strain VPI10463 (ATCC43255) and their equivalents in a different strain. The sequence of amino acids 1-1831 of toxin A from strain VPI10463 (ATCC43255) corresponds to amino acids 1-1831 of SEQ ID NO: 29.

In one embodiment the isolated *Clostridium difficile* toxin B protein comprises a toxin B repeating domain fragment. The term 'toxin B repeating domain' refers to the C-terminal domain of the toxin B protein from *C*. *difficile.* This domain refers to amino acids 1834-2366 from strain VPI10463 (ATCC43255) and their equivalents in a different strain. The sequence of amino acids 1834-2366 from strain VPI10463 (ATCC43255) corresponds to amino acids 1834-2366 of SEQ ID NO: 30.

In a further embodiment the isolated *Clostridium difficile* toxin B protein comprises a fragment of the toxin B N-terminal domain. The toxin B N-terminal domain refers to amino acids 1-1833 of toxin B from strain VBI10463 (ATCC43255) and their equivalents in a different strain. The sequence of amino acids 1-1833 of toxin B from strain VBI10463 (ATCC43255) corresponds to amino acids 1-1833 of SEQ ID NO: 30.

The *C*. *difficile* toxins A and B are conserved proteins, however the sequence differs a small amount between strains, moreover the amino acid sequence for toxins A and B in different strains may differ in number of amino acids.

For these reasons the terms toxin A repeating domain and/or toxin B repeating domain to refer to a sequence which is a variant with 90%, 95%, 98%, 99% or 100% sequence identity to amino acids 1832-2710 of SEQ ID NO: 29 or a variant with 90%, 95%, 98%, 99% or 100% sequence identity to amino acids 1834-2366 of SEQ ID NO: 30. Similarly the terms toxin A N-terminal domain and/or toxin B N terminal domain refer to a sequence which is a variant with 90%, 95%, 98%, 99% or 100% sequence identity to amino acids 1-1831 of SEQ ID NO: 29 or a variant with 90%, 95%, 98%, 99% or 100% sequence identity to amino acids 1-1833 of SEQ ID NO: 30.

Furthermore the amino acid numbering may differ between the C-terminal domains of toxin A (or toxin B) from one strain and toxin A (or toxin B) from another strain. For this reason the term 'equivalents in a different strain' refers to amino acids which correspond to those of a reference strain (e.g., *C*. *difficile* VPI10463), but which are found in a toxin from a different strain and which may thus be numbered differently. A region of 'equivalent' amino acids may be determined by aligning the sequences of the toxins from the different strains. The amino acids numbers provided throughout refer to those of strain VPI10463 (and are as set forth in SEQ ID NO: 29 and SEQ ID NO: 30).

In a further embodiment of any of the aspects of the invention, the isolated *C*. *difficile* toxin A protein and the isolated *C*. *difficile* toxin B protein form a fusion protein. In one embodiment the fusion protein is 80%, 85%, 90%, 95%, 98%, 99% or 100% identical to a sequence selected from the group consisting of SEQ ID NO: 31, 32, 33, 34, and 35. In a further embodiment the fusion protein is a fragment of at least 800, 850, 900 or 950 contiguous amino acids of a sequence selected from the group consisting of SEQ ID NO: 31, 32, 33, 34, and 35.

### Fragments

The term "fragment" as defined herein may refer to a fragment comprising a T cell epitope. T cell epitopes are short contiguous stretches of amino acids which are recognised by T cells (e.g. CD4+ or CD8+ T cells). Identification of T cell epitopes may be achieved through epitope mapping experiments which are well known to the person skilled in the art (see, for example, Paul, Fundamental Immunology, 3rd ed., 243-247 (1993); Beiβbarth et al Bioinformatics 2005 21(Suppl. 1):i29-i37).

The term "fragment" when used in relation to a polypeptide comprising a histidine tag includes the polypeptide without the histidine tag, for example from which the histidine tag has been cleaved. For example, suitable fragments of polypeptides comprising an amino acid sequence represented by any of sequence ID Nos. 5, 7-28 or 36-42 (each of which comprises a histidine tag) include the corresponding polypeptide without the histidine tag represented by sequence ID Nos 47-76, as set out in the following concordance:

| Polypeptide including histag: | Suitable fragment without histag: |
|---|---|
| SEQ ID NO: 5 | SEQ ID NO: 47 |
| SEQ ID NO: 7 | SEQ ID NO: 48 |
| SEQ ID NO: 8 | SEQ ID NO: 49 |
| SEQ ID NO: 9 | SEQ ID NO: 50 |
| SEQ ID NO: 10 | SEQ ID NO: 51 |
| SEQ ID NO: 11 | SEQ ID NO: 52 |
| SEQ ID NO: 12 | SEQ ID NO: 53 |
| SEQ ID NO: 13 | SEQ ID NO: 54 |
| SEQ ID NO: 14 | SEQ ID NO: 55 |
| SEQ ID NO: 15 | SEQ ID NO: 56 |
| SEQ ID NO: 16 | SEQ ID NO: 57 |
| SEQ ID NO: 17 | SEQ ID NO: 58 |
| SEQ ID NO: 18 | SEQ ID NO: 59 |
| SEQ ID NO: 19 | SEQ ID NO: 60 |
| SEQ ID NO: 20 | SEQ ID NO: 61 |
| SEQ ID NO: 21 | SEQ ID NO: 62 |
| SEQ ID NO: 22 | SEQ ID NO: 63 |
| SEQ ID NO: 23 | SEQ ID NO: 64 |
| SEQ ID NO: 24 | SEQ ID NO: 65 |
| SEQ ID NO: 25 | SEQ ID NO: 66 |
| SEQ ID NO: 26 | SEQ ID NO: 67 |
| SEQ ID NO: 27 | SEQ ID NO: 68 |
| SEQ ID NO: 28 | SEQ ID NO: 69 |
| SEQ ID NO: 36 | SEQ ID NO: 70 |
| SEQ ID NO: 37 | SEQ ID NO: 71 |
| SEQ ID NO: 38 | SEQ ID NO: 72 |
| SEQ ID NO: 39 | SEQ ID NO: 73 |
| SEQ ID NO: 40 | SEQ ID NO: 74 |
| SEQ ID NO: 41 | SEQ ID NO: 75 |
| SEQ ID NO: 42 | SEQ ID NO: 76 |

Suitably the fragments of the invention are immunogenic fragments. "Immunogenic fragments" according to the present invention will typically comprise at least 9 contiguous amino acids from the full length polypeptide sequence (e.g. at least 10), such as at least 12 contiguous amino acids (e.g. at least 15 or at least 20 contiguous amino acids), in particular at least 50 contiguous amino acids, such as at least 100 contiguous amino acids (for example at least 200 contiguous amino acids). Suitably the immunogenic fragments will be at least 20%, such as at least 50%, at least 70% or at least 80% of the length of the full length polypeptide sequence.

It will be understood that in a diverse out-bred population, such as humans, different HLA types mean that specific epitopes may not be recognised by all members of the population. Consequently, to maximise the level of recognition and scale of immune response to a polypeptide, it is generally desirable that an immunogenic fragment contains a plurality of the epitopes from the full length sequence (suitably all epitopes).

### Variants

"Variants" or "conservatively modified variants" applies to both amino acid and nucleic acid sequences. With respect to particular nucleic acid sequences, conservatively modified variants refers to those nucleic acids which encode identical or essentially identical amino acid sequences. Where the nucleic acid does not encode an amino acid sequence, "conservatively modified variants" refers to essentially identical sequences.

In respect of variants of a protein sequence, the skilled person will recognise that individual substitutions, deletions or additions to polypeptide, which alters, adds or deletes a single amino acid or a small percentage of amino acids is a "conservatively modified variant" where the alteration(s) results in the substitution of an amino acid with a functionally similar amino acid or the substitution/deletion/addition of residues which do not substantially impact the biological function of the variant.

Conservative substitution tables providing functionally similar amino acids are well known in the art. Such conservatively modified variants are in addition to and do not exclude polymorphic variants, interspecies homologs, and alleles of the invention.

A polypeptide of the invention (such as a CDTa protein or a CDTb protein) may contain a number of conservative substitutions (for example, 1-50, such as 1-25, in particular 1-10, and especially 1 amino acid residue(s) may be altered) when compared to the reference sequence. In general, such conservative substitutions will fall within one of the amino-acid groupings specified below, though in some circumstances other substitutions may be possible without substantially affecting the immunogenic properties of the antigen. The following eight groups each contain amino acids that are typically conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)
(see, e.g., Creighton, Proteins 1984).

Suitably such substitutions do not occur in the region of an epitope, and do not therefore have a significant impact on the immunogenic properties of the antigen.

Polypeptide variants may also include those wherein additional amino acids are inserted compared to the reference sequence, for example, such insertions may occur at 1-10 locations (such as 1-5 locations, suitably 1 or 2 locations, in particular 1 location) and may, for example, involve the addition of 50 or fewer amino acids at each location (such as 20 or fewer, in particular 10 or fewer, especially 5 or fewer). Suitably such insertions do not occur in the region of an epitope, and do not therefore have a significant impact on the immunogenic properties of the antigen. One example of insertions includes a short stretch of histidine residues (e.g. 2-6 residues) to aid expression and/or purification of the antigen in question.

Polypeptide variants include those wherein amino acids have been deleted compared to the reference sequence, for example, such deletions may occur at 1-10 locations (such as 1-5 locations, suitably 1 or 2 locations, in particular 1 location) and may, for example, involve the deletion of 50 or fewer amino acids at each location (such as 20 or fewer, in particular 10 or fewer, especially 5 or fewer). Suitably such deletions do not occur in the region of an epitope, and do not therefore have a significant impact on the immunogenic properties of the antigen.

The skilled person will recognise that a particular polypeptide variant may comprise substitutions, deletions and additions (or any combination thereof).

Variants preferably exhibit at least about 70% identity, more preferably at least about 80% identity and most preferably at least about 90% identity (such as at least about 95%, at least about 98% or at least about 99%) to the associated reference sequence.

The terms "identical" or percent "identity," in the context of two or more nucleic acids or polypeptide sequences, refer to two or more sequences or sub-sequences that are the same or have a specified percentage of amino acid residues or nucleotides that are the same (i.e., 70% identity, optionally 75%, 80%, 85%, 90%, 95%, 98% or 99% identity over a specified region), when compared and aligned for maximum correspondence over a comparison window, or designated region as measured using, for example, sequence comparison algorithms or by manual alignment and visual inspection. Such sequences are then said to be "substantially identical." This definition also refers to the compliment of a test sequence. Optionally, the identity exists over a region that is at least about 25 to about 50 amino acids or nucleotides in length, or optionally over a region that is 75-100 amino acids or nucleotides in length. Suitably, the comparison is performed over a window corresponding to the entire length of the reference sequence.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, references to a segment in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see*, *e*.*g*., Current Protocols in Molecular Biology (Ausubel et al., eds. 1995 supplement)).

One example of a useful algorithm is PILEUP. PILEUP creates a multiple sequence alignment from a group of related sequences using progressive, pairwise alignments to show relationship and percent sequence identity. It also plots a tree or dendogram showing the clustering relationships used to create the alignment. PILEUP uses a simplification of the progressive alignment method of Feng & Doolittle, J. Mol. Evol. 35:351-360 (1987). The method used is similar to the method described by Higgins & Sharp, CABIOS 5:151-153 (1989). The program can align up to 300 sequences, each of a maximum length of 5,000 nucleotides or amino acids. The multiple alignment procedure begins with the pairwise alignment of the two most similar sequences, producing a cluster of two aligned sequences. This cluster is then aligned to the next most related sequence or cluster of aligned sequences. Two clusters of sequences are aligned by a simple extension of the pairwise alignment of two individual sequences. The final alignment is achieved by a series of progressive, pairwise alignments. The program is run by designating specific sequences and their amino acid or nucleotide coordinates for regions of sequence comparison and by designating the program parameters. Using PILEUP, a reference sequence is compared to other test sequences to determine the percent sequence identity relationship using the following parameters: default gap weight (3.00), default gap length weight (0.10), and weighted end gaps. PILEUP can be obtained from the GCG sequence analysis software package, e.g., version 7.0 (Devereaux et al., Nuc. Acids Res. 12:387-395 (1984).

Another example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. Software for performing BLAST analyses is publicly available through the National Center for Biotechnology Information (website at www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al*., *supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (see Henikoff & Henikoff, Proc. Natl. Acad. Sci. USA 89:10915 (1989)) alignments (B) of 50, expectation (E) of 10, M=5, N=-4, and a comparison of both strands.

The BLAST algorithm also performs a statistical analysis of the similarity between two sequences *(see, e.g.,* Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

### POLYNUCLEOTIDE IDENTIFICATION AND CHARACTERISATION

Polynucleotides encoding the *Clostridium difficile* CDTa, CDTb, Toxin A and Toxin B proteins of the invention may be identified, prepared and/or manipulated using any of a variety of well established techniques. For example, a polynucleotide may be identified, as described in more detail below, by screening a microarray of cDNAs. Such screens may be performed, for example, using a Synteni microarray (Palo Alto, CA) according to the manufacturer's instructions (and essentially as described by Schena et al., Proc. Natl. Acad. Sci. USA 93:10614-10619 (1996) and Heller et al., Proc. Natl. Acad. Sci. USA 94:2150-2155 (1997)). Alternatively, polynucleotides may be amplified from cDNA prepared from cells expressing the proteins described herein, such as M. *tuberculosis* cells. Such polynucleotides may be amplified via polymerase chain reaction (PCR). For this approach, sequence-specific primers may be designed based on the sequences provided herein, and may be purchased or synthesised.

An amplified portion of a polynucleotide may be used to isolate a full length gene from a suitable library (e.g., a M. *tuberculosis* cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridisation techniques, a partial sequence may be labelled (e.g., by nick-translation or end-labelling with ³²P) using well known techniques. A bacterial or bacteriophage library is then generally screened by hybridising filters containing denatured bacterial colonies (or lawns containing phage plaques) with the labelled probe (see Sambrook et al., Molecular Cloning: A Laboratory Manual (2000)). Hybridising colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences can then be assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using, for example, software well known in the art. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR (see Triglia et al., Nucl. Acids Res. 16:8186 (1988)), which uses restriction enzymes to generate a fragment in the known region of the gene. The fragment is then circularised by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Another such technique is known as "rapid amplification of cDNA ends" or RACE. This technique involves the use of an internal primer and an external primer, which hybridises to a polyA region or vector sequence, to identify sequences that are 5' and 3' of a known sequence. Additional techniques include capture PCR (Lagerstrom et al., PCR Methods Applic. 1:111-19 (1991)) and walking PCR (Parker et al., Nucl. Acids. Res. 19:3055-60 (1991)). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (e.g., NCBI BLAST searches), and such ESTs may be used to generate a contiguous full length sequence. Full length DNA sequences may also be obtained by analysis of genomic fragments.

### POLYNUCLEOTIDE EXPRESSION IN HOST CELLS

Polynucleotide sequences or fragments thereof which encode the *Clostridium difficile* CDTa, CDTb, Toxin A and Toxin B proteins, or fusion proteins or functional equivalents thereof, may be used in recombinant DNA molecules to direct expression of a polypeptide in appropriate host cells. Due to the inherent degeneracy of the genetic code, other DNA sequences that encode substantially the same or a functionally equivalent amino acid sequence may be produced and these sequences may be used to clone and express a given polypeptide.

As will be understood by those of skill in the art, it may be advantageous in some instances to produce polypeptide-encoding nucleotide sequences possessing non-naturally occurring codons. For example, codons preferred by a particular prokaryotic or eukaryotic host can be selected to increase the rate of protein expression or to produce a recombinant RNA transcript having desirable properties, such as a half-life which is longer than that of a transcript generated from the naturally occurring sequence.

Moreover, the polynucleotide sequences can be engineered using methods generally known in the art in order to alter polypeptide encoding sequences for a variety of reasons, including but not limited to, alterations which modify the cloning, processing, and/or expression of the gene product. For example, DNA shuffling by random fragmentation and PCR reassembly of gene fragments and synthetic oligonucleotides may be used to engineer the nucleotide sequences. In addition, site-directed mutagenesis may be used to insert new restriction sites, alter glycosylation patterns, change codon preference, produce splice variants, or introduce mutations, and so forth.

Natural, modified, or recombinant nucleic acid sequences may be ligated to a heterologous sequence to encode a fusion protein. For example, to screen peptide libraries for inhibitors of polypeptide activity, it may be useful to encode a chimeric protein that can be recognised by a commercially available antibody. A fusion protein may also be engineered to contain a cleavage site located between the polypeptide-encoding sequence and the heterologous protein sequence, so that the polypeptide may be cleaved and purified away from the heterologous moiety.

Sequences encoding a desired polypeptide may be synthesised, in whole or in part, using chemical methods well known in the art (see Caruthers, M. H. et al., Nucl. Acids Res. Symp. Ser. pp. 215-223 (1980), Horn et al., Nucl. Acids Res. Symp. Ser. pp. 225-232 (1980)). Alternatively, the protein itself may be produced using chemical methods to synthesize the amino acid sequence of a polypeptide, or a portion thereof. For example, peptide synthesis can be performed using various solid-phase techniques (Roberge et al., Science 269:202-204 (1995)) and automated synthesis may be achieved, for example, using the ABI 431A Peptide Synthesizer (Perkin Elmer, Palo Alto, CA).

A newly synthesised peptide may be substantially purified by preparative high performance liquid chromatography (e.g., Creighton, Proteins, Structures and Molecular Principles (1983)) or other comparable techniques available in the art. The composition of the synthetic peptides may be confirmed by amino acid analysis or sequencing (e.g., the Edman degradation procedure). Additionally, the amino acid sequence of a polypeptide, or any part thereof, may be altered during direct synthesis and/or combined using chemical methods with sequences from other proteins, or any part thereof, to produce a variant polypeptide.

In order to express a desired polypeptide, the nucleotide sequences encoding the polypeptide, or functional equivalents, may be inserted into an appropriate expression vector, i.e., a vector which contains the necessary elements for the transcription and translation of the inserted coding sequence. Methods which are well known to those skilled in the art may be used to construct expression vectors containing sequences encoding a polypeptide of interest and appropriate transcriptional and translational control elements. These methods include *in vitro* recombinant DNA techniques, synthetic techniques, and *in vivo* genetic recombination. Such techniques are described in Sambrook et al., Molecular Cloning, A Laboratory Manual (2000), and Ausubel et al., Current Protocols in Molecular Biology (updated annually).

A variety of expression vector/host systems may be utilised to contain and express polynucleotide sequences. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus, CaMV; tobacco mosaic virus, TMV) or with bacterial expression vectors (e.g., Ti or pBR322 plasmids); or animal cell systems.

The "control elements" or "regulatory sequences" present in an expression vector are those non-translated regions of the vector--enhancers, promoters, 5' and 3' untranslated regionswhich interact with host cellular proteins to carry out transcription and translation. Such elements may vary in their strength and specificity. Depending on the vector system and host utilised, any number of suitable transcription and translation elements, including constitutive and inducible promoters, may be used. For example, when cloning in bacterial systems, inducible promoters such as the hybrid lacZ promoter of the PBLUESCRIPT phagemid (Stratagene, La Jolla, Calif.) or PSPORT1 plasmid (Gibco BRL, Gaithersburg, MD) and the like may be used. In mammalian cell systems, promoters from mammalian genes or from mammalian viruses are generally preferred. If it is necessary to generate a cell line that contains multiple copies of the sequence encoding a polypeptide, vectors based on SV40 or EBV may be advantageously used with an appropriate selectable marker.

In bacterial systems, a number of expression vectors may be selected depending upon the use intended for the expressed polypeptide. For example, when large quantities are needed, for example for the induction of antibodies, vectors which direct high level expression of fusion proteins that are readily purified may be used. Such vectors include, but are not limited to, the multifunctional E. *coli* cloning and expression vectors such as BLUESCRIPT (Stratagene), in which the sequence encoding the polypeptide of interest may be ligated into the vector in frame with sequences for the amino-terminal Met and the subsequent 7 residues of β-galactosidase so that a hybrid protein is produced; pIN vectors (Van Heeke &Schuster, J. Biol. Chem. 264:5503-5509 (1989)); and the like. pGEX Vectors (Promega, Madison, Wis; GE Healthcare.) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor XA protease cleavage sites so that the cloned polypeptide of interest can be released from the GST moiety at will.

In the yeast, *Saccharomyces cerevisiae or Pichia such* as *Pichia pastoris for example,* a number of vectors containing constitutive or inducible promoters such as alpha factor, alcohol oxidase, and PGH may be used. Other vectors containing constitutive or inducible promoters include GAP, PGK, GAL and ADH. For reviews, see Ausubel *et al. (supra)* and Grant et al., Methods Enzymol. 153:516-544 (1987) and Romas et al. Yeast 8 423-88 (1992).

In cases where plant expression vectors are used, the expression of sequences encoding polypeptides may be driven by any of a number of promoters. For example, viral promoters such as the 35S and 19S promoters of CaMV may be used alone or in combination with the omega leader sequence from TMV (Takamatsu, EMBO J. 6:307-311 (1987)). Alternatively, plant promoters such as the small subunit of RUBISCO or heat shock promoters may be used (Coruzzi et al., EMBO J. 3:1671-1680 (1984); Broglie et al., Science 224:838-843 (1984); and Winter et al., Results Probl. Cell Differ. 17:85-105 (1991)). These constructs can be introduced into plant cells by direct DNA transformation or pathogen-mediated transfection. Such techniques are described in a number of generally available reviews (*see*, *e*.*g*., Hobbs in McGraw Hill Yearbook of Science and Technology pp. 191-196 (1992)).

An insect system may also be used to express a polypeptide of interest. For example, in one such system, *Autographa californica* nuclear polyhedrosis virus (AcNPV) is used as a vector to express foreign genes in *Spodoptera frugiperda* cells or in *Trichoplusia larvae.* The sequences encoding the polypeptide may be cloned into a non-essential region of the virus, such as the polyhedrin gene, and placed under control of the polyhedrin promoter. Successful insertion of the polypeptide-encoding sequence will render the polyhedrin gene inactive and produce recombinant virus lacking coat protein. The recombinant viruses may then be used to infect, for example, S. *frugiperda* cells or *Trichoplusia larvae* in which the polypeptide of interest may be expressed (Engelhard et al., Proc. Natl. Acad. Sci. U.S.A. 91 :3224-3227 (1994)).

In mammalian host cells, a number of viral-based expression systems are generally available. For example, in cases where an adenovirus is used as an expression vector, sequences encoding a polypeptide of interest may be ligated into an adenovirus transcription/translation complex consisting of the late promoter and tripartite leader sequence. Insertion in a non-essential E1 or E3 region of the viral genome may be used to obtain a viable virus which is capable of expressing the polypeptide in infected host cells (Logan & Shenk, Proc. Natl. Acad. Sci. U.S.A. 81:3655-3659 (1984)). In addition, transcription enhancers, such as the Rous sarcoma virus (RSV) enhancer, may be used to increase expression in mammalian host cells. Methods and protocols for working with adenovirus vectors are reviewed in Wold, Adenovirus Methods and Protocols, 1998. Additional references regarding use of adenovirus vectors can be found in Adenovirus: A Medical Dictionary, Bibliography, and Annotated Research Guide to Internet References, 2004.

Specific initiation signals may also be used to achieve more efficient translation of sequences encoding a polypeptide of interest. Such signals include the ATG initiation codon and adjacent sequences. In cases where sequences encoding the polypeptide, its initiation codon, and upstream sequences are inserted into the appropriate expression vector, no additional transcriptional or translational control signals may be needed. However, in cases where only coding sequence, or a portion thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf. et al., Results Probl. Cell Differ. 20:125-162 (1994)).

In addition, a host cell strain may be chosen for its ability to modulate the expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding and/or function. Different host cells such as CHO, HeLa, MDCK, HEK293, and WI38, which have specific cellular machinery and characteristic mechanisms for such post-translational activities, may be chosen to ensure the correct modification and processing of the foreign protein.

For long-term, high-yield production of recombinant proteins, stable expression is generally preferred. For example, cell lines which stably express a polynucleotide of interest may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include, but are not limited to, the herpes simplex virus thymidine kinase (Wigler et al., Cell 11:223-32 (1977)) and adenine phosphoribosyltransferase (Lowy et al., Cell 22:817-23 (1990)) genes which can be employed in tk.sup.- or aprt.sup.- cells, respectively. Also, antimetabolite, antibiotic or herbicide resistance can be used as the basis for selection; for example, dhfr which confers resistance to methotrexate (Wigler et al., Proc. Natl. Acad. Sci. U.S.A. 77:3567-70 (1980)); npt, which confers resistance to the aminoglycosides, neomycin and G-418 (Colbere-Garapin et al., J. Mol. Biol. 150:1-14 (1981)); and als or pat, which confer resistance to chlorsulfuron and phosphinotricin acetyltransferase, respectively (Murry, *supra*). Additional selectable genes have been described, for example, trpB, which allows cells to utilise indole in place of tryptophan, or hisD, which allows cells to utilise histinol in place of histidine (Hartman & Mulligan, Proc. Natl. Acad. Sci. U.S.A. 85:8047-51 (1988)). Recently, the use of visible markers has gained popularity with such markers as anthocyanins, β-glucuronidase and its substrate GUS, and luciferase and its substrate luciferin, being widely used not only to identify transformants, but also to quantify the amount of transient or stable protein expression attributable to a specific vector system (Rhodes et al., Methods Mol. Biol. 55:121-131 (1995)).

Although the presence/absence of marker gene expression suggests that the gene of interest is also present, its presence and expression may need to be confirmed. For example, if the sequence encoding a polypeptide is inserted within a marker gene sequence, recombinant cells containing sequences can be identified by the absence of marker gene function. Alternatively, a marker gene can be placed in tandem with a polypeptide-encoding sequence under the control of a single promoter. Expression of the marker gene in response to induction or selection usually indicates expression of the tandem gene as well.

Alternatively, host cells which contain and express a desired polynucleotide sequence may be identified by a variety of procedures known to those of skill in the art. These procedures include, but are not limited to, DNA-DNA or DNA-RNA hybridisations and protein bioassay or immunoassay techniques which include membrane, solution, or chip based technologies for the detection and/or quantification of nucleic acid or protein.

A variety of protocols for detecting and measuring the expression of polynucleotide-encoded products, using either polyclonal or monoclonal antibodies specific for the product are known in the art. Examples include enzyme-linked immunosorbent assay (ELISA), radioimmunoassay (RIA), and fluorescence activated cell sorting (FACS). A two-site, monoclonal-based immunoassay utilising monoclonal antibodies reactive to two non-interfering epitopes on a given polypeptide may be preferred for some applications, but a competitive binding assay may also be employed. These and other assays are described, among other places, in Hampton et al., Serological Methods, a Laboratory Manual (1990) and Maddox et al., J. Exp. Med. 158:1211-1216 (1983).

A wide variety of labels and conjugation techniques are known by those skilled in the art and may be used in various nucleic acid and amino acid assays. Means for producing labelled hybridisation or PCR probes for detecting sequences related to polynucleotides include oligolabeling, nick translation, end-labelling or PCR amplification using a labelled nucleotide. Alternatively, the sequences, or any portions thereof may be cloned into a vector for the production of an mRNA probe. Such vectors are known in the art, are commercially available, and may be used to synthesize RNA probes *in vitro* by addition of an appropriate RNA polymerase such as T7, T3, or SP6 and labelled nucleotides. These procedures may be conducted using a variety of commercially available kits. Suitable reporter molecules or labels, which may be used include radionuclides, enzymes, fluorescent, chemiluminescent, or chromogenic agents as well as substrates, cofactors, inhibitors, magnetic particles, and the like.

Host cells transformed with a polynucleotide sequence of interest may be cultured under conditions suitable for the expression and recovery of the protein from cell culture. The protein produced by a recombinant cell may be secreted or contained intracellularly depending on the sequence and/or the vector used. As will be understood by those of skill in the art, expression vectors containing polynucleotides may be designed to contain signal sequences which direct secretion of the encoded polypeptide through a prokaryotic or eukaryotic cell membrane. Other recombinant constructions may be used to join sequences encoding a polypeptide of interest to nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilised immunoglobulin, and the domain utilized in the FLAGS extension/affinity purification system (Immunex Corp., Seattle, Wash.). The inclusion of cleavable linker sequences such as those specific for Factor XA or enterokinase (Invitrogen. San Diego, Calif.) between the purification domain and the encoded polypeptide may be used to facilitate purification. One such expression vector provides for expression of a fusion protein containing a polypeptide of interest and a nucleic acid encoding 6 histidine residues preceding a thioredoxin or an enterokinase cleavage site. The histidine residues facilitate purification on IMIAC (immobilised metal ion affinity chromatography) as described in Porath et al., Prot. Exp. Purif. 3:263-281 (1992) while the enterokinase cleavage site provides a means for purifying the desired polypeptide from the fusion protein. A discussion of vectors which contain fusion proteins is provided in Kroll et al., DNA Cell Biol. 12:441-453 (1993)).

### POLYPEPTIDE COMPOSITIONS

Generally, a polypeptide of use in the disclosure (for example the *Clostridium difficile* CDTa, CDTb, Toxin A and Toxin B proteins) will be an isolated polypeptide (i.e. separated from those components with which it may usually be found in nature).

For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

Polypeptides may be prepared using any of a variety of well known techniques. Recombinant polypeptides encoded by DNA sequences as described above may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant polypeptide. Suitable host cells include prokaryotes, yeast, and higher eukaryotic cells, such as mammalian cells and plant cells. Preferably, the host cells employed are *E. coli*, yeast or a mammalian cell line such as COS or CHO. Supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

Polypeptides for use in the invention, immunogenic fragments thereof, and other variants having less than about 100 amino acids, and generally less than about 50 amino acids, may also be generated by synthetic means, using techniques well known to those of ordinary skill in the art. For example, such polypeptides may be synthesised using any of the commercially available solid-phase techniques, such as the Merrifield solid-phase synthesis method, where amino acids are sequentially added to a growing amino acid chain. See Merrifield, J. Am. Chem. Soc. 85:2149-2146 (1963). Equipment for automated synthesis of polypeptides is commercially available from suppliers such as Perkin Elmer/Applied BioSystems Division (Foster City, CA), and may be operated according to the manufacturer's instructions.

Within certain specific embodiments, a polypeptide may be a fusion protein that comprises multiple polypeptides as described herein, or that comprises at least one polypeptide as described herein and an unrelated sequence, examples of such proteins include tetanus, tuberculosis and hepatitis proteins (*see*, *e*.*g*., Stoute et al., New Engl. J. Med. 336:86-91 (1997)). A fusion partner may, for example, assist in providing T helper epitopes (an immunological fusion partner), preferably T helper epitopes recognised by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein.

Fusion proteins may generally be prepared using standard techniques, including chemical conjugation. Preferably, a fusion protein is expressed as a recombinant protein, allowing the production of increased levels, relative to a non-fused protein, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion protein that retains the biological activity of both component polypeptides.

A peptide linker sequence may be employed to separate the first and second polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46 (1985); Murphy et al., Proc. Natl. Acad. Sci. USA 83:8258-8262 (1986); U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

### Adjuvants

In a further embodiment of any of the aspects of the invention, the immunogenic composition further comprises an adjuvant. In one embodiment the adjuvant comprises aluminium hydroxide or aluminium phosphate. Alternatively the immunogenic composition of the invention may comprise an aluminium-free adjuvant, the immunogenic composition is formulated with an adjuvant that is free of aluminum or aluminum salts, that is, an aluminum-free adjuvant or adjuvant system.

In certain embodiments, the immunogenic composition is formulated with an adjuvant comprising an immunologically active saponin fraction presented in the form of a liposome. The adjuvant may further comprise a lipopolysaccharide. The adjuvant may include QS21. For example, in one embodiment, the adjuvant contains QS21 in a liposomal formulation. In one embodiment, the adjuvant system includes 3D-MPL and QS21. For example, in one embodiment, the adjuvant contains 3D-MPL and QS21 in a liposomal formulation. Optionally, the adjuvant system also contains cholesterol. In one specific embodiment, the adjuvant includes QS21 and cholesterol. Optionally, the adjuvant system contains 1, 2-Dioleoyl-sn-Glycero-3-phosphocholine (DOPC). For example, in one specific adjuvant system contains cholesterol, DOPC, 3D-MPL and QS21.

In one specific example, the immunogenic composition includes an adjuvant formulated in a dose that includes: from about 0.1 to about 0.5 mg cholesterol; from about 0.25 to about 2 mg DOPC; from about 10 µg to about 100 µg 3D-MPL; and from about 10 µg to about 100 µg QS21. In another specific example, the immunogenic composition includes an adjuvant formulated in a dose that includes: from about 0.1 to about 0.5 mg cholesterol; from about 0.25 to about 2 mg DOPC; from about 10 µg to about 70 µg 3D-MPL; and from about 10 µg to about 70 µg QS21. In one specific formulation, the adjuvant is formulated in a single dose that contains: about 0.25 mg cholesterol; about 1.0 mg DOPC; about 50 µg 3D-MPL; and about 50 µg QS21. In other embodiments, the immunogenic composition is formulated with a fractional dose (that is a dose, which is a fraction of the preceding single dose formulations, such as one half of the preceding quantity of components (cholesterol, DOPC, 3D-MPL and QS21), ¼ of the preceding quantity of components, or another fractional dose (e.g., 1/3, 1/6, etc.) of the preceding quantity of components.

In one embodiment, the immunogenic compositions according to the invention include an adjuvant containing combinations of lipopolysaccharide and Quillaja saponins that have been disclosed previously, for example in EP0671948. This patent demonstrated a strong synergy when a lipopolysaccharide (3D-MPL) was combined with a Quillaja saponin (QS21).

The adjuvant may further comprise immunostimulatory oligonucleotides (for example, CpG) or a carrier.

A particularly suitable saponin for use in the present invention is Quil A and its derivatives. Quil A is a saponin preparation isolated from the South American tree *Quillaja Saponaria Molina* and was first described by Dalsgaard et al. in 1974 ("Saponin adjuvants", Archiv. für die gesamte Virusforschung, Vol. 44, Springer Verlag, Berlin, p243-254) to have adjuvant activity. Purified fragments of Quil A have been isolated by HPLC which retain adjuvant activity without the toxicity associated with Quil A (EP 0 362 278), for example QS7 and QS21 (also known as QA7 and QA21). QS21 is a natural saponin derived from the bark of *Quillaja saponaria* Molina, which induces CD8+ cytotoxic T cells (CTLs), Th1 cells and a predominant IgG2a antibody response and is a preferred saponin in the context of the present invention.

When the adjuvant comprises an immunologically active saponin fraction presented in the form of a liposome, the adjuvant may further comprise a sterol. Suitably the sterol is provided at a ratio of saponin:sterol of from 1:1 to 1:100 w/w, such as from 1:1 to 1:10w/w; or 1:1 to 1:5 w/w.

In a specific embodiment, QS21 is provided in its less reactogenic composition where it is quenched with an exogenous sterol, such as cholesterol for example. Several particular forms of less reactogenic compositions wherein QS21 is quenched with an exogenous cholesterol exist. In a specific embodiment, the saponin /sterol is in the form of a liposome structure (WO 96/33739, Example 1). In this embodiment the liposomes suitably contain a neutral lipid, for example phosphatidylcholine, which is suitably non-crystalline at room temperature, for example eggyolk phosphatidylcholine, dioleoyl phosphatidylcholine (DOPC) or dilauryl phosphatidylcholine. The liposomes may also contain a charged lipid which increases the stability of the lipsome-QS21 structure for liposomes composed of saturated lipids. In these cases the amount of charged lipid is suitably 1-20% w/w, preferably 5-10%. The ratio of sterol to phospholipid is 1-50% (mol/mol), suitably 20-25%.

Suitable sterols include β-sitosterol, stigmasterol, ergosterol, ergocalciferol and cholesterol. In one particular embodiment, the adjuvant composition comprises cholesterol as sterol. These sterols are well known in the art, for example cholesterol is disclosed in the Merck Index, 11th Edn., page 341, as a naturally occurring sterol found in animal fat.

Where the active saponin fraction is QS21, the ratio of QS21 : sterol will typically be in the order of 1:100 to 1:1 (w/w), suitably between 1:10 to 1:1 (w/w), and preferably 1:5 to 1:1 (w/w). Suitably excess sterol is present, the ratio of QS21:sterol being at least 1:2 (w/w). In one embodiment, the ratio of QS21:sterol is 1:5 (w/w). The sterol is suitably cholesterol.

In one embodiment, the invention provides a dose of an immunogenic composition comprising immunologically active saponin, preferably QS21, at a level of about 1 - about 70 µg per dose, for example at an amount of about 50 µg.

In one embodiment, the invention provides a dose of an immunogenic composition comprising immunologically active saponin, preferably QS21, at a level of 60µg or less, for example between 1 and 60µg. In one embodiment, the dose of the immunogenic composition comprises QS21 at a level of approximately around 50 µg, for example between 45 and 55 µg, suitably between 46 - 54 µg or between 47 and 53 µg or between 48 and 52 µg or between 49 and 51 µg, or 50 µg.

In another embodiment the dose of the immunogenic composition comprises QS21 at a level of around 25 µg, for example between 20 - 30 µg, suitably between 21 - 29 µg or between 22 and 28 µg or between 23 and 27 µg or between 24 and 26 µg, or 25 µg.

In another embodiment, the dose of the immunogenic composition comprises QS21 at a level of around 10 µg per, for example between 5 and 15 µg, suitably between 6 and 14 µg, for example between 7 and 13 µg or between 8 and 12 µg or between 9 and 11 µg, or 10µg.

Specifically, a 0.5 ml vaccine dose volume contains 25 µg or 50 µg of QS21 per dose. Specifically, a 0.5 ml vaccine dose volume contains 50 µg of QS21 per dose.

In compositions comprising a lipopolysaccharide, the lipopolysaccharide may be present at an amount of about 1 - about 70 µg per dose, for example at an amount of about 50 µg.

The lipopolysaccharide may be a non-toxic derivative of lipid A, particularly monophosphoryl lipid A or more particularly 3-Deacylated monophoshoryl lipid A (3D - MPL).

3D-MPL is sold under the name MPL by GlaxoSmithKline Biologicals S.A. and is referred throughout the document as MPL or 3D-MPL. See, for example, US Patent Nos. 4,436,727; 4,877,611; 4,866,034 and 4,912,094. 3D-MPL primarily promotes CD4+ T cell responses with an IFN-γ (Th1) phenotype. 3D-MPL can be produced according to the methods disclosed in GB 2 220 211 A. Chemically it is a mixture of 3-deacylated monophosphoryl lipid A with 3, 4, 5 or 6 acylated chains. Preferably in the compositions of the present invention small particle 3D-MPL is used. Small particle 3D-MPL has a particle size such that it may be sterile-filtered through a 0.22µm filter. Such preparations are described in WO 94/21292.

The invention therefore provides a dose of an immunogenic composition comprising lipopolysaccharide, preferably 3D-MPL, at a level of 75µg or less, for example between 1 and 60µg.

In one embodiment, the dose of the immunogenic composition comprises 3D-MPL at a level of around 50 µg, for example between 45 - 55 µg, suitably between 46 - 54 µg or between 47 and 53 µg or between 48 and 52 µg or between 49 and 51 µg, or 50 µg.

In one embodiment, the dose of the immunogenic composition comprises 3D-MPL at a level of around 25 µg, for example between 20 - 30 µg, suitably between 21 - 29 µg or between 22 and 28 µg or between 23 and 27 µg or between 24 and 26 µg, or 25 µg.

In another embodiment, the dose of the immunogenic composition comprises 3D-MPL at a level of around 10 µg, for example between 5 and 15 µg, suitably between 6 and 14 µg, for example between 7 and 13 µg or between 8 and 12 µg or between 9 and 11 µg, or 10µg.

In one embodiment, the volume of the dose is 0.5 ml. In a further embodiment, the immunogenic composition is in a volume suitable for a dose which volume is higher than 0.5 ml, for example 0.6, 0.7, 0.8, 0.9 or 1 ml. In a further embodiment, the human dose is between 1 ml and 1.5 ml.

Specifically, a 0.5 ml vaccine dose volume contains 25 µg or 50 µg of 3D-MPL per dose. Specifically, a 0.5 ml vaccine dose volume contains 50 µg of 3D-MPL per dose.

The dose of the immunogenic composition according to any aspect of the invention suitably refers to human dose. By the term "human dose" is meant a dose which is in a volume suitable for human use. Generally this is between 0.3 and 1.5 ml. In one embodiment, a human dose is 0.5 ml. In a further embodiment, a human dose is higher than 0.5 ml, for example 0.6, 0.7, 0.8, 0.9 or 1 ml. In a further embodiment, a human dose is between 1 ml and 1.5 ml.

Suitable compositions of the invention are those wherein liposomes are initially prepared without MPL (as described in WO 96/33739), and MPL is then added, suitably as small particles of below 100 nm particles or particles that are susceptible to sterile filtration through a 0.22 µm membrane. The MPL is therefore not contained within the vesicle membrane (known as MPL out). Compositions where the MPL is contained within the vesicle membrane (known as MPL in) also form an aspect of the invention. The polypeptide comprising a C. *difficile* toxin A fragment and / or a C. *difficile* toxin B fragment can be contained within the vesicle membrane or contained outside the vesicle membrane.

In a specific embodiment, QS21 and 3D-MPL are present in the same final concentration per dose of the immunogenic composition i.e. the ratio of QS21:3D-MPL is 1:1. In one aspect of this embodiment, a dose of immunogenic composition comprises a final level of 25 µg of 3D-MPL and 25 µg of QS21 or 50 µg of 3D-MPL and 50 µg of QS21.

In one embodiment, the adjuvant includes an oil-in-water emulsion. In one embodiment the adjuvant comprises an oil in water emulsion, wherein the oil in water emulsion comprises a metabolisable oil, a tocol and an emulsifier. For example, the oil-in-water emulsion can include an oil phase that incorporates a metabolisable oil, and an additional oil phase component, such as a tocol. The oil-in-water emulsion may also contain an aqueous component, such as a buffered saline solution (e.g., phosphate buffered saline). In addition, the oil-in-water emulsion typically contains an emulsifier. In one embodiment, the metabolisable oil is squalene. In one embodiment, the tocol is alpha-tocopherol. In one embodiment, the emulsifier is a nonionic surfactant emulsifier (such as polyoxyethethylene sorbitan monooleate, Polysorbate® 80 , TWEEN80™). In exemplary embodiments, the oil-in-water emulsion contains squalene and alpha tocopherol in a ratio which is equal or less than 1 (w/w).

The metabolisable oil in the oil-in-water emulsion may be present in an amount of 0.5-10mg. The tocol in the oil-in-water emulsion may be present in an amount of 0.5 - 11 mg. The emulsifying agent may be present in an amount of 0.4 - 4 mg,

In order for any oil in water composition to be suitable for human administration, the oil phase of the emulsion system has to comprise a metabolisable oil. The meaning of the term metabolisable oil is well known in the art. Metabolisable can be defined as 'being capable of being transformed by metabolism' (Dorland's Illustrated Medical Dictionary, W.B. Sanders Company, 25th edition (1974)). The oil may be any vegetable oil, fish oil, animal oil or synthetic oil, which is not toxic to the recipient and is capable of being transformed by metabolism. Nuts, seeds, and grains are common sources of vegetable oils. Synthetic oils are also part of this invention and can include commercially available oils such as NEOBEE® (caprylic/capric triglycerides made using glycerol from vegetable oil sources and medium-chain fatty acids (MCTs) from coconut or palm kernel oils) and others. A particularly suitable metabolisable oil is squalene. Squalene (2,6,10,15,19,23-Hexamethyl-2,6,10,14,18,22-tetracosahexaene) is an unsaturated oil which is found in large quantities in shark-liver oil, and in lower quantities in olive oil, wheat germ oil, rice bran oil, and yeast, and is a particularly preferred oil for use in this invention. Squalene is a metabolisable oil by virtue of the fact that it is an intermediate in the biosynthesis of cholesterol (Merck index, 10th Edition, entry no.8619).

Suitably the metabolisable oil is present in the adjuvant composition in an amount of 0.5-10 mg, preferably 1-10, 2-10, 3-9, 4-8, 5-7, or 5-6 mg (e.g. 2-3, 5-6, or 9-10mg), specifically about 5.35 mg or about 2.14 mg per dose.

Tocols are well known in the art and are described in EP0382271. Suitably the tocol is alpha-tocopherol or a derivative thereof such as alpha-tocopherol succinate (also known as vitamin E succinate). Said tocol is suitably present in an amount of 0.5-11 mg, preferably 1-11, 2-10, 3-9, 4-8, 5-7, 5-6 mg (e.g. 10-11, 5-6, 2.5-3.5 or 1-3 mg). In a specific embodiment the tocol is present in an amount of about 5.94 mg or about 2.38 mg per dose.

The oil in water emulsion further comprises an emulsifying agent. The emulsifying agent may suitably be polyoxyethylene sorbitan monooleate. In a particular embodiment the emulsifying agent may be Polysorbate® 80 (Polyoxyethylene (20) sorbitan monooleate) or Tween® 80.

Said emulsifying agent is suitably present in the adjuvant composition in an amount of 0.1-5, 0.2-5, 0.3-4, 0.4-3 or 2-3 mg (e.g. 0.4-1.2, 2-3 or 4-5 mg) emulsifying agent. In a specific embodiment the emulsifying agent is present in an amount of about 0.97 mg or about 2.425 mg.

In one embodiment, the amounts of specific components present in the composition are the amounts present in a 0.5 ml human dose. In a further embodiment, the immunogenic composition is in a volume suitable for a human dose which volume is higher than 0.5 ml, for example 0.6, 0.7, 0.8, 0.9 or 1 ml. In a further embodiment, the human dose is between 1 ml and 1.5 ml.

Where the adjuvant is in a liquid form and is to be combined with a liquid form of a polypeptide composition, the adjuvant composition in a human dose will be a fraction of the intended final volume of the human dose, for example approximately half of the intended final volume of the human dose, for example a 350 µl volume for an intended human dose of 0.7ml, or a 250 µl volume for an intended human dose of 0.5 ml. The adjuvant composition is diluted when combined with the polypeptide antigen composition to provide the final human dose of vaccine. The final volume of such dose will of course vary dependent on the initial volume of the adjuvant composition and the volume of polypeptide antigen composition added to the adjuvant composition. In an alternative embodiment, a liquid adjuvant is used to reconstitute a lyophilised polypeptide composition. In this embodiment, the human dose of the adjuvant composition is approximately equal to the final volume of the human dose. The liquid adjuvant composition is added to the vial containing the lyophilised polypeptide composition. The final human dose can vary between 0.5 and 1.5 ml.

The method of producing oil-in-water emulsions is well known to the person skilled in the art. Commonly, the method comprises mixing the tocol-containing oil phase with a surfactant such as a PBS/ polyoxyethylene sorbitan monooleate solution, followed by homogenisation using a homogenizer. It would be clear to a man skilled in the art that a method comprising passing the mixture twice through a syringe needle would be suitable for homogenising small volumes of liquid. Equally, the emulsification process in microfluidiser (M110S Microfluidics machine, maximum of 50 passes, for a period of 2 minutes at maximum pressure input of 6 bar (output pressure of about 850 bar)) could be adapted by the man skilled in the art to produce smaller or larger volumes of emulsion. The adaptation could be achieved by routine experimentation comprising the measurement of the resultant emulsion until a preparation was achieved with oil droplets of the required diameter.

In an oil in water emulsion, the oil and emulsifier should be in an aqueous carrier. The aqueous carrier may be, for example, phosphate buffered saline.

Preferably the oil-in-water emulsion systems of the present invention have a small oil droplet size in the sub-micron range. Suitably the droplet sizes will be in the range 120 to 750 nm, more preferably sizes from 120 to 600 nm in diameter. Most preferably the oil-in water emulsion contains oil droplets of which at least 70% by intensity are less than 500 nm in diameter, more preferably at least 80% by intensity are less than 300 nm in diameter, more preferably at least 90% by intensity are in the range of 120 to 200 nm in diameter.

In one embodiment, the immunogenic composition is not 3µg or 10 µg of any of SEQ ID Nos. 1 to 7 combined with an adjuvant comprising an oil in water emulsion having 0.125 mL SB62 emulsion (Total volume), 5.35 mg squalene, 5.94 mg DL-α-tocopherol and 2.425 mg polysorbate 80 per 0.5 ml dose. In one embodiment, the immunogenic composition is not 3µg or 10 µg of any of SEQ ID Nos. 1 to 7 combined with an adjuvant comprising an oil in water emulsion 5.35 mg squalene, 5.94 mg DL-α-tocopherol and 2.425 mg polysorbate 80 per 0.5 ml dose. In one embodiment, the immunogenic composition does not contain an adjuvant comprising a oil in water emulsion having squalene, DL-α-tocopherol and polysorbate 80.

### Immunogenic compositions and vaccines of the invention

In one embodiment the immunogenic composition has a volume of 0.5 to 1.5 ml.

In one embodiment the immunogenic composition further comprises additional antigens. In one embodiment the additional antigens are antigens derived from a bacterium selected from the group consisting of *S.pneumoniae, H.influenzae, N.meningitidis, E.coli, M.catarrhalis, Clostridium tetani* (tetanus), *Corynebacterium diphtheria* (diphtheria), *Bordetella pertussis* (pertussis), *S.epidermidis,* enterococci, *S.aureus,* and *Pseudomonas aeruginosa.*

In a further embodiment the immunogenic composition of the invention may comprise further antigens from C. *difficile,* for example, the S-layer proteins (WO01/73030). Optionally the immunogenic composition further comprises a saccharide from C. *difficile.*

There is further provided a vaccine comprising an immunogenic composition of the invention and a pharmaceutically acceptable excipient.
The vaccine preparations containing immunogenic compositions of the present invention may be used to protect a mammal susceptible to C. *difficile* infection or treat a mammal with a C. *difficile* infection, by means of administering said vaccine via systemic or mucosal route. These administrations may include injection via the intramuscular, intraperitoneal, intradermal or subcutaneous routes; or via mucosal administration to the oral/alimentary, respiratory, genitourinary tracts. Although the vaccine of the invention may be administered as a single dose, components thereof may also be co-administered together at the same time or at different times (for instance pneumococcal saccharide conjugates could be administered separately, at the same time or 1-2 weeks after the administration of the any bacterial protein component of the vaccine for coordination of the immune responses with respect to each other). In addition to a single route of administration, 2 different routes of administration may be used. For example, saccharides or saccharide conjugates may be administered intramuscularly (IM) or intradermally (ID) and bacterial proteins may be administered intranasally (IN) or intradermally (ID). In addition, the vaccines of the invention may be administered IM for priming doses and IN for booster doses.

The content of toxins in the vaccine will typically be in the range 1-250µg, preferably 5-50µg, most typically in the range 5 - 25µg. Following an initial vaccination, subjects may receive one or several booster immunizations adequately spaced. Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

In one aspect of the invention is provided a vaccine kit, comprising a vial containing an immunogenic composition of the invention, optionally in lyophilised form, and further comprising a vial containing an adjuvant as described herein. It is envisioned that in this aspect of the invention, the adjuvant will be used to reconstitute the lyophilised immunogenic composition.

A further aspect of the disclosure is a method of preventing or treating C. *difficile* infection comprising administering to the host an immunoprotective dose of the immunogenic composition or vaccine or kit of the invention. In one embodiment of the disclosure there is provided a method of preventing or treating primary and/or recurrence episodes of C. *difficile* infection comprising administering to the host an immunoprotective dose of the immunogenic composition or vaccine or kit of the invention.

In one embodiment of the invention there is provided an immunogenic composition or vaccine of the invention for use in the treatment or prevention of a condition or disease caused by *C.difficile.* "*C.difficile* disease" means a disease caused wholly or in part by C. *difficile.* In a further embodiment of the invention there is provided an immunogenic composition or vaccine of the invention for use in the treatment or prevention of a condition or disease caused wholly or in part by a strain of *C.difficile* selected from the group consisting of 078, 019, 023, 027, 033, 034, 036, 045, 058, 059, 063, 066, 075, 078, 080, 111, 112, 203, 250 and 571. Preferably the strain is strain 078.

As used herein "treatment" means the prevention of occurrence of symptoms of the condition or disease in a subject, the prevention of recurrence of symptoms of the condition or disease in a subject, the delay of recurrence of symptoms of the condition or disease in a subject, the decrease in severity orfrequency of symptoms of the condition or disease in a subject, slowing or eliminating the progression of the condition and the partial or total elimination of symptoms of the disease or condition in a subject.

In a further aspect of the invention there is provided a method of preventing or treating C. *difficile* disease comprising administering the immunogenic composition of the invention or the vaccine of the invention to a mammalian subject such as a human subject. In a further embodiment the disease is a disease caused by a strain of *C*. *difficile* selected from the group consisting of 078, 019, 023, 027, 033, 034, 036, 045, 058, 059, 063, 066, 075, 078, 080, 111, 112, 203, 250 and 571. Preferably the strain is strain 078.

### General

"Around" or "approximately" are defined as within 10% more or less of the given figure for the purposes of the invention.

The term "comprises" means "includes." Thus, unless the context requires otherwise, the word "comprises," and variations such as "comprise" and "comprising" will be understood to imply the inclusion of a stated compound or composition (e.g., nucleic acid, polypeptide, antigen) or step, or group of compounds or steps, but not to the exclusion of any other compounds, composition, steps, or groups thereof. The abbreviation, "e.g." is derived from the Latin exempli gratia, and is used herein to indicate a non-limiting example. Thus, the abbreviation "e.g." is synonymous with the term "for example."

The amino acid numbering used herein is derived from the sequences for CDTa, CDTb, Toxin A and Toxin B presented herein as SEQ ID NO: 1, SEQ ID NO: 3, SEQ ID NO: 29 and SEQ ID NO: 30, respectively, which are to be considered as reference sequences for these proteins.

Embodiments herein relating to "vaccine compositions" of the invention are also applicable to embodiments relating to "immunogenic compositions" of the invention, and vice versa.

Unless otherwise explained, all technical and scientific terms used herein have the same meaning as commonly understood by one of ordinary skill in the art to which this disclosure belongs. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

The singular terms "a," "an," and "the" include plural referents unless context clearly indicates otherwise. Similarly, the word "or" is intended to include "and" unless the context clearly indicates otherwise. The term "plurality" refers to two or more. It is further to be understood that all base sizes or amino acid sizes, and all molecular weight or molecular mass values, given for nucleic acids or polypeptides are approximate, and are provided for description. Additionally, numerical limitations given with respect to concentrations or levels of a substance, such as an antigen, may be approximate.

In order that this invention may be better understood, the following examples are set forth. These examples are for purposes of illustration only, and are not to be construed as limiting the scope of the invention in any manner.

### EXAMPLES

The AS01B adjuvant referred to is an adjuvant having 50 µg QS21 presented in the form of a liposome, 50 µg 3D-MPL, 0.25 mg cholesterol and 1.0 mg DOPC per 0.5ml dose. A dose of 50µl suitable for immunizing mice contains 5µg QS21, 5µg 3D-MPL, 0.025mg cholesterol and 0.1mg DOPC.

### Example 1: Design of binary toxin antigens

The Binary Toxin (other name: ADP-ribosyltransferase toxin) is composed by two components: the enzymatic component, named CDTa and the transport and binding component, named CDTb.

Based on literature data and information available for components of other bacterial binary toxins, CDTb could be divided into five domains. The first one is the prodomain, its cleavage by an enzyme having a chymotrypsin activity allows the heptamerisation of the mature protein. The second domain allows the binding to CDTa. The third and fourth ones are involved in the oligomerisation and membrane insertion. Finally, the last domain is the host cell receptor binding domain.

### CDTb mature

In order to avoid the chymotrypsin activation step in the CDTb process, it was tried to express only the mature CDTb protein (without its signal peptide and prodomain).

In the literature *(*Protein Expression and Purification, 2010, vol. 74 : 42-48), the mature CDTb was described as starting at Leucine 210 of SEQ ID NO: 3. This mature CDTb was named CDTb".

Other experimental data suggest that the activated CDTb starts at Serine 212 of SEQ ID NO: 3. This result was supported by analysis of a 3D modelised structure of CDTb. This model was built using SwissModel (Bioinformatics, 2006, vol. 22 : 195-201). The template used for the homology modelling was the B component of *Bacillus anthracis,* named Protective Antigen or PA (Protein Data Bank accession number: 3TEW).

### CDTb receptor-binding domain alone

The 3D homology structure model obtained for CDTb using the PA antigen from *B. anthraxis* is accurate for the four first domains of CDTb but not for the receptor-binding domain (these domains of CDTb and PA are too different). To design constructs expressing the receptor-binding domain alone, the C-terminal part of the fourth domain was analysed on the 3D structure model in order to decide where the last domain will start.

Two versions of the CDTb-receptor binding domain were designed. In the first one, this domain starts just after the modelised 3D structure of the fourth domain. In this version, the CDTb-receptor-binding domain will probably have a long flexible peptide in its N-terminal part. The second version of the receptor-binding domain starts where the 2D predicted structures performed on the C-terminal part of CDTb (predictions done using Psipred program, Bioinformatics, 2000, vol. 16 : 404-405) become more compacts after a lack of predicted secondary structures. This could indicate the beginning of a new structural domain. In this second version, no flexible peptide is present at the N-terminal part of the isolated CDTb receptor-binding domain.

### CDTb Ca²⁺ binding motif mutation

Following literature information, mutations in the Ca²⁺ binding domain of the B component of Iota toxin of *Clostridium perfringens* (Ib) abolish the binding with the A component of this binary toxin (Ia). These mutations could be very interesting in the case of a vaccine composition containing a mixture of mature CDTb protein and a wild type CDTa protein.
Using multiple protein sequence alignment, these mutations were located on the CDTb sequence and mutated. It concerns residues Asp²²⁰, Asp²²² and Asp²²⁴ of SEQ ID NO: 3. They were mutated into Ala residues.

### CDTb prodomain

In order to try to decrease the degradation issues observed with construct C55 (CDTb with the prodomain removed) in gel, some co-expression tests were evaluated. The working hypothesis of doing that is to improve the folding of the mature CDTb.
Two limits of prodomain were proposed. The first one starts at residue 43 of CDTb of SEQ ID NO: 3 (after the signal peptide cleavage) and finishes at residue Met²¹¹ (given that the experimentally determined first residue of the mature CDTb is Ser²¹²). The second prodomain was designed based on the predicted 3D structure of CDTb. The linker existing between the prodomain and the first structural domain of the mature CDTb protein is removed in this construct.

### Example 2 - Cloning, expression and purification of C. difficile CDTb protein

### Expression plasmid and recombinant strain: C37 and C55 (as set out in Table A).

Genes encoding the truncated protein of CDTb without signal peptide (Pro-CDTb', C37) and a His tag in C-term were cloned into the pGEX-6p1 expression vector (GE Healthcare) using the BamHI/XhoI restriction sites using standard procedures. This vector included GST (Gluthathione-S-transferase) as fusion partner in N-terminal of CDTb'(GST-Pro-CDTb'). The final construct was generated by the transformation of E. *coli* strain BL21 (DE3) with the recombinant expression vector according to standard method with CaCl2-treated cells (Hanahan D. « Plasmid transformation by Simanis. » In Glover, D. M. (Ed), DNA cloning. IRL Press London. (1985): p. 109-135.).

Genes encoding the truncated protein of CDTb without signal peptide and prodomain (CDTb": C55) and a His tag in C-term were cloned into the pET24b(+) expression vector (Novagen) using the Ndel/Xhol restriction sites using standard procedures. Final constructs were generated by the transformation of *E*. *coli* B834 (DE3) modified strain with the appropriate recombinant expression vectors according to standard method with CaCl2-treated cells (Hanahan D. « Plasmid transformation by Simanis. » In Glover, D. M. (Ed), DNA cloning. IRL Press London. (1985): p. 109-135.).

### Host Strain

BL21(DE3). BL21(DE3) is a non-methionine auxotroph derivative of B834. Strains having the designation (DE3) are lysogenic for a λ prophage that contains an IPTG inducible T7 RNA polymerase. λ DE3 lysogens are designed for protein expression from pET vectors This strain is also deficient in the *Ion* and *ompT* proteases.

Genotype : *E.coli* BL21::DE3 strain, F⁻ *ompT hsdS*_{B}(r_{B}⁻ m_{B}⁻) *gal dcm* (*DE3*).

B834 is the parental strain for BL21. These protease-deficient hosts are methionine auxotrophs. λ DE3 lysogens are designed for protein expression from pET vectors This strain is also deficient in the *Ion* and *ompT* proteases.

Modification: Including PGL gene to avoid phosphogluconoylation in the biotin locus (Strain is auxotroph for biotin).
Genotype : B834 ::DE3 strain, F- *ompT hsdS*B(rB - mB-) *gal dcm met* (DE3)
Modification : Δ(bioA-bioD)::PGL

### Expression of the recombinant proteins:

*E.coli* transformants were stripped from agar plate and used to inoculate 200 ml of LBT broth ± 1% (w/v) glucose +/- kanamycin (50 µg/ml) or ampicillin (100 µg/ml) to obtain O.D.₆₀₀ₙₘ between 0.1 -0.2. Cultures were incubated overnight at 37 °C, 250 RPM.

Overnight culture were diluted to 1:20 in 500 ml of LBT medium containing +/- kanamycin (50 µg/ml) or ampicillin (100 µg/ml) and grown at 37°C at a stirring speed of 250 rpm until O.D.₆₂₀ reached 0.5/0.6.

At an O.D.at 600nm of around 0.6, cultures were cooled down before inducing the expression of the recombinant protein by addition of 1 mM isopropyl β-D-1-thiogalactopyranoside (IPTG; EMD Chemicals Inc., catalogue number: 5815) and incubated overnight at 23 °C, 250 RPM.

After the overnight inductions (around 16 hours), O.D. at 600nm were evaluated after induction and cultures were centrifuged at 14 000 RPM for 15 minutes and pellets were frozen at -20°C separately.

### Purification

### C37 (SEQ ID NO: 5)

The bacterial pellet was re-suspended in 50mM bicine buffer (pH 8.0) containing 500mM NaCl , 5mM TCEP (Thermo Scientific Pierce, (2-carboxyethyl)phosphine hydrochloride) and a mixture of protease inhibitor (Complete, Roche). Bacteria were lysed using a French Press system 3 X 20 000 PSI. Soluble (supernatant) and insoluble (pellet) components were separated by centrifugation at 20 000g for 30 min at 4°C.

The 6-His tagged-protein was purified under native conditions on IMAC. The soluble components were loaded on a 5ml GE Histrap column (GE) pre-equilibrated with the same buffer used to bacterial re-suspension. After loading on the column, the column was washed with a 50mM bicine buffer pH8.0, containing 150mM NaCl, 25mM imidazole, 1mM TCEP.

Elution was performed using a 50mM bicine buffer pH8.0 containing 150mM NaCl, 250mM imidazole, 1mM TCEP.

After desalting step (BIORAD Bio-Gel P6 Desalting) in 50mM bicine buffer pH8.0 containing 150mM NaCl and 1mM TCEP, the product was treated (overnight at 4°C) with PreScission protease (GE-Healthcare) in order to cleave the GST tag. After overnight treatment, 0.2% Tween 20 was added to the digestion mixture.

Then the protein was passed through a GST affinity column (GE GSTrap FF) pre-equilibrated with buffer 50mM bicine buffer pH8.0 containing 150mM NaCl, 1mM TCEP, 0.2% tween20 and 20mM reduced glutation, in order to remove the cleaved tag, un-cleaved fusion protein and the PreScission protease.

The GST-free protein was collected in the flow through and loaded again on a 5ml GE Histrap column (GE) pre-equilibrated with 50mM bicine buffer pH8.0 containing 150mM NaCl, 1mM TCEP, 0.2% tween20. After loading on the column, the column was washed with a 50mM bicine buffer pH8.0, containing 150mM NaCl, 0.2% tween20, 1mM TCEP and 10mM imidazole. Elution was performed using a 50mM bicine buffer pH8.0 containing 150mM NaCl, 0.2% tween20, 1mM TCEP and 500mM imidazole.

After desalting step (BIORAD Bio-Gel P6 Desalting) in 50mM bicine buffer pH8.0 containing 150 mM NaCl, 1mM TCEP and 0.2% tween 20, the product was treated with α-chymotrypsin (from bovine pancreas - Sigma), followed by trypsin inhibitor treatment (from egg white-Sigma). The complete activation of CDTb by chymotrypsin was monitored by SDS-PAGE.

The fully activated product was loaded on SEC chromatography (SUPERDEX™75) in 50mM bicine buffer pH8.0 containing 300mM NaCl, 1mM TCEP. Fractions containing CDTb antigen were selected on the basis of purity by SDS-PAGE. Protein concentration was determined using Lowry RC/DC Protein Assay of BioRad. The purified bulk was sterile-filtered on 0.22 µm and stored at -80°C.

### C55 (SEQ ID NO: 7)

The bacterial pellet was re-suspended in 50mM bicine buffer (pH 8.0) containing 150 mM NaCl, 5mM TCEP (Thermo Scientific Pierce, (2-carboxyethyl) phosphine hydrochloride), 0.4% empigen and a mixture of protease inhibitors (Complete, Roche). Bacteria were lysed using a French Press system 3 X 20 000 PSI. Soluble (supernatant) and insoluble (pellet) components were separated by centrifugation at 20 000g for 30 min at 4°C.

The 6-His tagged-protein was purified under native conditions on IMAC. The soluble components were loaded on a 5ml GE Histrap column (GE) pre-equilibrated with 50mM bicine buffer (pH 8.0) containing 150mM NaCl, 0.15% empigen, 1mM TCEP. After loading on the column, the column was washed with a 50mM bicine buffer pH8.0, containing 150 mM NaCl, 0.2% tween 20, 20mM imidazole and 1mM TCEP. Elution was performed using a 50mM bicine buffer pH8.0 containing 150mM NaCl, 0.2% tween 20, 500mM imidazole and 1mM TCEP.

After desalting step (BIORAD Bio-Gel P6 Desalting) in 50mM bicine buffer pH8.0 containing 300mM NaCl, 1mM TCEP the product was loaded on SEC chromatography (SUPERDEX™ 75) in the same buffer. Fractions containing CDTb antigen were selected on the basis of purity by SDS-PAGE. Protein concentration was determined using Lowry RC/DC Protein Assay of BioRad. The purified bulk was sterile-filtered on 0.22 µm and stored at -80°C.

### Example 3: Cloning, expression and purification of CDTa protein

### Expression plasmid and recombinant strain: CDTa full length (C34, C44, C49, C50, C54, C67, C68, C69, C107, C108, C110 as set out in Table A)

Genes encoding the protein of full length without signal peptide of CDTa with and without mutations (see Table A) and a His tag in C-term were cloned into the pET24b(+) expression vector (Novagen) using the Ndel/Xhol restriction sites using standard procedures. Final constructs were generated by the transformation of *E. coli* strain HMS174 (DE3) or BLR (DE3) pLysS (C34) with each recombinant expression vector separately according to standard method with CaCl2-treated cells (Hanahan D. « Plasmid transformation by Simanis. » In Glover, D. M. (Ed), DNA cloning. IRL Press London. (1985): p. 109-135.).

### Host strain:

HMS 174 (DE3). HMS174 strains provide the *recA* mutation in a K-12 background. Strains having the designation (DE3) are lysogenic for a λ prophage that contains an IPTG inducible T7 RNA polymerase. λ DE3 lysogens are designed for protein expression from pET vectors **Genotype:** F⁻ *recA1 hsdR*(r_{K12}*⁻* m_{K12}⁺) (Rif^{R}).

BLR(DE3) pLysS. BLR is a recA derivative of BL21. Strains having the designation (DE3) are lysogenic for a λ prophage that contains an IPTG inducible T7 RNA polymerase. λ DE3 lysogens are designed for protein expression from pET vectors This strain is also deficient in the *Ion* and *ompT* proteases, pLysS strains express T7 lysozyme which further suppress basal expression of the T7 RNA polymerase prior to induction.

Genotype : *E.coli* BLR::DE3 strain, F⁻ *ompT hsdS*_{B}(r_{B}⁻ m_{B}⁻) *gal dcm* (*DE3*) Δ(srl-recA)306::Tn*10* pLysS (Cam^{R}, Tet^{R}).

### Expression of the recombinant proteins:

*E.coli* transformants were stripped from agar plate and used to inoculate 200 ml of LBT broth ± 1% (w/v) glucose + kanamycin (50 µg/ml) to obtain O.D.600nm between 0.1 -0.2. Cultures were incubated overnight at 37 °C, 250 RPM.

Each overnight culture were diluted to 1:20 in 500 ml of LBT medium containing kanamycin (50 µg/ml) and grown at 37°C at a stirring speed of 250 rpm until O.D.620 reached 0.5/0.6.

At O.D.600nm around 0.6, the cultures were cooled down before inducing the expression of the recombinant protein by addition of 1 mM isopropyl β-D-1-thiogalactopyranoside (IPTG; EMD Chemicals Inc., catalogue number: 5815) and incubated overnight at 23 °C, 250 RPM.

After overnight induction (around 16 hours), O.D.₆₀₀ₙₘ were evaluated after induction and culture were centrifuged at 14 000 RPM for 15 minutes and pellets were frozen at -20°C separately.

### Expression plasmid and recombinant strain: CDTa- N-term (C49 and C50 as set out in Table A)

Genes encoding protein of N-terminal, without signal peptide of CDTa (see Table A) and a His tag in C-term were cloned into the pET24b(+) expression vector (Novagen) using the Ndel/Xhol restriction sites using standard procedures. Final constructs were generated by the transformation of *E*. *coli* strain HMS174 (DE3) with each recombinant expression vectors separately according to standard method with CaCl2-treated cells (Hanahan D. « Plasmid transformation by Simanis. » In Glover, D. M. (Ed), DNA cloning. IRL Press London. (1985): p. 109-135.).

### Host strain:

HMS 174 (DE3). HMS174 strains provide the *recA* mutation in a K-12 background. Strains having the designation (DE3) are lysogenic for a λ prophage that contains an IPTG inducible T7 RNA polymerase. λ DE3 lysogens are designed for protein expression from pET vectors **Genotype:** F⁻ *recA1 hsdR*(r_{K12}*⁻* m_{K12}⁺) (Rif^{R}).

### Expression of the recombinant proteins:

*E.coli* transformants were stripped from agar plate and used to inoculate 200 ml of LBT broth ± 1% (w/v) glucose + kanamycin (50 µg/ml) to obtain O.D.600nm between 0.1 -0.2. Cultures were incubated overnight at 37 °C, 250 RPM.

This overnight culture was diluted to 1:20 in 500 ml of LBT medium containing kanamycin (50 µg/ml) and grown at 37°C at a stirring speed of 250 rpm until O.D.620 reached 0.5/0.6.

At O.D.600nm around 0.6, the culture was cooled down before inducing the expression of the recombinant protein by addition of 1 mM isopropyl β-D-1-thiogalactopyranoside (IPTG; EMD Chemicals Inc., catalogue number: 5815) and incubated overnight at 23 °C, 250 RPM.

After overnight induction (around 16 hours), O.D.₆₀₀ₙₘ was evaluated after induction and culture was centrifuged at 14 000 RPM for 15 minutes and pellets were frozen at -20°C separately.

### Purification

The following procedure was used to purify constructs C34, C44, C49, C50, C54, C67, C69, C107 and C110.
The bacterial pellets were re-suspended in 20mM or 50mM bicine buffers (pH 7.5 or pH 8.0), containing 500mM NaCl, 0mM or 5mM TCEP (Thermo Scientific Pierce, (2-carboxyethyl)phosphine hydrochloride) and a mixture of protease inhibitors (Complete, Roche, without EDTA). Bacteria were lysed using a French Press system 3 X 20 000 PSI. Soluble (supernatant) and insoluble (pellet) components were separated by centrifugation at 20 000g for 30 min at 4°C.

The 6-His tagged-proteins were purified under native conditions on IMAC. The soluble components were loaded on a 5ml GE Histrap column (GE) pre-equilibrated with the same buffer used to bacterial re-suspension. After loading on the column, the column was washed with a 20mM or 50mM bicine buffer (pH7.5 or pH8.0), containing 500mM NaCl, 10mM imidazole, 5mM TCEP. Elution was performed using a 50mM bicine buffer pH7.6, 500mM NaCl, 1mM TCEP and imidazole (250mM or 500mM).

After desalting (BIORAD Bio-Gel P6 Desalting) and concentration (Amicon Ultra 10kDa) steps, the product was loaded on SEC chromatography (SUPERDEX™75 or 200) in 20mM or 50mM bicine buffer(pH7.5 or pH8.0), 150mM NaCl, 1mM TCEP, for further purification step.

Fractions containing CDTa antigen were selected on the basis of purity by SDS-PAGE. Protein concentration was determined using Lowry RC/DC Protein Assay of BioRad. The purified bulk was sterile-filtered on 0.22 µm and stored at -80°C.

### Comparative Example 4: Design, cloning, expression and purification of C. difficile CDTb protein with modified pore forming ability.

A first strategy was evaluated in order to decrease the cytotoxicity observed for CDTb alone: avoid the possibility for CDTb of modifying its structure in an endosome in order to form a pore into the membrane of this endosome. The two strategies followed in this approach were based on publication data available for B components coming from Binary toxin of *Bacillus anthracis* (C123 and C149) and *Clostridium botulinium* (C126, C152, C164 and C166).The following CDTb proteins were designed:

| Name | Length(aa)* | Location | Description | SEQ ID |
|---|---|---|---|---|
| C123 | | | pGEX-CDTb - F455R-His | SEQ ID NO:10 |
| C126 | | | pGEX-CdtB-E426A-D453A | SEQ ID NO: 11 |
| C149 | 841 | 43-876 of CDTb of SEQ ID NO: 3 | pET24b-Cdtb - F455R-His | SEQ ID NO: 25 |
| C152 | 841 | 43-876 of CDTb of SEQ ID NO: 3 | pET24b-Cdtb-E426A-D453A-His | SEQ ID NO: 26 |
| C164 | 672 | 212-876 of CDTb of SEQ ID NO: 3 | pET24b-Cdtb-w/o prdomain-F455R-His | SEQ ID NO: 43 |
| C166 | 672 | 212-876 of CDTb of SEQ ID NO: 3 | pET24b-Cdtb-w/o prodomain-E426A-D453A-His | SEQ ID NO: 44 |

| | | | | |
|---|---|---|---|---|
| * Takes into account the start Met codon and the His-tag | | | | |

### Cloning and production

Histag in C-term/ cloning in pGEX-6p1 vector in BamH1-Xho1 site / Stains B834(DE3): genotype: E.coli BL21::DE3 strain, F- ompT hsdSB(rB- mB-) gal dcm (DE3).

B834 is the parental strain for BL2. These protease-deficient hosts are methionine auxotrophs. λ DE3 lysogens are designed for protein expression from pET and pGEX vectors This strain is also deficient in the Ion and ompT proteases. Selection antibiotic: Ampicillin: 50µg/ml/ production o/n at 16C

### Purification (C123, C126, C149 and C152):

French Press with lysis buffer: 50mM Bicine-500mM NaCl, - 5mMTCEP, Complete- pH8.0 Ni-NTA GE His trap 5ml (profinia) + Desalting
Equilibrated : 50mM Bicine - 500mM NaCl, 1mMTCEP - pH8.0
Wash:50mM Bicine - 150mM NaCl, 1mMTCEP - imidazole 0mM pH8.0
Elution: 50mM Bicine - 150mM NaCl, 1mMTCEP - imidazole 250mM pH8.0
Desalting: 50mMBicine-150mMNaCl-1 mMTCEP pH8.0
Prescission Clivage (GST tag):
+375µl (500UI/250µl) : 750 UI
ON at 4°C + add Tween 20 0.2% final conc.
GSTrap FF (1ml)
Equilibrated buffer: 50mM Bicine - 150mM NaCl- 1mMTCEP - 0.2%Tween20 pH8.0
Keep the FT
Wash buffer: same as equil buffer
Elution buffer: 50mM Bicine - 150mM NaCl- 1mMTCEP- 0.2%Tween 20 -250mM Imidazole, -pH8.0
IMAC GE 5ml + Desalting
Equilibrated buffer: 50mM Bicine - 150mM NaCl- 1mMTCEP 0.2%Tween20, -pH8.0
Wash buffer: 50mM Bicine - 150mM NaCl- 1mMTCEP- 10mM imidazole 0.2%Tween20, - pH8.0
Elution buffer: 50mM Bicine - 150mM NaCl- 1mMTCEP- 250mM Imidazole - 0.2%Tween 20, -pH8.0
Desalting buffer: 50mM Bicine - 150mM NaCl- 1mMTCEP - 0.2%Tween20, -pH8.0
RC/DC dosage
Activation : Chymotrypsin treatment:
   100µg chymotrypsin (1µg/µl) active 1mg of protein
   (42mg + 3.6mg of chymotrypsin in 12.5ml final vol)
   50 min at RT
   + inactivation : chymotrypsin inhibitor 12.6mg
   SEC Superdex 200
   Equilibrated: 50mM Bicine - 150mM NaCl- 1mMTCEP, -pH8.0
   Concentration on IMAC GE 1ml + Desalting
   Equilibrated buffer: 50mM Bicine - 150mM NaCl- 1mMTCEP, -pH8.0
   Wash buffer: 50mM Bicine - 150mM NaCl- 1mMTCEP, -pH8.0
   Elution buffer: 50mM Bicine - 150mM NaCl- 1mMTCEP- 250mM Imidazole, -pH8.0
   Desalting buffer: 50mM Bicine - 150mM NaCl- 1mMTCEP, -pH8.0
   Filtration 0.22µm, RC/DC dosage

Purification of C164 was carried out in accordance with the following scheme 1:

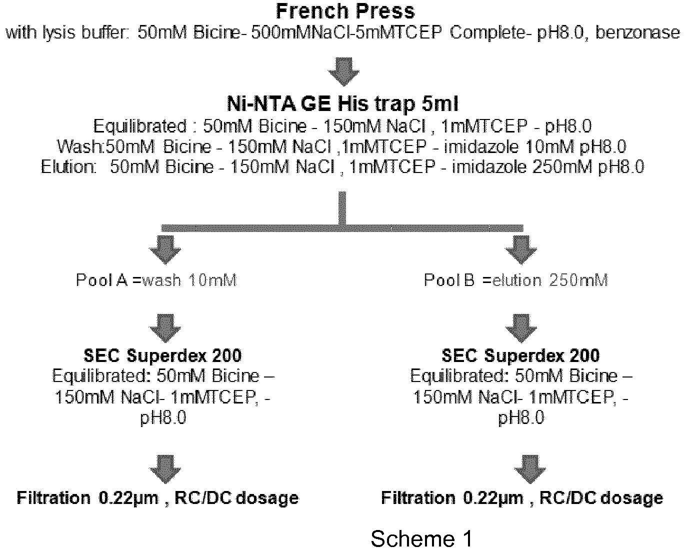

Purification of C166 was carried out in accordance with the following scheme 2:

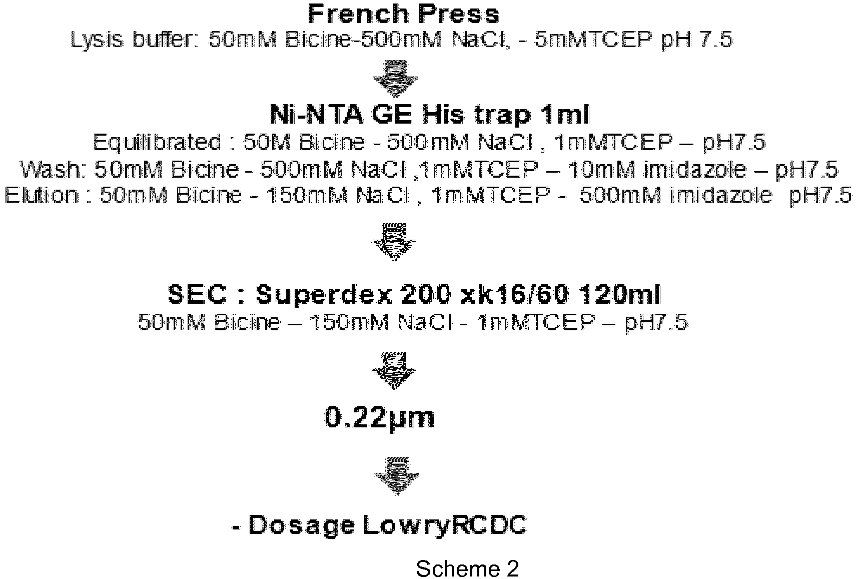

### Characterisation:

Dynamic light scattering is used to evaluate hydrodynamic radius in solution of CDTb purified proteins, in addition to provide information about homogeneity and to detect presence of high molecular weight aggregates within a protein sample. This is based on the calculation of the diffusion coefficient of the different species that are obtained by measuring the light scattering fluctuation, which depends on protein molecular size and shape, and on the other minor constituents of the sample.

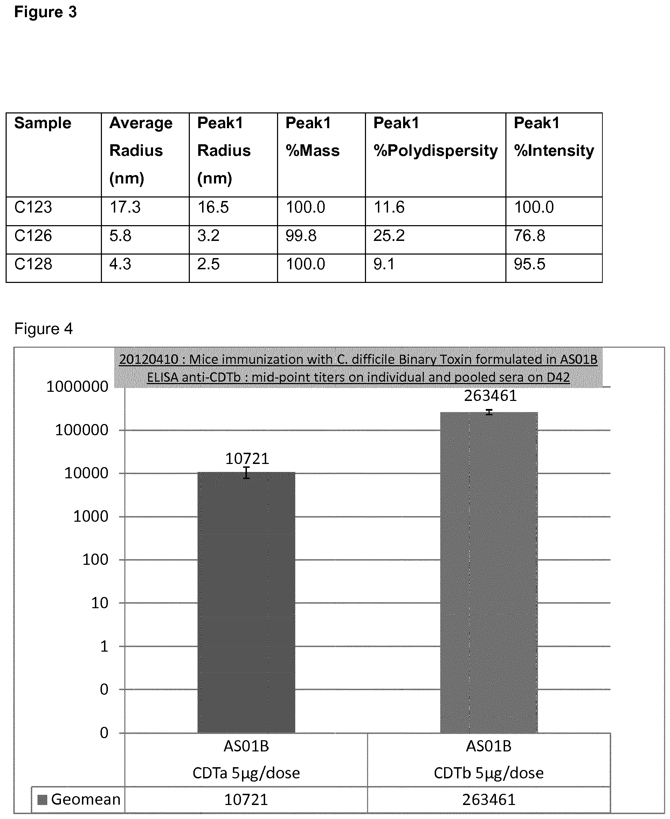

Protein samples were analysed on a Dynapro plate reader (Wyatt technology), using five acquisition of 15 seconds at 25°C
a- Buffer: 50mM Bicine - 150mM NaCl- 1mMTCEP, -pH8.0
Results are shown in Figure 3.

### Comparative Example 5: Design, cloning, expression and purification of C. difficile CDTb protein with the signal peptide and the prodomain removed and also the receptor binding domain removed and/or the CDTa binding domain removed.

The rational of expressing the different structural domains of CDTb alone was to understand which one leads to degradation and aggregation issues. Two domains were evaluated using this strategy: the receptor-binding domain and the CDTa-binding domain were removed on construct C116 and C117, respectively. The "oligomerisation" and "membrane insertion" domains of CDTb were not expressed separately because they are potentially structurally related. The following CDTb proteins were designed:

| Name | Length(aa)* | Location | Description | SEQ ID |
|---|---|---|---|---|
| C116 | 414 | 212-616 of CDTb of SEQ ID NO: 3 | pET24b-CDTb ΔRBD-His | SEQ ID NO:8 |
| C117 | 588 | 296-876 of CDTb of SEQ ID NO: 3 | pET24b-CDTb ΔCDTa bdg-His | SEQ ID NO: 9 |
| C118 | 330 | 296-616 of CDTb of SEQ ID NO: 3 | pET24b-CDTb-ΔRBD-ΔCDTa bdg-His | SEQ ID NO:37 |

| | | | | |
|---|---|---|---|---|
| * Takes into account the start Met codon and the His-tag | | | | |

### Cloning and production

Histag in C-term/ cloning in pET24b vector in Nde1-Xho1 site / Stains B834(DE3): genotype: E.coli BL21::DE3 strain, F- ompT hsdSB(rB- mB-) gal dcm (DE3).

B834 is the parental strain for BL2. These protease-deficient hosts are methionine auxotrophs. λ DE3 lysogens are designed for protein expression from pET vectors This strain is also deficient in the Ion and ompT proteases. Selection antibiotic: Kanamycin: 50µg/ml/ production o/n at 16C

### Purification and Characterisation

### C116

### French Press

Lysis buffer: 50mM Bicine-500mM NaCl, - 5mMTCEP, Complete- pH8.0

### Ni-NTA GE His trap 5ml

Equilibrated : **8M Urea** - 50mM Bicine - 500mM NaCl, 1mMTCEP - pH8.0
Wash: **8M urea** -50mM Bicine - 500mM NaCl, 1mMTCEP - 5mM imidazole - pH8.0

### Refoldind on column His trap

Gardient : 100min at 1ml/min : 8M -50mM Bicine - 150mM NaCl - 1mM TECP - pH 8.0 1mMTCEP to 0M Urea 50mM Bicine - 150mM NaCl - 1mM TECP - pH 8.0

### Elution

50mM Bicine - 150mM NaCl, 1mMTCEP - imidazole 500mM pH7.5

### SEC : Superdex 200

50mM Bicine - 150mM NaCl - 1mMTCEP - pH7.5

### 0.22µm - Dosage LowryRCDC

### C117

### French Press

with lysis buffer: 50mM Bicine-500mM NaCl, - 5mMTCEP, Complete- pH8.0

### Ni-NTA GE His trap 5ml (profinia)

Equilibrated : 50mM Bicine - 500mM NaCl, 1mMTCEP - pH8.0
Wash:50mM Bicine - 500mM NaCl, 1mMTCEP - imidazole 0mM pH8.0
Elution: 50mM Bicine - 150mM NaCl, 1mMTCEP - imidazole 500mM pH7.5

### SEC Superdex 200

Equilibrated: 50mM Bicine - 150mM NaCl- 1mMTCEP, -pH7.5

### Filtration 0.22µm, RC/DC dosage

### Comparative Example 6: Design, cloning, expression and purification of C. difficile CDTb protein with modified heptamerisation ability

A second strategy was used in order to decrease the cytotoxicity observed for CDTb alone : avoid the heptamerisation of CDTb. A 3D model of a heptameric complex of CDTb were designed. Using this model, one loop was identified as potentially interacting between monomers. Two approaches were evaluated : the remove of this loop (C127) or the mutation of residues contained in this loop that are probably involved in the interaction between monomer (C128)The following proteins were designed:

| Name | Length(aa)* | Location | Description | SEQ ID |
|---|---|---|---|---|
| C127 | 834 | 43-876 of CDTb of SEQ ID NO: 3 | pET24b-Truncated CDTb-His | SEQ ID NO: 12 |
| C128 | 841 | 43-876 of CDTb of SEQ ID NO:3 | pET24b-mutated CDTb-His | SEQ ID NO: 13 |

| | | | | |
|---|---|---|---|---|
| * Takes into account the start Met codon and the His-tag | | | | |

### Cloning and production

Histag in C-term/ cloning in pET24b vector in Nde1-Xho1 site / Stains B834(DE3): genotype: E.coli BL21::DE3 strain, F- ompT hsdSB(rB- mB-) gal dcm (DE3).

B834 is the parental strain for BL2. These protease-deficient hosts are methionine auxotrophs. λ DE3 lysogens are designed for protein expression from pET vectors This strain is also deficient in the Ion and ompT proteases. Selection antibiotic: Kanamycin: 50µg/ml/ production o/n at 16C.

### Purification and Characterisation

### French Press

with lysis buffer: 50mM Bicine-150mM NaCl- 0.4% Empigen- 5mMTCEP - Complete- pH8.0 Ni-NTA GE His trap 1ml (profinia)
Equilibrated : 50mM Bicine - 150mM NaCl -0.15% Empigen - 1mMTCEP - pH8.0
Wash: 50mM Bicine - 150mM NaCl- 0.2% Tween 20 - 1mMTCEP - imidazole 10mM pH8.0 Elution: 50mM Bicine - 150mM NaCl - 0.2% Tween 20 - 1mMTCEP - imidazole 500mM pH8.0 Desalting G-25: 50mM Bicine - 150mM NaCl - 0.2% Tween 20 - 1mMTCEP pH8.0

### Chymotrypsin activation

SEC : Superdex 200 XK16/60 (120ml)
Equilibrated: 50mM Bicine - 150mM NaCl - 1mMTCEP, -pH8.0
Concentration: GE-His Trap (1ml) + Desalting
50mM Bicine - 150mM NaCl - 1mMTCEP, -pH8.0
0.22µm - Dosage LowryRCDC

### Characterization:

Dynamic light scattering is used to evaluate hydrodynamic radius in solution of CdtB purified proteins, in addition to provide information about homogeneity and to detect presence of high molecular weight aggregates within a protein sample. This is based on the calculation of the diffusion coefficient of the different species that are obtained by measuring the light scattering fluctuation, which depends on protein molecular size and shape, and on the other minor constituents of the sample.

Protein samples were analysed on a Dynapro plate reader (Wyatt technology), using five acquisition of 15 seconds at 25°C
a- Buffer: 50mM Bicine - 150mM NaCl- 1mMTCEP, -pH8.0

### Example 7: Cytotoxicity of CDTb proteins on HT29 cells.

The chymotrypsin-activated CDTb sub-units (C37) alone appear cytotoxic on HT29 cells. This suggests that the pore-forming could be responsible of the cytotoxicity.

A second generation of antigen design has been produced in order to inhibit the pore forming step by disrupting the hydrophobic interactions between beta-sheets of individual proteins.

This binary toxin subunit B (binding cells part) of binary toxins was added alone or mixed with binary toxin subunit A (enzymatic part) on Human colonic epithelial cells (HT29 cells) to evaluate their residual cytotoxicity.

The cytotoxicity of candidates C123, C126, C128, C164, C166, C116, C117, C149 and C152 was tested on HT29 cell lines.

### Binary toxin cytotoxicity assay

Human colonic epithelial cells (HT29 cells) were cultured at 37°C with 5% CO2 in DMEM + 10% fetal bovine serum + 1% glutamine + 1% antibiotics (penicillin-streptomycin-amphotericin) and were seeded in 96-well black tissue culture plates (Greiner Bio-one, Ref:655090) at a density of 4 10³ cells/well for HT29.
After 24h, 50µl of the cell media was removed from the wells.

The binary toxin candidates to characterize were diluted in order to reach 2µg/ml of CDTa and 6 µg/ml of CDTb. Further three-fold dilutions were made in microplate (NUNC, Ref: 163320). 50 µl of serial dilutions of binary toxin preparations were added to the black plates and the microplates were incubated at 37°C with 5% CO2 for 6 days.

After 6 days, the mix of binary toxin and medium were removed from the well and 100µl of Hoechst stain (BD Pharmingen, Ref. 561908) diluted 1:500 in phosphate buffer saline (PBS) was added in each well for 2 hours in the dark at room temperature.

After coloration, the Hoechst stain was removed from the wells and the cells fluorescence cells was measured using an Axiovision microscope.

The cytotoxicity is expressed in percentage of surface covered by fluorescent staining in each well. No cytotoxic (100% of recovery) effect is obtained with cells alone. And Higher level of cytotoxicity is obtained with binary toxin full activated (CDTa C34+ CDTb C37).

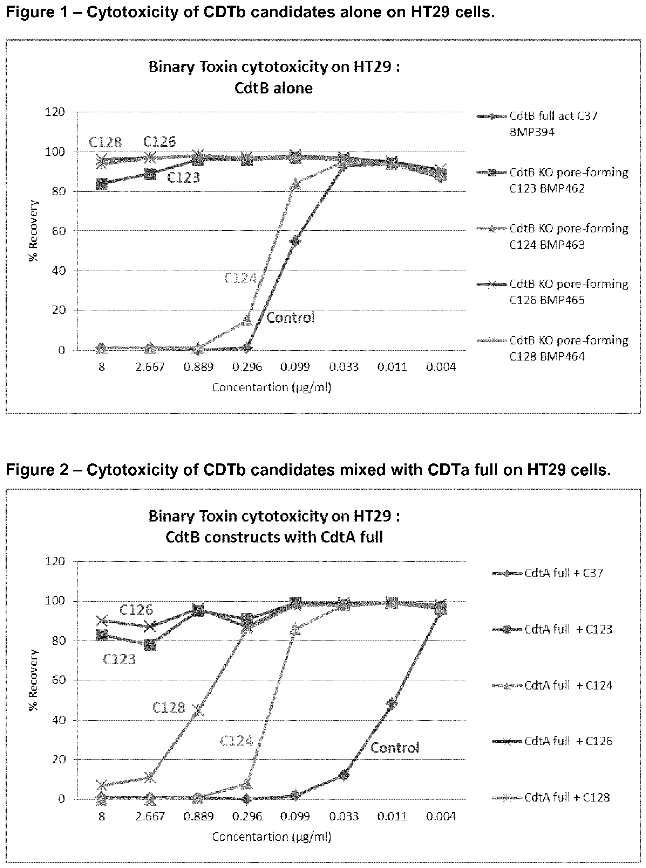

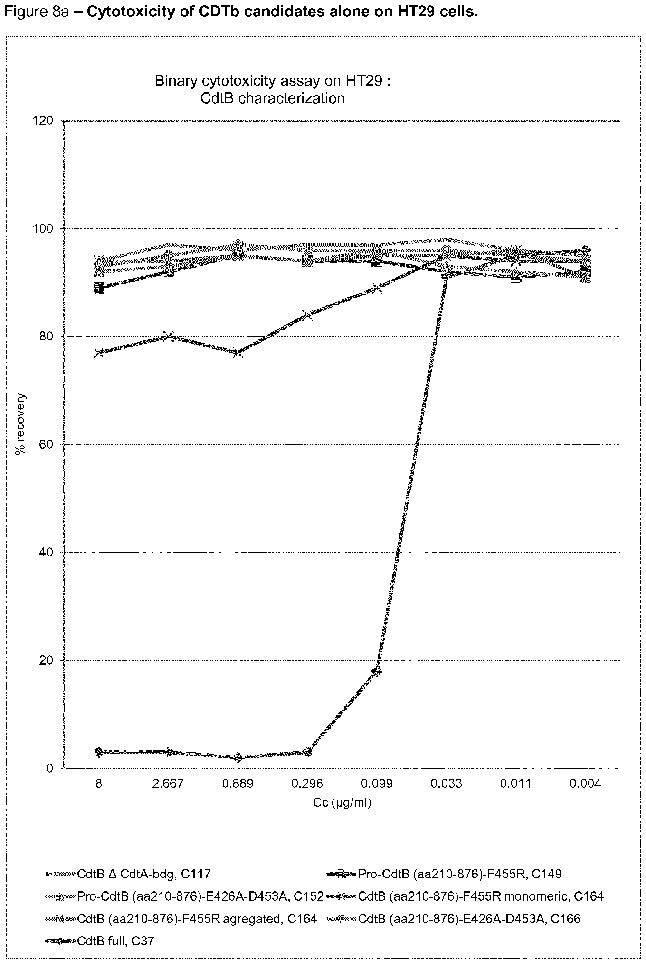

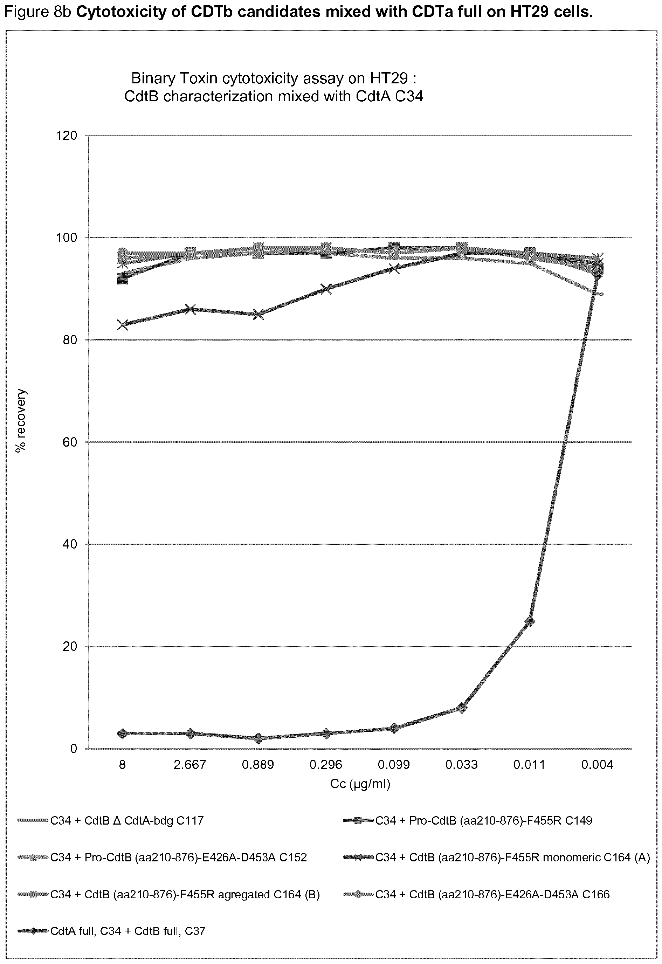

Results are shown in Figure 1, Figure 2 and Figure 8.

### Example 8: Analysis of CDTb proteins by DLS

Dynamic light scattering (DLS) was used to evaluate hydrodynamic radius in solution of CDTb purified proteins C123, C126 and C128, in addition to provide information about homogeneity and to detect presence of high molecular weight aggregates within a protein sample. This is based on the calculation of the diffusion coefficient of the different species that are obtained by measuring the light scattering fluctuation, which depends on protein molecular size and shape, and on the other minor constituents of the sample.

The following protein samples were analysed on a Dynapro plate reader (Wyatt technology), using five acquisition of 15 seconds at 25°C.
All proteins are in a buffer composed of 50mM Bicine - 150mM NaCl- 1mMTCEP, -pH8.0. Measurements were performed on the purified bulk kept at 4°C that had not been frozen.

As shown on figure 3, both C126 KO pore forming construct and C128 KO heptamerisation construct have a hydrodynamic radius of the main population around 3nm that might be coherent with a monomer. The C123 KO heptamerisation construct is found as a homogeneous high molecular weight oligomer or aggregate, with a hydrodynamic radius of 15,7nm.
The fact that average hydrodynamic radius of the total samples are close to the radius of the main populations, together with their low polydispersity, suggest that both C123, C126 and C128 his tagged purified proteins have an homogeneous size distribution.

### Example 9: Design, cloning, expression and purification of C. difficile CDTb fusion protein comprising a full length CDTa protein and a CDTb protein

There are two big advantages for a fusion of CDTa and CDTb : the first one is only one process to obtain 2 proteins. The second one : CDTa is easier than CDTb to produce -> Putting the CDTa as first partner of the fusion could potentially have a positive effect on the entire processability of the fusion. In C139, both mature CDTa (without its signal peptide) and CDTb (without its signal peptide and without its prodomain) proteins were fused.
In C139 the CDTa partner is mutated at position 428 (a Glutamate is mutated into a Glutamine) in order to mutate the cytotoxic activity of CDTa, and the CDTa partner of this fusion is mutated in position 45 (a Cys was mutated into a Tyr residue) in order to avoid an observed dimerisation of CDTa.
In C145, the CDTa part of the fusion is the same as for C139. the CDTa-binding domain of CDTb partner was removed in order to potentially avoid interaction between this domain and the CDTa partner. Between both partners a linker/spacer (6 Gly residues) was added in order to improve the structural flexibility between partners and allow a independent correct folding of the two proteins.
The receptor-binding domain of CDTb has a very good expression yield, is homogenous but less immunogenic than CDTb mature. We tried to increase the immunogenicity of this domain by fusing it to the mature CDTa partner. Two different limits were designed for this receptor-binding domain so 2 fusions were evaluated. In C155 and C156 the CDTa partner is the same as for C139 and C145. In C156, between both partners of the fusion, a spacer/linker was added (6 Gly residues). This linker was not needed in the C155 construct because the receptor-binding domain of CDTb used in C155 begins by a long un-structured and flexible peptide that could be used as linker/spacer.
The following fusion proteins were designed:

| Name | Length(aa)* | Location | Description | SEQ ID |
|---|---|---|---|---|
| C139 | 1094 | CDTa: 44-463 of SEQ ID NO: 1 CDTb: 212-876 of SEQ ID NO: 3 | pET24b-CdtA-E428Q-Cdtb mature-his | SEQ ID NO: 14 |
| C145 | 1016 | CDTa: 44-463 of SEQ ID NO: 1CDTb: 296-876 of SEQ ID NO: 3 | pET24b-CdtA-E428Q-Cdtb ΔCDTa bdg-His | SEQ ID NO: 15 |
| C155 | 686 | CDTa: 44-463 of SEQ ID NO: 1 CDTb: 620-876 of SEQ ID NO: 3 | pET24b-CdtA-E428Q-Cdtb RBD long | SEQ ID NO: 16 |
| C156 | 676 | CDTa: 44-463 of SEQ ID NO: 1 CDTb: 636-876 of SEQ ID NO: 3 | pET24b-CdtA-E428Q-Cdtb RBD short | SEQ ID NO: 17 |

| | | | | |
|---|---|---|---|---|
| * Takes into account the start Met codon and the His-tag | | | | |

### Cloning and production

As for Example 5.

### Example 10: Molecular weight evaluation of CDTb and CDTa-CDTb fusion constructions

Analytical ultracentrifugation may be used to determine the homogeneity and size distribution in solution of the different species within a protein sample by measuring the rate at which molecules move in response to a centrifugal force. This is based on the calculation of the coefficients of sedimentation of the different species that are obtained by sedimentation velocity experiment, which depend on their molecular shape and mass.
1. Protein samples are spun in a Beckman-Coulter ProteomeLab XL-1 analytical ultracentrifuge at 8000RPM, 25000RPM or 42000RPM depending of the target protein size, after the AN-60Ti rotor had been equilibrated to 15°C.
2. For data collection, scans are recorded at 280nm every 5 minutes.
3. Data analysis is performed using the program SEDFIT for determination of the C(S) distribution. Determination of the partial specific volume of the proteins is performed with the SEDNTERP software from their amino acid sequence. Sednterp may also be used to determine the viscosity and the density of the buffer.
4. The molecular weight of the different species may be determined from the C(S) distribution plot (concentration vs sedimentation coefficient), considering that it's a better representation of the raw data than the C(M) distribution (concentration vs molecular weight) to characterize the size distribution of a mixture.

### Example 11: Immunisation of mice with C. difficile CDTa and CDTb sub-units proteins in a AS01B formulation.

### Mice immunisation

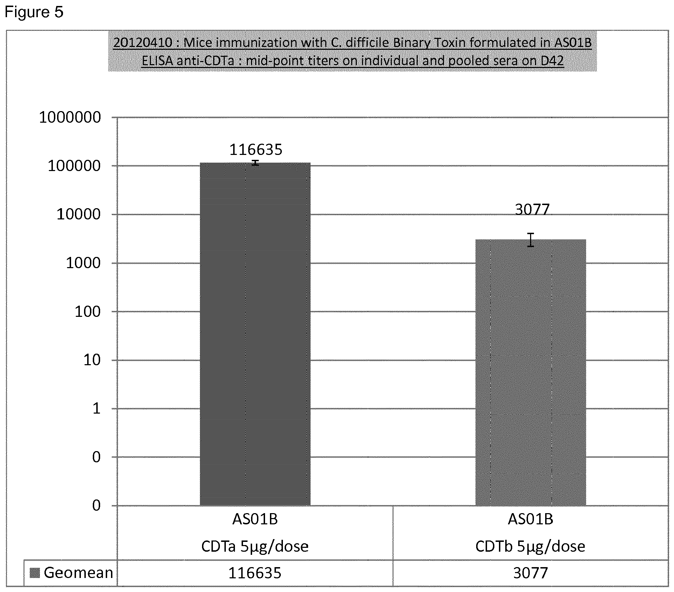

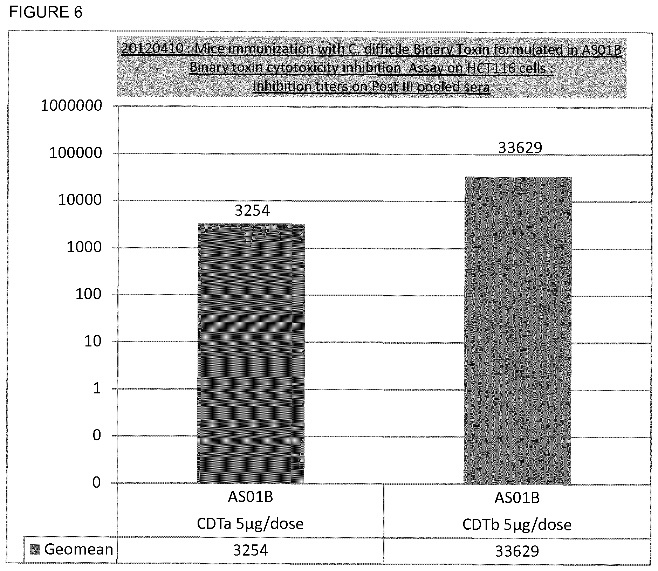

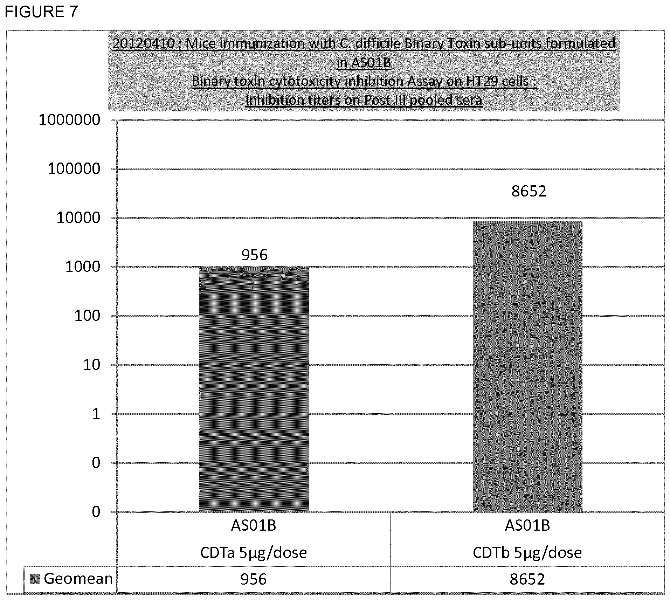

Groups of 25 female Balb/C mice were immunized IM at days 0, 14 and 28 with 5 µg of full CDTa and CDTb binary toxin purified sub-units. These antigens were injected in an AS01B formulation.
Anti-CDTa and anti-CDTb ELISA titres were determined in individual sera collected at day 42 (Post III 14). Results are shown in Figures 4-5.
A binary toxin cytotoxicity inhibition assay was also performed on pooled Post III sera (day42). Results are shown in Figures 6-7

### Anti-CDTa and anti-CDTb ELISA response: Protocol

Full CDTa (C34) or full CDTb (C37)sub-units were coated at 1µg/ml (for CDTa) or 2µg/ml (for CDTb) in phosphate buffered saline (PBS) on high-binding microtitre plates (Nunc MAXISORP™), overnight at 4° C. The plates were blocked with PBS-BSA 1% for 30 min at RT with agitation. The mice anti-sera are prediluted 1/500 in PBS-BSA0.2%-TWEEN™ 0.05%. and then, further twofold dilutions were made in microplates and incubated at RT for 30 min. After washing, bound mouse antibody was detected using Jackson ImmunoLaboratories Inc. peroxidase-conjugated Anti-Mouse (ref: 115-035-003) diluted 1:5000 in PBS-BSA0.2%-tween 0.05%. The detection antibodies were incubated for 30 min. at room temperature (RT) with agitation. The colour was developed using 4 mg O-phenylenediamine (OPD) + 5 µl H₂O₂ per 10 ml pH 4.5 0.1M citrate buffer for 15 minutes in the dark at room temperature. The reaction was stopped with 50 µl HCl, and the optical density (OD) was read at 490 nm relative to 620 nm.

The level of anti-CDTa or anti-CDTb antibodies are expressed in mid-point titres. A GMT was calculated for the 25 samples in each treatment group.

### Binary toxin cytotoxicity inhibition assay

Human colonic epithelial cells (HT29 or HCT-116 cells) were cultured at 37°C with 5%CO₂ in DMEM +10% fetal bovine serum +1% glutamine +1% antibiotics (penicillin-streptomycin-amphotericin) and were seeded in 96-well black tissue culture plates (Greiner Bio-one, Ref : 655090) at a density of 4.10⁴ cells/well for HT29 and 1.10⁴ cells/well for HCT116.
After 24h, the cell media was removed from the wells.

The mice anti-sera were prediluted 1:50 in cell media and then, further three-fold dilutions were made in microplate (NUNC, Ref: 163320). 50µl of serial dilutions of mice pooled antisera were added to the black plates. 50µl of a mix of CDTa (25ng/ml) and chymotrypsin-activated CDTb (75 ng/ml) were then added and the black plates incubated at 37°C with 5% CO₂ for 6 days.

After 6 days, the mix of antisera and toxin were removed from the wells and 100µl of Hoescht stain (BD Pharmingen, Ref : 561908) diluted 1:500 in phosphate buffer saline (PBS) was added in each well for 2 hours in the dark at room temperature.

After coloration, the Hoescht stain was removed from the wells and the cells fluorescence cells was measured using an Axiovision microscope.

The surface covered by fluorescent staining was determined in each well and cytotoxicity inhibition titres were defined as the reciprocal dilution inducing a 50% inhibition of the fluorescent signal.

### Example 12: Cytotoxicity of CDTb proteins on HCT116 cells

The chymotrypsin-activated CDTb sub-units (C37) alone appear cytotoxic on HCT116 cells. This suggests that the pore-forming could be responsible of the cytotoxicity.

A second generation of antigen design has been produced in order to inhibit the pore forming step by disrupting the hydrophobic interactions between beta-sheets of individual proteins.

This binary toxin subunit B (binding cells part) of binary toxins was added alone or mixed with binary toxin subunit A (enzymatic part) on Human colonic epithelial cells (HCT116) to evaluate their residual cytotoxicity.

The cytotoxicity of candidates C123, C126, C128, C164, C166, C116, C117, C149 and C152 was tested on HCT116 cell lines.

### Binary toxin cytotoxicity assay

Human colonic epithelial cells (HCT116 cells) were cultured at 37°C with 5% CO2 in DMEM + 10% fetal bovine serum + 1% glutamine + 1% antibiotics (penicillin-streptomycin-amphotericin) and were seeded in 96-well black tissue culture plates (Greiner Bio-one, Ref:655090) at a density of 10³ cells/well for HCT116.
After 24h, 50µl of the cell media was removed from the wells.

The binary toxin candidates to characterize were diluted in order to reach 2µg/ml of CDTa and 6 µg/ml of CDTb. Further three-fold dilutions were made in microplate (NUNC, Ref: 163320). 50 µl of serial dilutions of binary toxin preparations were added to the black plates and the microplates were incubated at 37°C with 5% CO2 for 6 days.

After 6 days, the mix of binary toxin and medium were removed from the well and 100µl of Hoechst stain (BD Pharmingen, Ref. 561908) diluted 1:500 in phosphate buffer saline (PBS) was added in each well for 2 hours in the dark at room temperature.

After coloration, the Hoechst stain was removed from the wells and the cells fluorescence cells was measured using an Axiovision microscope.

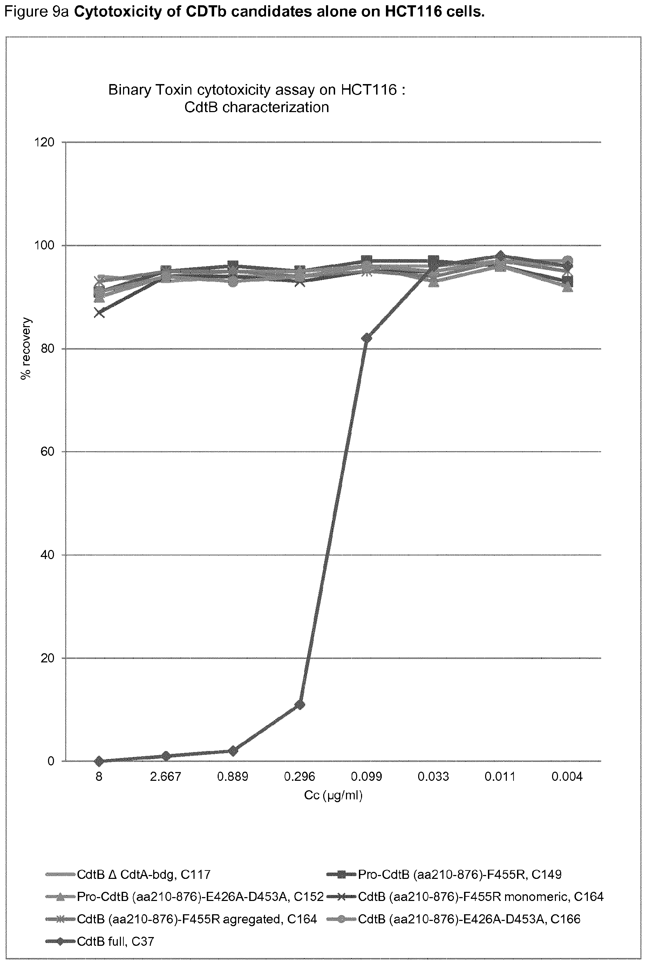

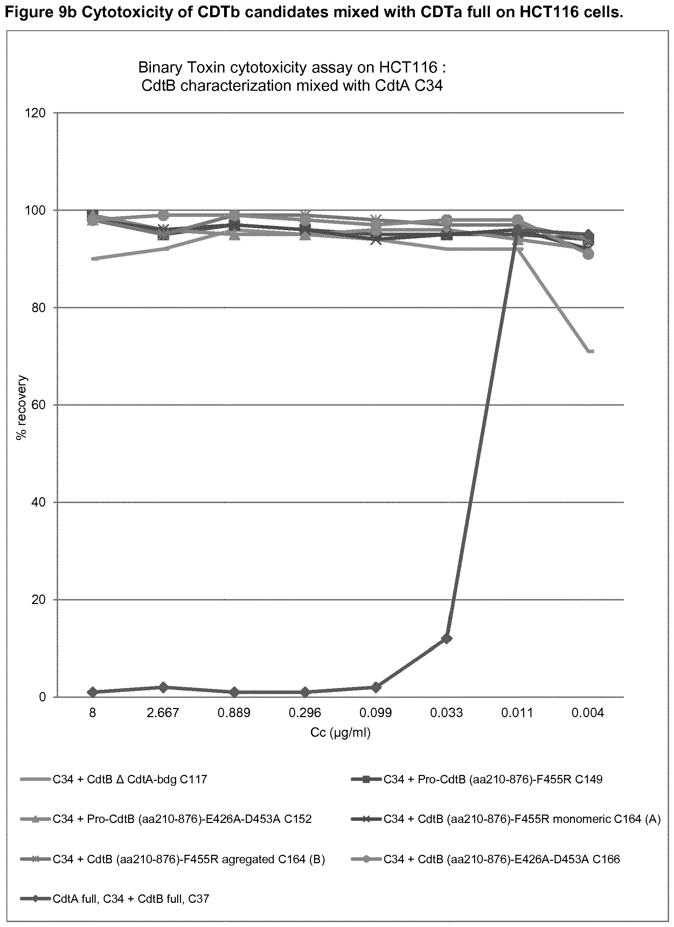

The cytotoxicity is expressed in percentage of surface covered by fluorescent staining in each well. No cytotoxic (100% of recovery) effect is obtained with cells alone. And Higher level of cytotoxicity is obtained with binary toxin full activated (CDTa C34+ CDTb C37).
Results are shown in Figure 9

### Example 13: Cytotoxicity of CDTa-CDTb fusion proteins on HT29 cells

The cytotoxicity of fusions C139, C145, C155 and C156 was tested on HT29 cell lines. The potential residual cytotoxicity associated with the CdtB sub-unit was evaluated by spiking with full CdtA sub-unit. The potential residual cytotoxicity associated with the CdtA sub-unit was evaluated by spiking with full CdtB sub-unit. Full length CDTa and full length CDTb were used as controls.

### Binary toxin cytotoxicity assay

Human colonic epithelial cells (HT29 cells) were cultured at 37°C with 5% CO2 in DMEM + 10% fetal bovine serum + 1% glutamine + 1% antibiotics (penicillin-streptomycin-amphotericin) and were seeded in 96-well black tissue culture plates (Greiner Bio-one, Ref:655090) at a density of 4 10³ cells/well for HT29.
After 24h, 50µl of the cell media was removed from the wells.

The binary toxin candidates to characterize were diluted in order to reach 2µg/ml of CDTa and 6 µg/ml of CDTb. Further three-fold dilutions were made in microplate (NUNC, Ref: 163320). 50 µl of serial dilutions of binary toxin preparations were added to the black plates and the microplates were incubated at 37°C with 5% CO2 for 6 days.

After 6 days, the mix of binary toxin and medium were removed from the well and 100µl of Hoechst stain (BD Pharmingen, Ref. 561908) diluted 1:500 in phosphate buffer saline (PBS) was added in each well for 2 hours in the dark at room temperature.

After coloration, the Hoechst stain was removed from the wells and the cells fluorescence cells was measured using an Axiovision microscope.

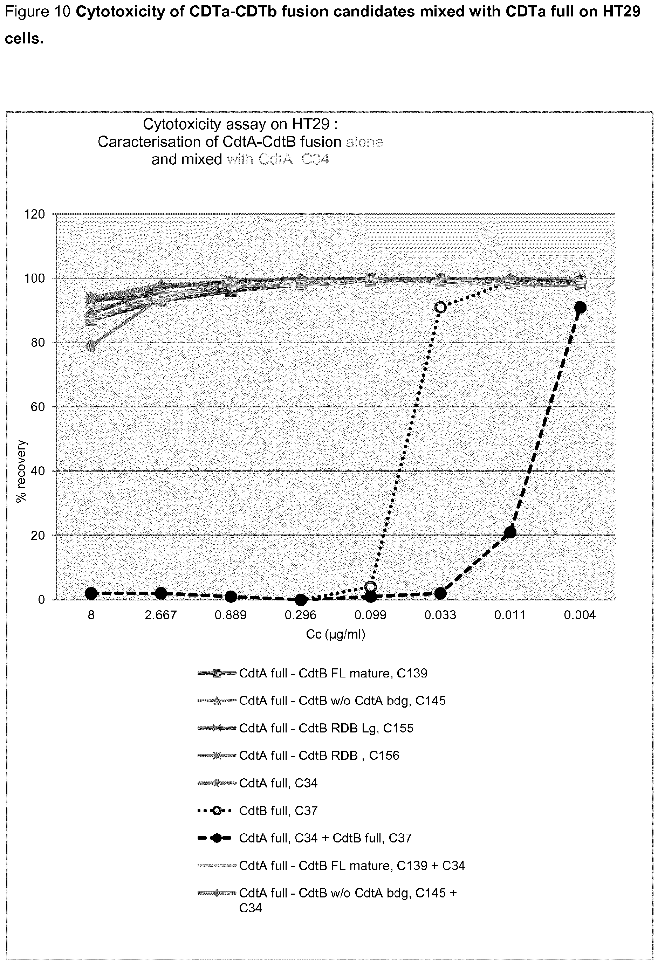

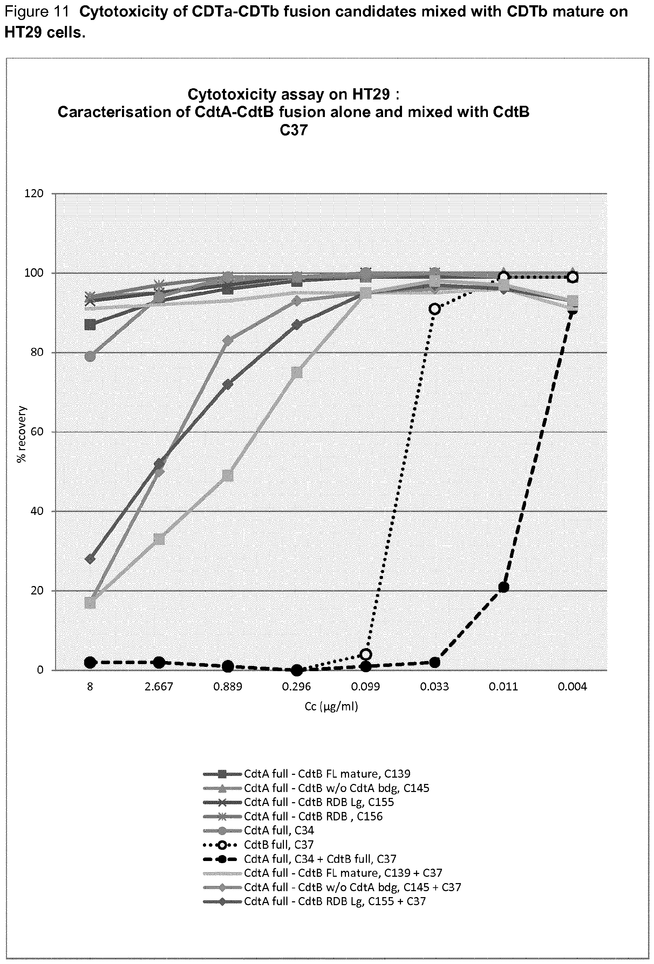

The cytotoxicity is expressed in percentage of surface covered by fluorescent staining in each well. No cytotoxic (100% of recovery) effect is obtained with cells alone. And Higher level of cytotoxicity is obtained with binary toxin full activated (CDTa C34+ CDTb C37).
None of the fusions were cytotoxic and there was no observed residual cytotoxicity associated with the CDTb sub-unit of the fusions. There was no observed residual cytotoxicity associated with the CDTa sub-unit in the CdtA full-CdtB full fusion (C139). A very low residual cytotoxicity associated with the CDTa sub-unit was observed with the 3 fusions not including full lenth CdtB (C145, C155, C156).
Results are shown in Figures 10 and 11.

### Example 14: Immunisation of mice with C. difficile CDTb proteins or CDTa-CDTb fusions in AS01B formulation.

### Mice immunisation

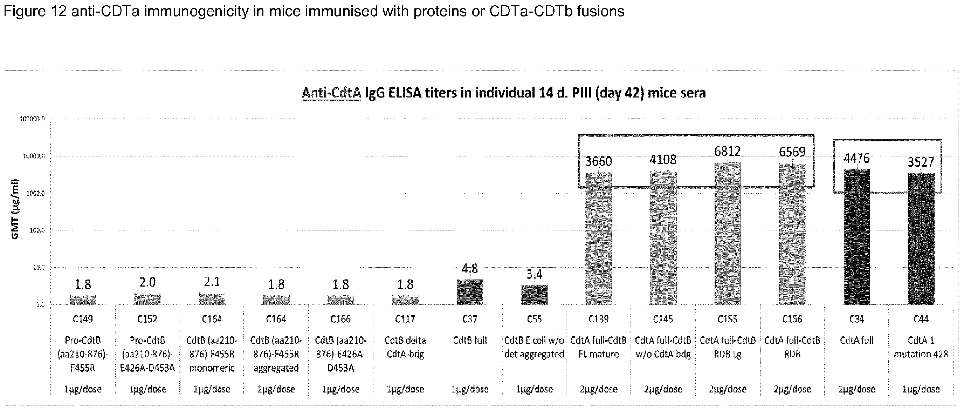

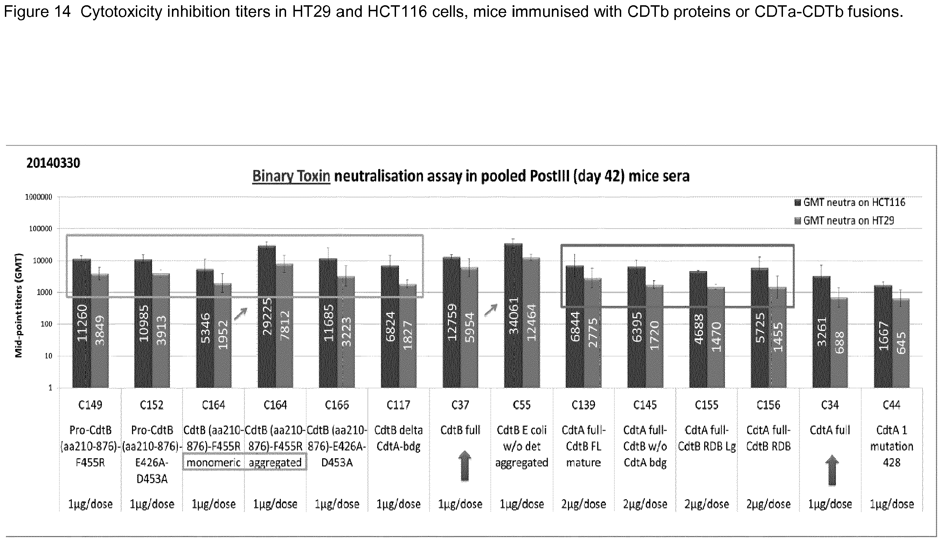

Groups of 12 female Balb/C mice were immunized IM at days 0, 14 and 28 with 1 µg of CDTa and CDTb binary toxin purified sub-units and with 2 µg of CDTa-CDTb fusions. These antigens were injected in an AS01B formulation.
Anti-CDTa and anti-CDTb ELISA titres were determined in individual sera collected at day 42 (Post III 14). Results are shown in Figures 12-13.
A binary toxin cytotoxicity inhibition assay was also performed on pooled Post III sera (day42) on HT29 and HCT116 cell lines. Results are shown in Figure 14.

### Anti-CDTa and anti-CDTb ELISA response: Protocol

E-428 mutated CDTa (C44) or non activated CDTb (C46)sub-units were coated at 1µg/ml in phosphate buffered saline (PBS) on high-binding microtitre plates (Nunc MAXISORP™), overnight at 4° C. The plates were blocked with PBS-BSA 1% for 30 min at RT with agitation. The mice anti-sera are prediluted 1/500 in PBS-BSA0.2%-TWEEN™ 0.05%. and then, further twofold dilutions were made in microplates and incubated at RT for 30 min. After washing, bound mouse antibody was detected using Jackson ImmunoLaboratories Inc. peroxidase-conjugated Anti-Mouse (ref: 115-035-003) diluted 1:5000 in PBS-BSA0.2%-tween 0.05%. The detection antibodies were incubated for 30 min. at room temperature (RT) with agitation. The colour was developed using 4 mg O-phenylenediamine (OPD) + 5 µl H₂O₂ per 10 ml pH 4.5 0.1M citrate buffer for 15 minutes in the dark at room temperature. The reaction was stopped with 50 µl HCl, and the optical density (OD) was read at 490 nm relative to 620 nm.

The level of anti-CDTa or anti-CDTb antibodies is expressed in mid-point titers. A GMT was calculated for the 12 samples in each treatment group.

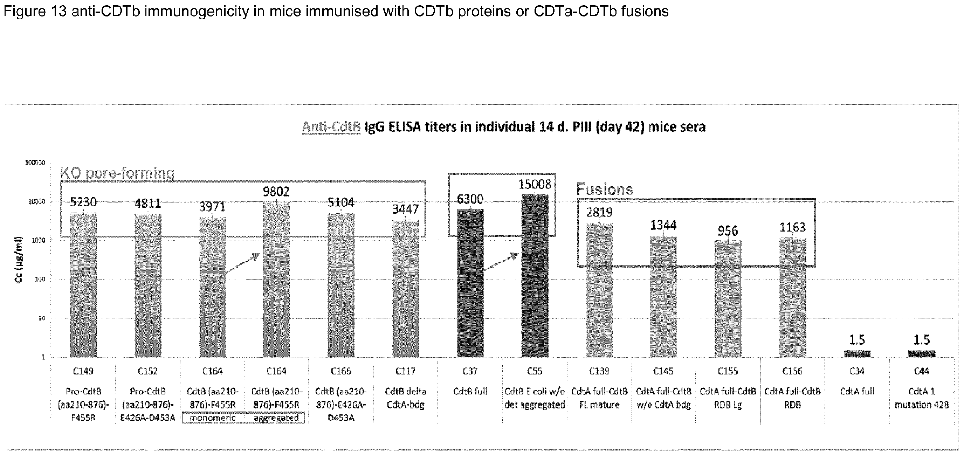

The anti-CdtA antibody titers achieved are shown in Figure 12.
The anti-CdtB antibody titers achieved are shown in Figure 13

### Example 15: CDTb and CDTa-CDTb fusion cytotoxicity inhibition assay

### Binary toxin cytotoxicity inhibition assay

Human colonic epithelial cells (HT29 or HCT-116 cells) were cultured at 37°C with 5%CO₂ in DMEM +10% fetal bovine serum +1% glutamine +1% antibiotics (penicillin-streptomycin-amphotericin) and were seeded in 96-well black tissue culture plates (Greiner Bio-one, Ref : 655090) at a density of 4.10³ cells/well for HT29 and 1.10³ cells/well for HCT116.
After 24h, the cell media was removed from the wells.

The mice anti-sera were prediluted 1:50 in cell media and then, further three-fold dilutions were made in microplate (NUNC, Ref: 163320). 50µl of serial dilutions of mice pooled antisera were added to the black plates. 50µl of a mix of CDTa (25ng/ml) and chymotrypsin-activated CDTb (75 ng/ml) were then added and the black plates incubated at 37°C with 5% CO₂ for 6 days.

After 6 days, the mix of antisera and toxin were removed from the wells and 100µl of Hoescht stain (BD Pharmingen, Ref : 561908) diluted 1:500 in phosphate buffer saline (PBS) was added in each well for 2 hours in the dark at room temperature.

After coloration, the Hoescht stain was removed from the wells and the cells fluorescence cells was measured using an Axiovision microscope.

The surface covered by fluorescent staining was determined in each well and cytotoxicity inhibition titres were defined as the reciprocal dilution inducing a 50% inhibition of the fluorescent signal.
Results are shown in Figure 14

**Sequence Summary (Table A)**

| **Description** | **Construct reference** | **Amino acid sequence** | **Polynucleotide sequence** |
|---|---|---|---|
| CDTa full length (strain R20291) | N/A | SEQ.I.D.NO:1 | SEQ.I.D.NO:2 |
| CDTb full length (strain R20291) | N/A | SEQ.I.D.NO:3 | SEQ.I.D.NO:4 |
| CDTb' (minus signal peptide) ligated to Glutathione-S-transferase protein.(GST underlined) | C37 | SEQ.I.D.NO:5 | SEQ.I.D.NO:6 |
| CDTb with prodomain removed (CDTb", aa212-876) | C55 | SEQ ID NO:7 | |
| CDTb with prodomain and receptor binding domain removed (corresponding to aa 212-616 of SEQ ID NO: 3), includes histag | C116 | SEQ ID NO: 8 | |
| CDTb with prodomain and CDTa binding domain removed (corresponding to aa296-876 of SEQ ID NO: 3), includes histag | C117 | SEQ ID NO: 9 | |
| CDTb (corresponding to SEQ ID NO: 3) mut -F455R, Histag | C123 | SEQ ID NO: 10 | |
| CDTb sequence (corresponding to SEQ ID NO: 3) in which residues 423 (Glutamate) and 453 (Aspartate) are both mutated into alanine. | C126 | SEQ ID NO: 11 | |
| CDTb (corresponding to aa 43-876 of SEQ ID NO: 3) in which aa 532 to aa 542 are deleted and replaced by 3 Gly residues | C127 | SEQ ID NO: 12 | |
| CDTb (corresponding to aa43-876 SEQ ID NO: 3) mut -D537G-E539G-D540G-K541G, Histag | C128 | SEQ ID NO: 13 | |
| Fusion CdtA-E428Q-Cdtb mature, histag (CDTa: aa44-463 of SEQ ID NO: 1; CDTb: aa212-876 of SEQ ID NO: 3) | C139 | SEQ ID NO: 14 | |
| Fusion CdtA-E428Q-Cdtb withCdtA bdg domain removed,Histag (CDTa: aa44-463 of SEQ ID NO: 1; CDTb: aa296-876 of SEQ ID NO: 3) | C145 | SEQ ID NO: 15 | |
| Fusion CdtA-E428Q-Cdtb RBD long (CDTa: aa44-463 of SEQ ID NO: 1; CDTb: aa620-876 of SEQ ID NO: 3) | C155 | SEQ ID NO: 16 | |
| Fusion CdtA-E428Q-Cdtb RBD short (CDTa: aa44-463 of SEQ ID NO: 1; CDTb: aa636-876 of SEQ ID NO: 3) | C156 | SEQ ID NO: 17 | |
| CDTa linker (aa44-268 SEQ ID NO: 1) | C49 | SEQ ID NO: 18 | |
| CDTa without linker (aa44-260 SEQ ID NO:1) | C50 | SEQ ID NO: 19 | |
| CDTa N terminal domain (residue 44 to residue 240) | Gulke et al 2001 | SEQ ID NO: 20 | |
| CDTa (aa44-463 SEQ ID NO:1) mut E428Q-E430Q | C67 | SEQ ID NO: 21 | |
| CDTa (aa44-463 SEQ ID NO: 1) mut R345A-Q350A-N385A-R402AS388 F-E428Q-E430Q | C69 | SEQ ID NO: 22 | |
| CDTa (aa44-463 SEQ ID NO: 1) mut R345A-Q350A-N385A-R402AS388F | C107 | SEQ ID NO: 23 | |
| CDTa (aa44-463 SEQ ID NO: 1) mut R345A-Q350A-N385A-R402AS388F-E430Q | C108 | SEQ ID NO: 24 | |
| CDTb with prodomain, mut F455R | C149 | SEQ ID NO: 25 | |
| CDTb with prodomain, mutE426A-D453A | C152 | SEQ ID NO: 26 | |
| CDTb receptor binding domain with linker in N-term of sequence, from aa 620-876 SEQ ID NO: 3 | C52 | SEQ ID NO: 27 | |
| CDTb receptor binding domain without linker in N-term of sequence, from aa 636-876SEQ ID NO: 3 | C53 | SEQ ID NO: 28 | |
| Toxin A | | SEQ ID NO: 29 | |
| Toxin B | | SEQ ID NO: 30 | |
| Fusion 1 | F1 | SEQ ID NO: 31 | |
| Fusion 2 | F2 | SEQ ID NO: 32 | |
| Fusion 3 | F3 | SEQ ID NO: 33 | |
| Fusion 4 | F4 | SEQ ID NO: 34 | |
| Fusion 5 | F5 | SEQ ID NO: 35 | |
| CDTa (aa44-463 of SEQ ID NO: 1) | C34 | SEQ ID NO: 36 | |
| truncated CDTb protein with the signal peptide, prodomain, receptor binding domain and CDTa binding domain removed, corresponding to aa296 to aa 616 of SEQ ID NO: 3 | C118 | SEQ ID NO: 37 | |
| CDTb' (minus signal peptide) | C46 | SEQ ID NO: 38 | |
| CDTa (aa44-463 SEQ ID NO:1) mut E428Q | C44 | SEQ ID NO: 39 | |
| CDTa (aa44-463 SEQ ID NO:1) mut E430Q | C54 | SEQ ID NO: 40 | |
| CDTa (aa44-463 SEQ ID NO: 1) mut R345A-Q350A-N385A-R402A-E428Q-E430Q | C68 | SEQ ID NO: 41 | |
| CDTa (aa44-463 SEQ ID NO: 1) mut R345A-Q350A-N385A-R402AS388F-E428Q | C110 | SEQ ID NO: 42 | |
| CDTb without prodomain, mut F455R | C164 | SEQ ID NO: 43 | SEQ ID NO: 45 |
| CDTb without prodomain, mutE426A-D453A | C166 | SEQ ID NO: 44 | SEQ ID NO: 46 |
| CDTb' (minus signal peptide) ligated to Glutathione-S-transferase protein.(GST underlined) (without his tag) | C37 without his tag | SEQ ID NO: 47 | |
| CDTb with prodomain removed (CDTb", aa212-876)(without histag) | C55 without histag | SEQ ID NO: 48 | |
| CDTb with prodomain and receptor binding domain removed (corresponding to aa 212-616 of SEQ ID NO: 3), without histag | C116 without histag | SEQ ID NO: 49 | |
| CDTb with prodomain and CDTa binding domain removed (corresponding to aa296-876 of SEQ ID NO: 3), without histag | C117 without histag | SEQ ID NO: 50 | |
| CDTb (corresponding to SEQ ID NO: 3) mut -F455R, without Histag | C123 without histag | SEQ ID NO: 51 | |
| CDTb sequence (corresponding to SEQ ID NO: 3) in which residues 423 (Glutamate) and 453 (Aspartate) are both mutated into alanine. Without histag | C126 without histag | SEQ ID NO: 52 | |
| CDTb (corresponding to aa 43-876 of SEQ ID NO: 3) in which aa 532 to aa 542 are deleted and replaced by 3 Gly residues, without histag | C127 without histag | SEQ ID NO: 53 | |
| CDTb (corresponding to aa43-876 SEQ ID NO: 3) mut -D537G-E539G-D540G-K541 G, without histag | C128 without histag | SEQ ID NO: 54 | |
| Fusion CdtA-E428Q-Cdtb mature, without histag (CDTa: aa44-463 of SEQ ID NO: 1; CDTb: aa212-876 of SEQ ID NO: 3) | C139 without histag | SEQ ID NO:55 | |
| Fusion CdtA-E428Q-Cdtb with CdtA bdg domain removed, without histag (CDTa: aa44-463 of SEQ ID NO: 1; CDTb: aa296-876 of SEQ ID NO: 3) | C145 without histag | SEQ ID NO:56 | |
| Fusion CdtA-E428Q-Cdtb RBD long (CDTa: aa44-463 of SEQ ID NO: 1; CDTb: aa620-876 of SEQ ID NO: 3) without histag | C155 without histag | SEQ ID NO:57 | |
| Fusion CdtA-E428Q-Cdtb RBD short (CDTa: aa44-463 of SEQ ID NO: 1; CDTb: aa636-876 of SEQ ID NO: 3) without histag | C156 without histag | SEQ ID NO:58 | |
| CDTa linker (aa44-268 SEQ ID NO: 1) without histag | C49 without histag | SEQ ID NO:59 | |
| CDTa without linker (aa44-260 SEQ ID NO:1) without histag | C50 without histag | SEQ ID NO:60 | |
| CDTa N terminal domain (residue 44 to residue 240) without histag | Gulke et al 2001 without histag | SEQ ID NO:61 | |
| CDTa (aa44-463 SEQ ID NO:1) mut E428Q-E430Q without histag | C67 without histag | SEQ ID NO:62 | |
| CDTa (aa44-463 SEQ ID NO: 1) mut R345A-Q350A-N385A-R402AS388F-E428Q-E430Q without histag | C69 without histag | SEQ ID NO:63 | |
| CDTa (aa44-463 SEQ ID NO: 1) mut R345A-Q350A-N385A-R402AS388F without histag | C107 without histag | SEQ ID NO:64 | |
| CDTa (aa44-463 SEQ ID NO: 1) mut R345A-Q350A-N385A-R402AS388F-E430Q without histag | C108 without histag | SEQ ID NO:65 | |
| CDTb with prodomain, mut F455R without histag | C149 without histag | SEQ ID NO:66 | |
| CDTb with prodomain, mutE426A-D453A without histag | C152 without histag | SEQ ID NO:67 | |
| CDTb receptor binding domain with linker in N-term of sequence, from aa 620-876 SEQ ID NO: 3 without histag | C52 without histag | SEQ ID NO:68 | |
| CDTb receptor binding domain without linker in N-term of sequence, from aa 636-876SEQ ID NO: 3 without histag | C53 without histag | SEQ ID NO:69 | |
| CDTa (aa44-463 of SEQ ID NO: 1) without histag | C34 without histag | SEQ ID NO:70 | |
| truncated CDTb protein with the signal peptide, prodomain, receptor binding domain and CDTa binding domain removed, corresponding to aa296 to aa 616 of SEQ ID NO: 3, without histag | C118 without histag | SEQ ID NO:71 | |
| CDTb' (minus signal peptide), without histag | C46 without histag | SEQ ID NO:72 | |
| CDTa (aa44-463 SEQ ID NO:1) mut E428Q, without histag | C44 without histag | SEQ ID NO:73 | |
| CDTa (aa44-463 SEQ ID NO:1) mut E430Q, without histag | C54 without histag | SEQ ID NO:74 | |
| CDTa (aa44-463 SEQ ID NO: 1) mut R345A-Q350A-N385A-R402A-E428Q-E430Q, without histag | C68 without histag | SEQ ID NO:75 | |
| CDTa (aa44-463 SEQ ID NO: 1) mut R345A-Q350A-N385A-R402AS388F-E428Q, without histag | C110 without histag | SEQ ID NO:76 | |

### SEQUENCE LISTING

**SEQ ID 1 - CDTa full length polypeptide sequence** 
MKKFRKHKRISNCISILLILYLTLGGLLPNNIYAQDLQSYSEKVCNTTYKAPIERPEDFLKDKE
KAKEWERKEAERIEQKLERSEKEALESYKKDSVEISKYSQTRNYFDYQIEANSREKEYKEL
RNAISKNKIDKPMYVYYFESPEKFAFNKVIRTENQNEISLEKFNEFKETIQNKLFKQDGFKDIS
LYEPGKGDEKPTPLLMHLKLPRNTGMLPYTNTNNVSTLIEQGYSIKIDKIVRIVIDGKHYIKAE
ASVVSSLDFKDDVSKGDSWGKANYNDWSNKLTPNELADVNDYMRGGYTAINNYLISNGPV
NNPNPELDSKITNIENALKREPIPTNLTVYRRSGPQEFGLTLTSPEYDFNKLENIDAFKSKWE
GQALSYPNFISTSIGSVNMSAFAKRKIVLRITIPKGSPGAYLSAIPGYAGEYEVLLNHGSKFKI
NKIDSYKDGTITKLIVDATLIP
**SEQ ID 2 - CDTa full length polynucleotide sequence** 
ATGAAAAAATTTAGGAAACATAAAAGGATTAGTAATTGTATATCTATATTGTTGATATTAT
ATCTAACTTTAGGTGGTTTGTTACCTAATAACATTTATGCACAAGACTTACAAAGCTATA
GTGAAAAAGTTTGCAATACTACTTACAAGGCTCCTATAGAAAGACCAGAAGATTTTCTTA
AAGATAAAGAAAAGGCTAAAGAATGGGAAAGAAAAGAAGCAGAAAGAATAGAGCAAAAA
CTTGAAAGATCTGAAAAAGAAGCATTAGAATCATATAAAAAAGATTCTGTAGAAATAAGT
AAATATTCTCAGACAAGAAATTATTTTTATGATTATCAAATAGAAGCAAATTCTCGAGAAA
AAGAATATAAAGAACTTCGAAATGCTATATCAAAAAATAAAATAGATAAACCTATGTATGT
CTATTATTTTGAATCTCCAGAAAAATTTGCATTTAATAAAGTAATAAGAACAGAAAATCAA
AACGAAATTTCATTAGAAAAATTTAATGAGTTTAAAGAAACTATACAAAACAAATTATTTA
AGCAAGATGGATTTAAAGATATTTCTTTATATGAACCTGGAAAAGGTGATGAAAAACCTA
CACCATTACTTATGCACTTAAAATTACCTAGAAATACTGGTATGTTACCATATACAAATAC
TAACAATGTAAGTACATTAATAGAGCAAGGATATAGTATAAAAATAGATAAAATTGTTCGT
ATAGTTATAGATGGGAAGCACTATATTAAAGCAGAAGCATCTGTTGTAAGTAGTCTTGAT
TTTAAAGATGATGTAAGTAAGGGGGATTCTTGGGGTAAAGCAAATTATAATGATTGGAG
TAATAAATTAACACCTAATGAACTTGCTGATGTAAATGATTATATGCGTGGAGGATATAC
TGCAATTAATAATTATTTAATATCAAATGGTCCAGTAAATAATCCTAACCCAGAATTAGAT
TCTAAAATCACAAACATTGAAAATGCATTAAAACGTGAACCTATTCCAACTAATTTAACTG
TATATAGAAGATCTGGTCCTCAAGAATTTGGTTTAACTCTTACTTCCCCTGAATATGATTT
TAACAAACTAGAAAATATAGATGCTTTTAAATCAAAATGGGAAGGACAAGCACTGTCTTA
TCCAAACTTTATTAGTACTAGTATTGGTAGTGTGAATATGAGTGCATTTGCTAAAAGAAA
AATAGTACTACGTATAACTATACCTAAAGGTTCTCCTGGAGCTTATCTATCAGCTATTCC
AGGTTATGCAGGTGAATATGAAGTGCTTTTAAATCATGGAAGCAAATTTAAAATCAATAA
 
AATTGATTCTTACAAAGATGGTACTATAACAAAATTAATTGTTGATGCAACATTGATACCT
TAA
**SEQ ID 3 - CDTb full length polypeptide sequence** 
MKIQMRNKKVLSFLTLTAIVSQALVYPVYAQTSTSNHSNKKKEIVNEDILPNNGLMGYYFTDE
HFKDLKLMAPIKDGNLKFEEKKVDKLLDKDKSDVKSIRWTGRIIPSKDGEYTLSTDRDDVLM
QVNTESTISNTLKVNMKKGKEYKVRIELQDKNLGSIDNLSSPNLYWELDGMKKIIPEENLFLR
DYSNIEKDDPFIPNNNFFDPKLMSDWEDEDLDTDNDNIPDSYERNGYTIKDLIAVKWEDSFA
EQGYKKYVSNYLESNTAGDPYTDYEKASGSFDKAIKTEARDPLVAAYPIVGVGMEKLIISTN
EHASTDQGKTVSRATTNSKTESNTAGVSVNVGYQNGFTANVTTNYSHTTDNSTAVQDSNG
ESWNTGLSINKGESAYINANVRYYNTGTAPMYKVTPTTNLVLDGDTLSTIKAQENQIGNNLS
PGDTYPKKGLSPLALNTMDQFSSRLIPINYDQLKKLDAGKQIKLETTQVSGNFGTKNSSGQI
VTEGNSWSDYISQIDSISASIILDTENESYERRVTAKNLQDPEDKTPELTIGEAIEKAFGATKK
DGLLYFNDIPIDESCVELIFDDNTANKIKDSLKTLSDKKIYNVKLERGMNILIKTPTYFTNFDDY
NNYPSTWSNVNTTNQDGLQGSANKLNGETKIKIPMSELKPYKRYVFSGYSKDPLTSNSIIVKI
KAKEEKTDYLVPEQGYTKFSYEFETTEKDSSNIEITLIGSGTTYLDNLSITELNSTPEILDEPEV
KIPTDQEIMDAHKIYFADLNFNPSTGNTYINGMYFAPTQTNKEALDYIQKYRVEATLQYSGFK
DIGTKDKEMRNYLGDPNQPKTNYVNLRSYFTGGENIMTYKKLRIYAITPDDRELLVLSVD
**SEQ ID 4 - CDTb full length polynucleotide sequence** 
ATGAAAATACAAATGAGGAATAAAAAGGTATTAAGTTTTTAACACTTACAGCTATAGTTA
GTCAAGCACTAGTATATCCTGTATATGCTCAAACTAGTACAAGTAATCATTCTAATAAGA
AAAAAGAAATTGTAAATGAAGATATACTCCCAAACAATGGATTAATGGATATTATTTCA
CAGATGAGCACTTTAAAGATTTAAAATTAATGGCACCCATAAAAGATGGTAATTTAAAAT
TTGAAGAAAAGAAAGTAGATAAACTTCTGGATAAAGACAAATCAGATGTAAAATCTATAC
GATGGACAGGAAGAATAATTCCTTCTAAGGATGGTGAATATACATTATCAACTGATAGA
GATGATGTCTTAATGCAAGTAAATACTGAGAGTACTATATCAAATACACTTAAAGTTAATA
TGAAAAAGGGTAAAGAATATAAAGTTAGAATAGAGCTACAAGATAAAAATTTAGGTTCAA
TAGATAATTTATCATCACCTAATCTTTATTGGGAATTAGATGGTATGAAGAAAATTATACC
AGAAGAAAATTTATTCTTAAGAGATTATTCTAATATAGAAAAAGATGATCCATTTATCCCA
AATAACAATTTCTTTGACCCAAAGTTGATGTCTGATTGGGAAGACGAAGATTTGGATACA
GATAATGATAATATACCAGATTCATATGAACGAAATGGATATACTATTAAGGACTTAATT
GCAGTTAAGTGGGAAGATAGTTTTGCAGAACAAGGCTATAAGAAATATGTATCAAATTAT
TTAGAGTCAAATACTGCTGGAGATCCATATACAGATTATGAAAAAGCTTCAGGTTCTTTT
GACAAGGCTATAAAGACTGAAGCAAGAGATCCGTTAGTTGCAGCATATCCAATTGTTGG
AGTAGGTATGGAAAAATTAATTATATCTACAAATGAACATGCCTCTACTGATCAAGGTAA
AACTGTTTCCAGAGCTACTACTAACAGTAAAACTGAATCTAATACAGCTGGTGTGTCTGT
 
TAATGTAGGATATCAAAATGGATTCACAGCTAATGTAACTACAAATTATTCCCATACAAC
AGATAATTCAACTGCTGTTCAAGATAGTAATGGAGAATCATGGAATACTGGATTAAGTAT
AAACAAAGGAGAATCTGCATATATAAATGCAAATGTTAGATATTACAACACAGGTACTGC
ACCTATGTACAAAGTGACACCAACAACAAATTTAGTGTTAGATGGAGATACATTATCAAC
TATCAAAGCACAAGAAAATCAAATTGGCAATAATCTATCTCCTGGAGATACTTATCCCAA
AAAAGGGCTTTCACCTCTAGCTCTTAACACAATGGATCAATTTAGCTCTAGACTGATTCC
TATAAATTATGATCAATTAAAAAAATTAGATGCTGGAAAGCAAATTAAATTAGAAACAACA
CAAGTAAGTGGAAATTTTGGTACAAAAAATAGTTCTGGACAAATAGTAACAGAAGGAAAT
AGTTGGTCAGACTATATAAGTCAAATTGACAGTATTTCTGCATCTATTATATTAGATACAG
AGAATGAATCTTACGAAAGAAGAGTTACTGCTAAAAATTTACAGGATCCAGAAGATAAAA
CACCTGAACTTACAATTGGAGAAGCAATTGAAAAAGCTTTTGGCGCTACTAAAAAAGAT
GGTTTGTTATATTTTAATGATATACCAATAGATGAAAGTTGTGTTGAACTCATATTTGATG
ATAATACAGCCAATAAGATTAAAGATAGTTTAAAAACTTTGTCTGATAAAAAGATATATAA
TGTTAAACTTGAAAGAGGAATGAATATACTTATAAAAACACCAACTTACTTTACTAATTTT
GATGATTATAATAATTACCCTAGTACATGGAGTAATGTCAATACTACGAATCAAGATGGT
TTACAAGGCTCAGCAAATAAATTAAATGGTGAGACGAAGATTAAAATCCCTATGTCTGAG
CTAAAACCTTATAAACGTTATGTTTTTAGTGGATATTCAAAGGATCCTTTAACATCTAATT
CAATAATTGTAAAGATAAAAGCAAAAGAAGAGAAAACGGATTATTTGGTACCAGAACAA
GGATATACAAAATTTAGTTATGAATTTGAAACTACTGAAAAAGATTCTTCTAATATAGAGA
TAACATTAATTGGTAGTGGTACAACATACTTAGATAACTTATCTATTACAGAGCTAAATAG
TACTCCTGAAATACTTGATGAACCAGAAGTTAAAATTCCAACTGACCAAGAAATAATGGA
TGCACATAAAATATATTTTGCAGATTTAAATTTTAATCCAAGTACAGGAAATACTTATATA
AATGGTATGTATTTTGCACCAACACAAACTAATAAAGAAGCTCTCGATTATATCCAAAAA
TATAGAGTTGAAGCTACTTTACAATATTCTGGATTTAAAGATATTGGAACTAAAGATAAA
GAAATGCGTAATTATTTAGGAGATCCAAATCAGCCTAAAACTAATTATGTTAATCTTAGG
AGTTATTTTACAGGTGGAGAAAATATTATGACATACAAGAAATTAAGAATATATGCAATTA
CTCCAGACGATAGAGAGTTATTAGTTCTTAGTGTTGATTAG
**SEQ ID 5 - CDTb C37 construct. CDTb' (minus signal peptide) ligated to Glutathione-S-transferase protein (GST underlined) polypeptide sequence.** 
MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR
DDVLMQVNTESTISNTLKVNMKKGKEYKVRIELQDKNLGSIDNLSSPNLYWELDGMKKIIPE
ENLFLRDYSNIEKDDPFIPNNNFFDPKLMSDWEDEDLDTDNDNIPDSYERNGYTIKDLIAVK
 
WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA
VQDSNGESWNTGLSINKGESAYINANVRYYNTGTAPMYKVTPTTNLVLDGDTLSTIKAQEN
QIGNNLSPGDTYPKKGLSPLALNTMDQFSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVDHHHHHH
**SEQ ID 6 - CDTb C37construct. CDTb' (minus pro-domain) ligated to Glutathione-S-transferase protein (GST underlined) polynucleotide sequence.** 
atgtcccctatactaggttattggaaaattaagggccttgtgcaacccactcgacttcttttggaatatcttgaagaaaaatatgaag
agcatttgtatgagcgcgatgaaggtgataaatggcgaaacaaaaagtttgaattgggtttggagtttcccaatcttccttattatatt
gatggtgatgttaaattaacacagtctatggccatcatacgttatatagctgacaagcacaacatgttgggtggttgtccaaaagag
cgtgcagagatttcaatgcttgaaggagcggttttggatattagatacggtgtttcgagaattgcatatagtaaagactttgaaactct
caaagttgattttcttagcaagctacctgaaatgctgaaaatgttcgaagatcgtttatgtcataaaacatatttaaatggtgatcatgt
aacccatcctgacttcatgttgtatgacgctcttgatgttgttttatacatggacccaatgtgcctggatgcgttcccaaaattagtttgttt
taaaaaacgtattgaagctatcccacaaattgataagtacttgaaatccagcaagtatatagcatggcctttgcagggctggcaa
gccacgtttggtggtggcgaccatcctccaaaatcggatctggaagttctgttccaggggcccctgggatcccatatggaaattgtg
aatgaagatattctgccgaataatggtctgatgggatactttaccgatgaacattttaaagatctgaaactgatggcaccgatta
aagatggcaatctgaaatttgaagaaaaaaagtggataaactgctggataaagataaaagtgatgtgaaaagcattcgttgg
accggtcgtattattccgagcaaagatggtgaatacaccctgagcaccgatcgtgatgatgttctgatgcaggttaataccgaaag
caccattagcaataccctgaaagtgaatatgaaaaaggcaaagaatataaagtgcgcattgaactgcaggataaaaatctgg
gtagcattgataatctgagcagcccgaatctgtattgggaactggatggtatgaaaaaaatcattccggaagaaaacctgtttctg
cgcgattatagcaatattgaaaaagatgatccgtttattccgaataataactttttgatccgaaactgatgagcgattgggaagatg
aagatctggataccgataatgataatattccggatagctatgaacgcaatggctataccattaaagatctgattgccgtgaaatgg
gaagatagctttgcagaacagggctataagaaatatgtgagcaattatctggaaagcaataccgcaggcgatccgtataccgat
tatgaaaaagcaagcggcagctttgataaagccattaaaaccgaagcacgtgatccgctggttgcagcatatccgattgttggtgt
tggtatggaaaaactgattattagcaccaatgaacatgcaagcaccgatcagggtaaaaccgttagccgtgcaaccaccaata
gcaaaaccgaaagcaatacagccggtgttagcgttaatgttggttatcagaatggttttaccgccaatgtgaccaccaattatagc
cataccaccgataatagcaccgcagttcaggatagcaatggtgaaagctggaataccggtctgagcattaacaaaggtgaaag
cgcatatatcaatgccaatgtgcgctattataacaccggcaccgcaccgatgtataaagttaccccgaccaccaatctggttctgg
atggtgataccctgagtaccattaaagcacaagaaaatcagattggcaataatctgagtccgggtgatacctatccgaaaaaag
 
gtctgagtccgctggcactgaataccatggatcagtttagcagccgtctgattccgattaactatgatcagctgaaaaaactggatg
ccggtaaacaaatcaaactggaaaccacccaggttagcggtaattttggcaccaaaaattcaagcggtcagattgttaccgaag
gtaatagctggtcagattatatcagccagattgatagcattagcgccagcattattctggatacagaaaatgaaagctatgaacgt
cgtgtgaccgcaaaaaatctgcaggacccggaagataaaacaccggaactgaccattggtgaagcaattgaaaaagcatttg
gtgccaccaaaaaagatggcctgctgtattttaacgatattccgattgatgaaagctgcgtggaactgatttttgatgataataccgc
caataaaatcaaagatagcctgaaaaccctgagcgacaaaaaaatctataatgtgaaactggaacgcggtatgaatattctgat
taaaaccccgacctattttaccaatttgatgattataacaattatccgagcacttggagcaatgtgaataccaccaatcaggatggt
ctgcagggtagcgcaaataaactgaatggtgaaaccaaaatcaaaattccgatgagcgaactgaaaccgtataaacgttatgt
gtttagcggctatagcaaagatccgctgaccagcaatagcattattgtgaaaatcaaagccaaagaagaaaaaaccgattatct
ggttccggaacagggttataccaaatttagctatgaatttgaaaccaccgaaaaagatagcagtaatattgaaattaccctgattg
gtagcggcaccacctatctggataatctgagtattaccgaactgaatagcacaccggaaattctggatgaaccggaagtgaaaa
ttccgaccgatcaagaaattatggatgcccataaaatctattttgccgatctgaactttaatccgagcaccggcaatacctatattaa
cggcatgtattttgcaccgacccagaccaataaagaagccctggattatattcagaaatatcgtgttgaagccaccctgcagtata
gcggttttaaagatattggcaccaaagataaagaaatgcgtaattatctgggcgatccgaatcagccgaaaaccaattatgttaat
ctgcgcagctattttaccggtggcgaaaacattatgacctacaaaaaactgcgcatttatgccattacaccggatgatcgtgaact
gctggttctgagcgttgatcaccaccatcatcatcattaa
**SEQ ID NO: 7 - amino acid sequence of CDTb with prodomain removed (CDTb", aa212-876) (C55)** 
MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR
YYNTGTAPMYKVTPTTNLVLDGDTLSTIKAQENQIGNNLSPGDTYPKKGLSPLALNTMDQFS
SRLIPINYDQLKKLDAGKQIKLETTQVSGNFGTKNSSGQIVTEGNSWSDYISQIDSISASIILDT
ENESYERRVTAKNLQDPEDKTPELTIGEAIEKAFGATKKDGLLYFNDIPIDESCVELIFDDNTA
NKIKDSLKTLSDKKIYNVKLERGMNILIKTPTYFTNFDDYNNYPSTWSNVNTTNQDGLQGSA
NKLNGETKIKIPMSELKPYKRYVFSGYSKDPLTSNSIIVKIKAKEEKTDYLVPEQGYTKFSYEF
ETTEKDSSNIEITLIGSGTTYLDNLSITELNSTPEILDEPEVKIPTDQEIMDAHKIYFADLNFNPS
TGNTYINGMYFAPTQTNKEALDYIQKYRVEATLQYSGFKDIGTKDKEMRNYLGDPNQPKTN
YVNLRSYFTGGENIMTYKKLRIYAITPDDRELLVLSVDHHHHHH
**SEQ ID NO: 8 C116** 
MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR
YYNTGTAPMYKVTPTTNLVLDGDTLSTIKAQENQIGNNLSPGDTYPKKGLSPLALNTMDQFS
SRLIPINYDQLKKLDAGKQIKLETTQVSGNFGTKNSSGQIVTEGNSWSDYISQIDSISASIILDT
 
ENESYERRVTAKNLQDPEDKTPELTIGEAIEKAFGATKKDGLLYFNDIPIDESCVELIFDDNTA
NKIKDSLKTLSDKKIYNVKLERGMNILIKTPGGHHHHHH
**SEQ ID NO: 9 C117** 
MAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG
DTLSTIKAQENQIGNNLSPGDTYPKKGLSPLALNTMDQFSSRLIPINYDQLKKLDAGKQIKLE
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPTYFTNFDDYNNYPSTWSNVNTTNQDGLQGSANKLNGETKIKIPMSELKPYKRY
VFSGYSKDPLTSNSIIVKIKAKEEKTDYLVPEQGYTKFSYEFETTEKDSSNIEITLIGSGTTYLD
NLSITELNSTPEILDEPEVKIPTDQEIMDAHKIYFADLNFNPSTGNTYINGMYFAPTQTNKEAL
DYIQKYRVEATLQYSGFKDIGTKDKEMRNYLGDPNQPKTNYVNLRSYFTGGENIMTYKKLRI
YAITPDDRELLVLSVDHHHHHH
**SEQ ID NO: 10 C123 CDTb sequence with F455R.** 
MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR
DDVLMQVNTESTISNTLKVNMKKGKEYKVRIELQDKNLGSIDNLSSPNLYWELDGMKKIIPE
ENLFLRDYSNIEKDDPFIPNNNFFDPKLMSDWEDEDLDTDNDNIPDSYERNGYTIKDLIAVK
WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA
VQDSNGESWNTGLSINKGESAYINANVRYYNTGTAPMYKVTPTTNLVLDGDTLSTIKAQEN
QIGNNLSPGDTYPKKGLSPLALNTMDQRSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVDHHHHHH
**SEQ ID NO: 11 C126 CDTb sequence in which residues 426 (Glutamate) and 453 (Aspartate) were both mutated into alanine.** 
MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR
DDVLMQVNTESTISNTLKVNMKKGKEYKVRIELQDKNLGSIDNLSSPNLYWELDGMKKIIPE
ENLFLRDYSNIEKDDPFIPNNNFFDPKLMSDWEDEDLDTDNDNIPDSYERNGYTIKDLIAVK
WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA
VQDSNGESWNTGLSINKGESAYINANVRYYNTGTAPMYKVTPTTNLVLDGDTLSTIKAQAN
QIGNNLSPGDTYPKKGLSPLALNTMAQFSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVDHHHHHH
**SEQ ID NO: 12 C127** 
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTAV
TTNYSHTTDNSTAVQDSNGESWNTGLSINKGESAYINANVRYYNTGTAPMYKVTPTTNLVL
DGDTLSTIKAQENQIGNNLSPGDTYPKKGLSPLALNTMDQFSSRLIPINYDQLKKLDAGKQIK
LETTQVSGNFGTKNSSGQIVTEGNSWSDYISQIDSISASIILDTENESYERRVTAgggPELTIG
EAIEKAFGATKKDGLLYFNDIPIDESCVELIFDDNTANKIKDSLKTLSDKKIYNVKLERGMNILI
KTPTYFTNFDDYNNYPSTWSNVNTTNQDGLQGSANKLNGETKIKIPMSELKPYKRYVFSGY
SKDPLTSNSIIVKIKAKEEKTDYLVPEQGYTKFSYEFETTEKDSSNIEITLIGSGTTYLDNLSITE
LNSTPEILDEPEVKIPTDQEIMDAHKIYFADLNFNPSTGNTYINGMYFAPTQTNKEALDYIQKY
RVEATLQYSGFKDIGTKDKEMRNYLGDPNQPKTNYVNLRSYFTGGENIMTYKKLRIYAITPD
DRELLVLSVDHHHHHH
**SEQ ID NO: 13 C128** 
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQENQIGNNLSPGDTYPKKGLSPLALNTMDQFSSRLIPINYDQLKKLDAGK
QIKLETTQVSGNFGTKNSSGQIVTEGNSWSDYISQIDSISASIILDTENESYERRVTAKNLQgP
gggTPELTIGEAIEKAFGATKKDGLLYFNDIPIDESCVELIFDDNTANKIKDSLKTLSDKKIYNVK
LERGMNILIKTPTYFTNFDDYNNYPSTWSNVNTTNQDGLQGSANKLNGETKIKIPMSELKPY
KRYVFSGYSKDPLTSNSIIVKIKAKEEKTDYLVPEQGYTKFSYEFETTEKDSSNIEITLIGSGTT
YLDNLSITELNSTPEILDEPEVKIPTDQEIMDAHKIYFADLNFNPSTGNTYINGMYFAPTQTNK
EALDYIQKYRVEATLQYSGFKDIGTKDKEMRNYLGDPNQPKTNYVNLRSYFTGGENIMTYK
KLRIYAITPDDRELLVLSVDHHHHHH
**SEQ ID NO: 14 C139** 
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPSDWEDEDLDTDNDNIPDSYE
RNGYTIKDLIAVKWEDSFAEQGYKKYVSNYLESNTAGDPYTDYEKASGSFDKAIKTEARDPL
VAAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG
DTLSTIKAQENQIGNNLSPGDTYPKKGLSPLALNTMDQFSSRLIPINYDQLKKLDAGKQIKLE
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPTYFTNFDDYNNYPSTWSNVNTTNQDGLQGSANKLNGETKIKIPMSELKPYKRY
VFSGYSKDPLTSNSIIVKIKAKEEKTDYLVPEQGYTKFSYEFETTEKDSSNIEITLIGSGTTYLD
NLSITELNSTPEILDEPEVKIPTDQEIMDAHKIYFADLNFNPSTGNTYINGMYFAPTQTNKEAL
DYIQKYRVEATLQYSGFKDIGTKDKEMRNYLGDPNQPKTNYVNLRSYFTGGENIMTYKKLRI
YAITPDDRELLVLSVDGGHHHHHH
**SEQ ID NO: 15 C145** 
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPGGGGGGAYPIVGVGMEKLIIS
TNEHASTDQGKTVSRATTNSKTESNTAGVSVNVGYQNGFTANVTTNYSHTTDNSTAVQDS
NGESWNTGLSINKGESAYINANVRYYNTGTAPMYKVTPTTNLVLDGDTLSTIKAQENQIGNN
LSPGDTYPKKGLSPLALNTMDQFSSRLIPINYDQLKKLDAGKQIKLETTQVSGNFGTKNSSG
QIVTEGNSWSDYISQIDSISASIILDTENESYERRVTAKNLQDPEDKTPELTIGEAIEKAFGATK
KDGLLYFNDIPIDESCVELIFDDNTANKIKDSLKTLSDKKIYNVKLERGMNILIKTPTYFTNFDD
YNNYPSTWSNVNTTNQDGLQGSANKLNGETKIKIPMSELKPYKRYVFSGYSKDPLTSNSIIV
KIKAKEEKTDYLVPEQGYTKFSYEFETTEKDSSNIEITLIGSGTTYLDNLSITELNSTPEILDEP
EVKIPTDQEIMDAHKIYFADLNFNPSTGNTYINGMYFAPTQTNKEALDYIQKYRVEATLQYSG
FKDIGTKDKEMRNYLGDPNQPKTNYVNLRSYFTGGENIMTYKKLRIYAITPDDRELLVLSVD
GGHHHHHH
**SEQ ID NO: 16 C155** 
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPTNFDDYNNYPSTWSNVNTTN
QDGLQGSANKLNGETKIKIPMSELKPYKRYVFSGYSKDPLTSNSIIVKIKAKEEKTDYLVPEQ
GYTKFSYEFETTEKDSSNIEITLIGSGTTYLDNLSITELNSTPEILDEPEVKIPTDQEIMDAHKIY
FADLNFNPSTGNTYINGMYFAPTQTNKEALDYIQKYRVEATLQYSGFKDIGTKDKEMRNYL
GDPNQPKTNYVNLRSYFTGGENIMTYKKLRIYAITPDDRELLVLSVDGGHHHHHH
**SEQ ID NO: 17 C156** 
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
 
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPGGGGGGNTTNQDGLQGSAN
KLNGETKIKIPMSELKPYKRYVFSGYSKDPLTSNSIIVKIKAKEEKTDYLVPEQGYTKFSYEFE
TTEKDSSNIEITLIGSGTTYLDNLSITELNSTPEILDEPEVKIPTDQEIMDAHKIYFADLNFNPST
GNTYINGMYFAPTQTNKEALDYIQKYRVEATLQYSGFKDIGTKDKEMRNYLGDPNQPKTNY
VNLRSYFTGGENIMTYKKLRIYAITPDDRELLVLSVDGGHHHHHH
**SEQ ID NO: 18: C49** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSHHHHHH
**SEQ ID NO: 19: C50** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSHHHHHH
**SEQ ID NO: 20: CDTa N terminal domain (residue 44 to residue 240) polypeptide sequence.** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIHHHHHH
**SEQ ID NO: 21: C67** 
MVCNTTYKAPIERPEDFLKDKEKAKEWERKEAERIEQKLERSEKEALESYKKDSVEISKYSQ
TRNYFDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGqYqVLLNHGSKFKINKIDSYKDGTITKLIVDATLIPHHHHHH
**SEQ ID NO: 22: C69** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGQYQVLLNHGSKFKINKIDSYKDGTITKLIVDATLIPHHHHHH
**SEQ ID NO: 23: C107** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGEYEVLLNHGSKFKINKIDSYKDGTITKLIVDATLIPHHHHHH
**SEQ ID NO: 24: C108** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGEYQVLLNHGSKFKIN
KIDSYKDGTITKLIVDATLIPHHHHHH
**SEQ ID NO: 25: C149** 
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQENQIGNNLSPGDTYPKKGLSPLALNTMDQRSSRLIPINYDQLKKLDAGK
QIKLETTQVSGNFGTKNSSGQIVTEGNSWSDYISQIDSISASIILDTENESYERRVTAKNLQD
PEDKTPELTIGEAIEKAFGATKKDGLLYFNDIPIDESCVELIFDDNTANKIKDSLKTLSDKKIYN
 
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVDHHHHHH
**SEQ ID NO: 26: C152** 
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQANQIGNNLSPGDTYPKKGLSPLALNTMAQFSSRLIPINYDQLKKLDAGK
QIKLETTQVSGNFGTKNSSGQIVTEGNSWSDYISQIDSISASIILDTENESYERRVTAKNLQD
PEDKTPELTIGEAIEKAFGATKKDGLLYFNDIPIDESCVELIFDDNTANKIKDSLKTLSDKKIYN
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVDHHHHHH
**SEQ ID NO: 27: C52** 
MTNFDDYNNYPSTWSNVNTTNQDGLQGSANKLNGETKIKIPMSELKPYKRYVFSGYSKDP
LTSNSIIVKIKAKEEKTDYLVPEQGYTKFSYEFETTEKDSSNIEITLIGSGTTYLDNLSITELNST
PEILDEPEVKIPTDQEIMDAHKIYFADLNFNPSTGNTYINGMYFAPTQTNKEALDYIQKYRVE
ATLQYSGFKDIGTKDKEMRNYLGDPNQPKTNYVNLRSYFTGGENIMTYKKLRIYAITPDDRE
LLVLSVDGGHHHHHH
**SEQ ID NO: 28: C53** 
MNTTNQDGLQGSANKLNGETKIKIPMSELKPYKRYVFSGYSKDPLTSNSIIVKIKAKEEKTDY
LVPEQGYTKFSYEFETTEKDSSNIEITLIGSGTTYLDNLSITELNSTPEILDEPEVKIPTDQEIM
DAHKIYFADLNFNPSTGNTYINGMYFAPTQTNKEALDYIQKYRVEATLQYSGFKDIGTKDKE
MRNYLGDPNQPKTNYVNLRSYFTGGENIMTYKKLRIYAITPDDRELLVLSVDGGHHHHHH
**SEQ ID NO: 29 amino acid sequence of Toxin A** MSLISKEELIKLAYSIRPRENEYKTILTNLDEYNKLTTNNNENKYLQLKKLNESIDVFMN 
KYKTSSRNRALSNLKKDILKEVILIKNSNTSPVEKNLHFVWIGGEVSDIALEYIKQWADI
NAEYNIKLWYDSEAFLVNTLKKAIVESSTTEALQLLEEEIQNPQFDNMKFYKKRMEFIYD
RQKRFINYYKSQINKPTVPTIDDIIKSHLVSEYNRDETVLESYRTNSLRKINSNHGIDIR
ANSLFTEQELLNIYSQELLNRGNLAAASDIVRLLALKNFGGVYLDVDMLPGIHSDLFKTI
SRPSSIGLDRWEMIKLEAIMKYKKYINNYTSENFDKLDQQLKDNFKLIIESKSEKSEIFS
KLENLNVSDLEIKIAFALGSVINQALISKQGSYLTNLVIEQVKNRYQFLNQHLNPAIESD
NNFTDTTKIFHDSLFNSATAENSMFLTKIAPYLQVGFMPEARSTISLSGPGAYASAYYDF
INLQENTIEKTLKASDLIEFKFPENNLSQLTEQEINSLWSFDQASAKYQFEKYVRDYTGG
SLSEDNGVDFNKNTALDKNYLLNNKIPSNNVEEAGSKNYVHYIIQLQGDDISYEATCNLF
SKNPKNSIIIQRNMNESAKSYFLSDDGESILELNKYRIPERLKNKEKVKVTFIGHGKDEF
NTSEFARLSVDSLSNEISSFLDTIKLDISPKNVEVNLLGCNMFSYDFNVEETYPGKLLLS
IMDKITSTLPDVNKNSITIGANQYEVRINSEGRKELLAHSGKWINKEEAIMSDLSSKEYI
FFDSIDNKLKAKSKNIPGLASISEDIKTLLLDASVSPDTKFILNNLKLNIESSIGDYIYY
EKLEPVKNIIHNSIDDLIDEFNLLENVSDELYELKKLNNLDEKYLISFEDISKNNSTYSV
RFINKSNGESVYVETEKEIFSKYSEHITKEISTIKNSIITDVNGNLLDNIQLDHTSQVNT
LNAAFFIQSLIDYSSNKDVLNDLSTSVKVQLYAQLFSTGLNTIYDSIQLVNLISNAVNDT
INVLPTITEGIPIVSTILDGINLGAAIKELLDEHDPLLKKELEAKVGVLAINMSLSIAAT
VASIVGIGAEVTIFLLPIAGISAGIPSLVNNELILHDKATSVVNYFNHLSESKKYGPLKT
EDDKILVPIDDLVISEIDFNNNSIKLGTCNILAMEGGSGHTVTGNIDHFFSSPSISSHIP
SLSIYSAIGIETENLDFSKKIMMLPNAPSRVFWWETGAVPGLRSLENDGTRLLDSIRDLY
PGKFYWRFYAFFDYAITTLKPVYEDTNIKIKLDKDTRNFIMPTITTNEIRNKLSYSFDGA
GGTYSLLLSSYPISTNINLSKDDLWIFNIDNEVREISIENGTIKKGKLIKDVLSKIDINK
NKLIIGNQTIDFSGDIDNKDRYIFLTCELDDKISLIIEINLVAKSYSLLLSGDKNYLISN
LSNTIEKINTLGLDSKNIAYNYTDESNNKYFGAISKTSQKSIIHYKKDSKNILEFYNDST
LEFNSKDFIAEDINVFMKDDINTITGKYYVDNNTDKSIDFSISLVSKNQVKVNGLYLNES
VYSSYLDFVKNSDGHHNTSNFMNLFLDNISFWKLFGFENINFVIDKYFTLVGKTNLGYVE
FICDNNKNIDIYFGEWKTSSSKSTIFSGNGRNVVVEPIYNPDTGEDISTSLDFSYEPLYG
IDRYINKVLIAPDLYTSLININTNYYSNEYYPEIIVLNPNTFHKKVNINLDSSSFEYKWS
TEGSDFILVRYLEESNKKILQKIRIKGILSNTQSFNKMSIDFKDIKKLSLGYIMSNFKSF
NSENELDRDHLGFKIIDNKTYYYDEDSKLVKGLININNSLFYFDPIEFNLVTGWQTINGK
KYYFDINTGAALTSYKIINGKHFYFNNDGVMQLGVFKGPDGFEYFAPANTQNNNIEGQAI
VYQSKFLTLNGKKYYFDNNSKAVTGWRIINNEKYYFNPNNAIAAVGLQVIDNNKYYFNPD
TAIISKGWQTVNGSRYYFDTDTAIAFNGYKTIDGKHFYFDSDCVVKIGVFSTSNGFEYFA
PANTYNNNIEGQAIVYQSKFLTLNGKKYYFDNNSKAVTGLQTIDSKKYYFNTNTAEAATG
WQTIDGKKYYFNTNTAEAATGWQTIDGKKYYFNTNTAIASTGYTIINGKHFYFNTDGIMQ
IGVFKGPNGFEYFAPANTDANNIEGQAILYQNEFLTLNGKKYYFGSDSKAVTGWRIINNK
KYYFNPNNAIAAIHLCTINNDKYYFSYDGILQNGYITIERNNFYFDANNESKMVTGVFKG
 
PNGFEYFAPANTHNNNIEGQAIVYQNKFLTLNGKKYYFDNDSKAVTGWQTIDGKKYYFNL
NTAEAATGWQTIDGKKYYFNLNTAEAATGWQTIDGKKYYFNTNTFIASTGYTSINGKHFY
FNTDGIMQIGVFKGPNGFEYFAPANTDANNIEGQAILYQNKFLTLNGKKYYFGSDSKAVT
GLRTIDGKKYYFNTNTAVAVTGWQTINGKKYYFNTNTSIASTGYTIISGKHFYFNTDGIM
QIGVFKGPDGFEYFAPANTDANNIEGQAIRYQNRFLYLHDNIYYFGNNSKAATGWVTIDG
NRYYFEPNTAMGANGYKTIDNKNFYFRNGLPQIGVFKGSNGFEYFAPANTDANNIEGQAI
RYQNRFLHLLGKIYYFGNNSKAVTGWQTINGKVYYFMPDTAMAAAGGLFEIDGVIYFFGV
DGVKAPGIYG
**SEQ ID NO: 30 - amino acid sequence of Toxin B** 
MSLVNRKQLEKMANVRFRTQEDEYVAILDALEEYHNMSENTVVEKYLKLKDINSLTDIYI
DTYKKSGRNKALKKFKEYLVTEVLELKNNNLTPVEKNLHFVWIGGQINDTAINYINQWKD
VNSDYNVNVFYDSNAFLINTLKKTVVESAINDTLESFRENLNDPRFDYNKFFRKRMEIIY
DKQKNFINYYKAQREENPELIIDDIVKTYLSNEYSKEIDELNTYIEESLNKITQNSGNDV
RNFEEFKNGESFNLYEQELVERWNLAAASDILRISALKEIGGMYLDVDMLPGIQPDLFES
IEKPSSVTVDFWEMTKLEAIMKYKEYIPEYTSEHFDMLDEEVQSSFESVLASKSDKSEIF
SSLGDMEASPLEVKIAFNSKGIINQGLISVKDSYCSNLIVKQIENRYKILNNSLNPAISE
DNDFNTTTNTFIDSIMAEANADNGRFMMELGKYLRVGFFPDVKTTINLSGPEAYAAAYQD
LLMFKEGSMNIHLIEADLRNFEISKTNISQSTEQEMASLWSFDDARAKAQFEEYKRNYFE
GSLGEDDNLDFSQNIVVDKEYLLEKISSLARSSERGYIHYIVQLQGDKISYEAACNLFAK
TPYDSVLFQKNIEDSEIAYYYNPGDGEIQEIDKYKIPSIISDRPKIKLTFIGHGKDEFNT
DIFAGFDVDSLSTEIEAAIDLAKEDISPKSIEINLLGCNMFSYSINVEETYPGKLLLKVK
DKISELMPSISQDSIIVSANQYEVRINSEGRRELLDHSGEWINKEESIIKDISSKEYISF
NPKENKITVKSKNLPELSTLLQEIRNNSNSSDIELEEKVMLTECEINVISNIDTQIVEER
IEEAKNLTSDSINYIKDEFKLIESISDALCDLKQQNELEDSHFISFEDISETDEGFSIRF
INKETGESIFVETEKTIFSEYANHITEEISKIKGTIFDTVNGKLVKKVNLDTTHEVNTLN
AAFFIQSLIEYNSSKESLSNLSVAMKVQVYAQLFSTGLNTITDAAKVVELVSTALDETID
LLPTLSEGLPIIATIIDGVSLGAAIKELSETSDPLLRQEIEAKIGIMAVNLTTATTAIIT
SSLGIASGFSILLVPLAGISAGIPSLVNNELVLRDKATKVVDYFKHVSLVETEGVFTLLD
DKIMMPQDDLVISEIDFNNNSIVLGKCEIWRMEGGSGHTVTDDIDHFFSAPSITYREPHL
SIYDVLEVQKEELDLSKDLMVLPNAPNRVFAWETGWTPGLRSLENDGTKLLDRIRDNYEG
EFYWRYFAFIADALITTLKPRYEDTNIRINLDSNTRSFIVPIITTEYIREKLSYSFYGSG
GTYALSLSQYNMGINIELSESDVWIIDVDNVVRDVTIESDKIKKGDLIEGILSTLSIEEN
KIILNSHEINFSGEVNGSNGFVSLTFSILEGINAIIEVDLLSKSYKLLISGELKILMLNS
NHIQQKIDYIGFNSELQKNIPYSFVDSEGKENGFINGSTKEGLFVSELPDVVLISKVYMD
DSKPSFGYYSNNLKDVKVITKDNVNILTGYYLKDDIKISLSLTLQDEKTIKLNSVHLDES
GVAEILKFMNRKGNTNTSDSLMSFLESMNIKSIFVNFLQSNIKFILDANFIISGTTSIGQ
 
FEFICDENDNIQPYFIKFNTLETNYTLYVGNRQNMIVEPNYDLDDSGDISSTVINFSQKY
LYGIDSCVNKVVISPNIYTDEINITPVYETNNTYPEVIVLDANYINEKINVNINDLSIRY
VWSNDGNDFILMSTSEENKVSQVKIRFVNVFKDKTLANKLSFNFSDKQDVPVSEIILSFT
PSYYEDGLIGYDLGLVSLYNEKFYINNFGMMVSGLIYINDSLYYFKPPVNNLITGFVTVG
DDKYYFNPINGGAASIGETIIDDKNYYFNQSGVLQTGVFSTEDGFKYFAPANTLDENLEG
EAIDFTGKLIIDENIYYFDDNYRGAVEWKELDGEMHYFSPETGKAFKGLNQIGDYKYYFN
SDGVMQKGFVSINDNKHYFDDSGVMKVGYTEIDGKHFYFAENGEMQIGVFNTEDGFKYFA
HHNEDLGNEEGEEISYSGILNFNNKIYYFDDSFTAVVGWKDLEDGSKYYFDEDTAEAYIG
LSLINDGQYYFNDDGIMQVGFVTINDKVFYFSDSGIIESGVQNIDDNYFYIDDNGIVQIG
VFDTSDGYKYFAPANTVNDNIYGQAVEYSGLVRVGEDVYYFGETYTIETGWIYDMENESD
KYYFNPETKKACKGINLIDDIKYYFDEKGIMRTGLISFENNNYYFNENGEMQFGYINIED
KMFYFGEDGVMQIGVFNTPDGFKYFAHQNTLDENFEGESINYTGWLDLDEKRYYFTDEYI
AATGSVIIDGEEYYFDPDTAQLVISE
**SEQ ID NO: 31: F1** 
MGWQTIDGKKYYFNTNTAIASTGYTIINGKHFYFNTDGIMQIGVFKGPNGFEYFAPANTDAN
NIEGQAILYQNEFLTLNGKKYYFGSDSKAVTGWRIINNKKYYFNPNNAIAAIHLCTINNDKYYF
SYDGILQNGYITIERNNFYFDANNESKMVTGVFKGPNGFEYFAPANTHNNNIEGQAIVYQNK
FLTLNGKKYYFDNDSKAVTGWQTIDGKKYYFNLNTAEAATGWQTIDGKKYYFNLNTAEAAT
GWQTIDGKKYYFNTNTFIASTGYTSINGKHFYFNTDGIMQIGVFKGPNGFEYFAPANTDANN
IEGQAILYQNKFLTLNGKKYYFGSDSKAVTGLRTIDGKKYYFNTNTAVAVTGWQTINGKKYY
FNTNTSIASTGYTIISGKHFYFNTDGIMQIGVFKGPDGFEYFAPANTDANNIEGQAIRYQNRF
LYLHDNIYYFGNNSKAATGWVTIDGNRYYFEPNTAMGANGYKTIDNKNFYFRNGLPQIGVF
KGSNGFEYFAPANTDANNIEGQAIRYQNRFLHLLGKIYYFGNNSKAVTGWQTINGKVYYFM
PDTAMAAAGGLFEIDGVIYFFGVDGVKAPGFVSINDNKHYFDDSGVMKVGYTEIDGKHFYF
AENGEMQIGVFNTEDGFKYFAHHNEDLGNEEGEEISYSGILNFNNKIYYFDDSFTAVVGWK
DLEDGSKYYFDEDTAEAYIGLSLINDGQYYFNDDGIMQVGFVTINDKVFYFSDSGIIESGVQN
IDDNYFYIDDNGIVQIGVFDTSDGYKYFAPANTVNDNIYGQAVEYSGLVRVGEDVYYFGETY
TIETGWIYDMENESDKYYFNPETKKACKGINLIDDIKYYFDEKGIMRTGLISFENNNYYFNEN
GEMQFGYINIEDKMFYFGEDGVMQIGVFNTPDGFKYFAHQNTLDENFEGESINYTGWLDLD
EKRYYFTDEYIAATGSVIIDGEEYYFDPDTAQLVISE
**SEQ ID NO: 32: F2** 
MGWQTIDGKKYYFNTNTAIASTGYTIINGKHFYFNTDGIMQIGVFKGPNGFEYFAPANTDAN
NIEGQAILYQNEFLTLNGKKYYFGSDSKAVTGWRIINNKKYYFNPNNAIAAIHLCTINNDKYYF
SYDGILQNGYITIERNNFYFDANNESKMVTGVFKGPNGFEYFAPANTHNNNIEGQAIVYQNK
 
FLTLNGKKYYFDNDSKAVTGWQTIDGKKYYFNLNTAEAATGWQTIDGKKYYFNLNTAEAAT
GWQTIDGKKYYFNTNTFIASTGYTSINGKHFYFNTDGIMQIGVFKGPNGFEYFAPANTDANN
IEGQAILYQNKFLTLNGKKYYFGSDSKAVTGLRTIDGKKYYFNTNTAVAVTGWQTINGKKYY
FNTNTSIASTGYTIISGKHFYFNTDGIMQIGVFKGPDGFEYFAPANTDANNIEGQAIRYQNRF
LYLHDNIYYFGNNSKAATGWVTIDGNRYYFEPNTAMGANGYKTIDNKNFYFRNGLPQIGVF
KGSNGFEYFAPANTDANNIEGQAIRYQNRFLHLLGKIYYFGNNSKAVTGWQTINGKVYYFM
PDTAMAAAGGLNQIGDYKYYFNSDGVMQKGFVSINDNKHYFDDSGVMKVGYTEIDGKHFY
FAENGEMQIGVFNTEDGFKYFAHHNEDLGNEEGEEISYSGILNFNNKIYYFDDSFTAVVGW
KDLEDGSKYYFDEDTAEAYIGLSLINDGQYYFNDDGIMQVGFVTINDKVFYFSDSGIIESGVQ
NIDDNYFYIDDNGIVQIGVFDTSDGYKYFAPANTVNDNIYGQAVEYSGLVRVGEDVYYFGET
YTIETGWIYDMENESDKYYFNPETKKACKGINLIDDIKYYFDEKGIMRTGLISFENNNYYFNE
NGEMQFGYINIEDKMFYFGEDGVMQIGVFNTPDGFKYFAHQNTLDENFEGESINYTGWLDL
DEKRYYFTDEYIAATGSVIIDGEEYYFDPDTAQLVISE
**SEQ ID NO: 33: F3** 
MGWQTIDGKKYYFNTNTAIASTGYTIINGKHFYFNTDGIMQIGVFKGPNGFEYFAPANTDAN
NIEGQAILYQNEFLTLNGKKYYFGSDSKAVTGWRIINNKKYYFNPNNAIAAIHLCTINNDKYYF
SYDGILQNGYITIERNNFYFDANNESKMVTGVFKGPNGFEYFAPANTHNNNIEGQAIVYQNK
FLTLNGKKYYFDNDSKAVTGWQTIDGKKYYFNLNTAEAATGWQTIDGKKYYFNLNTAEAAT
GWQTIDGKKYYFNTNTFIASTGYTSINGKHFYFNTDGIMQIGVFKGPNGFEYFAPANTDANN
IEGQAILYQNKFLTLNGKKYYFGSDSKAVTGLRTIDGKKYYFNTNTAVAVTGWQTINGKKYY
FNTNTSIASTGYTIISGKHFYFNTDGIMQIGVFKGPDGFEYFAPANTDANNIEGQAIRYQNRF
LYLHDNIYYFGNNSKAATGWVTIDGNRYYFEPNTAMGANGYKTIDNKNFYFRNGLPQIGVF
KGSNGFEYFAHHNEDLGNEEGEEISYSGILNFNNKIYYFDDSFTAVVGWKDLEDGSKYYFD
EDTAEAYIGLSLINDGQYYFNDDGIMQVGFVTINDKVFYFSDSGIIESGVQNIDDNYFYIDDN
GIVQIGVFDTSDGYKYFAPANTVNDNIYGQAVEYSGLVRVGEDVYYFGETYTIETGWIYDME
NESDKYYFNPETKKACKGINLIDDIKYYFDEKGIMRTGLISFENNNYYFNENGEMQFGYINIE
DKMFYFGEDGVMQIGVFNTPDGFKYFAHQNTLDENFEGESINYTGWLDLDEKRYYFTDEYI
AATGSVIIDGEEYYFDPDTAQLVISE
**SEQ ID NO: 34: F4** 
MGWQTIDGKKYYFNTNTAIASTGYTIINGKHFYFNTDGIMQIGVFKGPNGFEYFAPANTDAN
NIEGQAILYQNEFLTLNGKKYYFGSDSKAVTGWRIINNKKYYFNPNNAIAAIHLCTINNDKYYF
SYDGILQNGYITIERNNFYFDANNESKMVTGVFKGPNGFEYFAPANTHNNNIEGQAIVYQNK
FLTLNGKKYYFDNDSKAVTGWQTIDGKKYYFNLNTAEAATGWQTIDGKKYYFNLNTAEAAT
GWQTIDGKKYYFNTNTFIASTGYTSINGKHFYFNTDGIMQIGVFKGPNGFEYFAPANTDANN
 
IEGQAILYQNKFLTLNGKKYYFGSDSKAVTGLRTIDGKKYYFNTNTAVAVTGWQTINGKKYY
FNTNTSIASTGYTIISGKHFYFNTDGIMQIGVFKGPDGFEYFAPANTDANNIEGQAIRYQNRF
LYLHDNIYYFGNNSKAATGWVTIDGNRYYFEPNTAMGANGYKTIDNKNFYFRNGLPQIGVF
KGSNGFEYFAPANTDANNIEGQAIRYQNRFLHLLGKIYYFGNNSKAVTGWQTINGKVYYFM
PDTAMAAAGGETIIDDKNYYFNQSGVLQTGVFSTEDGFKYFAPANTLDENLEGEAIDFTGKL
IIDENIYYFDDNYRGAVEWKELDGEMHYFSPETGKAFKGLNQIGDYKYYFNSDGVMQKGFV
SINDNKHYFDDSGVMKVGYTEIDGKHFYFAENGEMQIGVFNTEDGFKYFAHHNEDLGNEE
GEEISYSGILNFNNKIYYFDDSFTAVVGWKDLEDGSKYYFDEDTAEAYIGLSLINDGQYYFND
DGIMQVGFVTINDKVFYFSDSGIIESGVQNIDDNYFYIDDNGIVQIGVFDTSDGYKYFAPANT
VNDNIYGQAVEYSGLVRVGEDVYYFGETYTIETGWIYDMENESDKYYFNPETKKACKGINLI
DDIKYYFDEKGIMRTGLISFENNNYYFNENGEMQFGYINIEDKMFYFGEDGVMQIGVFNTPD
GFKYFAHQNTLDENFEGESINYTGWLDLDEKRYYFTDEYIAATGSVIIDGEEYYFDPDTAQL
VISE
**SEQ ID NO: 35: F5** 
MGWQTIDGKKYYFNTNTAIASTGYTIINGKHFYFNTDGIMQIGVFKGPNGFEYFAPANTDAN
NIEGQAILYQNEFLTLNGKKYYFGSDSKAVTGWRIINNKKYYFNPNNAIAAIHLCTINNDKYYF
SYDGILQNGYITIERNNFYFDANNESKMVTGVFKGPNGFEYFAPANTHNNNIEGQAIVYQNK
FLTLNGKKYYFDNDSKAVTGWQTIDGKKYYFNLNTAEAATGWQTIDGKKYYFNLNTAEAAT
GWQTIDGKKYYFNTNTFIASTGYTSINGKHFYFNTDGIMQIGVFKGPNGFEYFAPANTDANN
IEGQAILYQNKFLTLNGKKYYFGSDSKAVTGLRTIDGKKYYFNTNTAVAVTGWQTINGKKYY
FNTNTSIASTGYTIISGKHFYFNTDGIMQIGVFKGPDGFEYFAPANTDANNIEGQAIRYQNRF
LYLHDNIYYFGNNSKAATGWVTIDGNRYYFEPNTAMGANGYKTIDNKNFYFRNGLPQIGVF
KGSNGFEYFAPANTDANNIEGQAIRYQNRFLHLLGKIYYFGNNSKAVTGWQTINGKVYYFM
PDTAMAAAGGLFEIDGVIYFFGVDGVKAPGIYGGGFVSINDNKHYFDDSGVMKVGYTEIDG
KHFYFAENGEMQIGVFNTEDGFKYFAHHNEDLGNEEGEEISYSGILNFNNKIYYFDDSFTAV
VGWKDLEDGSKYYFDEDTAEAYIGLSLINDGQYYFNDDGIMQVGFVTINDKVFYFSDSGIIE
SGVQNIDDNYFYIDDNGIVQIGVFDTSDGYKYFAPANTVNDNIYGQAVEYSGLVRVGEDVYY
FGETYTIETGWIYDMENESDKYYFNPETKKACKGINLIDDIKYYFDEKGIMRTGLISFENNNY
YFNENGEMQFGYINIEDKMFYFGEDGVMQIGVFNTPDGFKYFAHQNTLDENFEGESINYTG
WLDLDEKRYYFTDEYIAATGSVIIDGEEYYFDPDTAQLVISE
**SEQ ID NO: 36: CDTa C34 construct polypeptide sequence** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
 
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGEYEVLLNHGSKFKINKIDSYKDGTITKLIVDATLIPHHHHHH
**SEQ ID NO: 37: CDTb C118 construct. CDTb without signal peptide, without prodomain, without CDTa-binding domain and without Receptor-binding domain** 
MAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG
DTLSTIKAQENQIGNNLSPGDTYPKKGLSPLALNTMDQFSSRLIPINYDQLKKLDAGKQIKLE
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPGGHHHHHH
**SEQ ID NO: 38: CDTb C46 construct. CDTb' (minus signal peptide) polypeptide sequence** 
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQENQIGNNLSPGDTYPKKGLSPLALNTMDQFSSRLIPINYDQLKKLDAGK
QIKLETTQVSGNFGTKNSSGQIVTEGNSWSDYISQIDSISASIILDTENESYERRVTAKNLQD
PEDKTPELTIGEAIEKAFGATKKDGLLYFNDIPIDESCVELIFDDNTANKIKDSLKTLSDKKIYN
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVDHHHHHH
**SEQ ID NO: 39: C44 construct. CDTa mutation E428Q polypeptide sequence.** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
 
LTSPEYDFNKLENIDAFKSKWEGQALSYPNFISTSIGSVNMSAFAKRKIVLRITIPKGSPGAYL
SAIPGYAGqYEVLLNHGSKFKINKIDSYKDGTITKLIVDATLIPHHHHHH
**SEQ ID NO: 40: C54 construct. CDTa mutation E430Q polypeptide sequence.** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGEYqVLLNHGSKFKINKIDSYKDGTITKLIVDATLIPHHHHHH
**SEQ ID NO: 41: C68** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFISTSIGSVNMSAFAKAKIVLRITIPKGSPGAYL
SAIPGYAGQYQVLLNHGSKFKINKIDSYKDGTITKLIVDATLIPHHHHHH
**SEQ ID NO: 42: Amino acid sequence of CDTa without signal peptide, with six mutations (R345A, Q350A, N385A, R402A, S388F, E428Q, aa 44-463) (C110** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGQYEVLLNHGSKFKINKIDSYKDGTITKLIVDATLIPHHHHHH
**SEQ ID NO: 43: Amino acid sequence of C164** (KO pore forming mutation F455R on CdtB without prodomain) 
MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR
YYNTGTAPMYKVTPTTNLVLDGDTLSTIKAQENQIGNNLSPGDTYPKKGLSPLALNTMDQR
 
SSRLIPINYDQLKKLDAGKQIKLETTQVSGNFGTKNSSGQIVTEGNSWSDYISQIDSISASIILD
TENESYERRVTAKNLQDPEDKTPELTIGEAIEKAFGATKKDGLLYFNDIPIDESCVELIFDDNT
ANKIKDSLKTLSDKKIYNVKLERGMNILIKTPTYFTNFDDYNNYPSTWSNVNTTNQDGLQGS
ANKLNGETKIKIPMSELKPYKRYVFSGYSKDPLTSNSIIVKIKAKEEKTDYLVPEQGYTKFSYE
FETTEKDSSNIEITLIGSGTTYLDNLSITELNSTPEILDEPEVKIPTDQEIMDAHKIYFADLNFNP
STGNTYINGMYFAPTQTNKEALDYIQKYRVEATLQYSGFKDIGTKDKEMRNYLGDPNQPKT
NYVNLRSYFTGGENIMTYKKLRIYAITPDDRELLVLSVD
**SEQ ID NO: 44: Amino acid sequence of C166** (KO pore forming mutation E426A-D453A on CdtB without prodomain) 
MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR
YYNTGTAPMYKVTPTTNLVLDGDTLSTIKAQANQIGNNLSPGDTYPKKGLSPLALNTMAQFS
SRLIPINYDQLKKLDAGKQIKLETTQVSGNFGTKNSSGQIVTEGNSWSDYISQIDSISASIILDT
ENESYERRVTAKNLQDPEDKTPELTIGEAIEKAFGATKKDGLLYFNDIPIDESCVELIFDDNTA
NKIKDSLKTLSDKKIYNVKLERGMNILIKTPTYFTNFDDYNNYPSTWSNVNTTNQDGLQGSA
NKLNGETKIKIPMSELKPYKRYVFSGYSKDPLTSNSIIVKIKAKEEKTDYLVPEQGYTKFSYEF
ETTEKDSSNIEITLIGSGTTYLDNLSITELNSTPEILDEPEVKIPTDQEIMDAHKIYFADLNFNPS
TGNTYINGMYFAPTQTNKEALDYIQKYRVEATLQYSGFKDIGTKDKEMRNYLGDPNQPKTN
YVNLRSYFTGGENIMTYKKLRIYAITPDDRELLVLSVD
**SEQ ID NO: 45: Nucleic acid sequence of C164** (KO pore forming mutation F455R on CdtB without prodomain) 
ATGAGCGATTGGGAAGATGAAGATCTGGATACCGATAATGATAATATTCCGGATAGCTA
TGAACGCAATGGCTATACCATTAAAGATCTGATTGCCGTGAAATGGGAAGATAGCTTTG
CAGAACAGGGCTATAAGAAATATGTGAGCAATTATCTGGAAAGCAATACCGCAGGCGAT
CCGTATACCGATTATGAAAAAGCAAGCGGCAGCTTTGATAAAGCCATTAAAACCGAAGC
ACGTGATCCGCTGGTTGCAGCATATCCGATTGTTGGTGTTGGTATGGAAAAACTGATTA
TTAGCACCAATGAACATGCAAGCACCGATCAGGGTAAAACCGTTAGCCGTGCAACCAC
CAATAGCAAAACCGAAAGCAATACAGCCGGTGTTAGCGTTAATGTTGGTTATCAGAATG
GTTTTACCGCCAATGTGACCACCAATTATAGCCATACCACCGATAATAGCACCGCAGTT
CAGGATAGCAATGGTGAAAGCTGGAATACCGGTCTGAGCATTAACAAAGGTGAAAGCG
CATATATCAATGCCAATGTGCGCTATTATAACACCGGCACCGCACCGATGTATAAAGTT
ACCCCGACCACCAATCTGGTTCTGGATGGTGATACCCTGAGTACCATTAAAGCACAAGA
 
AAATCAGATTGGCAATAATCTGAGTCCGGGTGATACCTATCCGAAAAAGGTCTGAGTC
CGCTGGCACTGAATACCATGGATCAGCGCAGCAGCCGTCTGATTCCGATTAACTATGAT
CAGCTGAAAAACTGGATGCCGGTAAACAAATCAAACTGGAAACCACCCAGGTTAGCG
GTAATTTTGGCACCAAAAATTCAAGCGGTCAGATTGTTACCGAAGGTAATAGCTGGTCA
GATTATATCAGCCAGATTGATAGCATTAGCGCCAGCATTATTCTGGATACAGAAAATGAA
AGCTATGAACGTCGTGTGACCGCAAAAAATCTGCAGGACCCGGAAGATAAAACACCGG
AACTGACCATTGGTGAAGCAATTGAAAAAGCATTTGGTGCCACCAAAAAAGATGGCCTG
CTGTATTTTAACGATATTCCGATTGATGAAAGCTGCGTGGAACTGATTTTTGATGATAAT
ACCGCCAATAAAATCAAAGATAGCCTGAAAACCCTGAGCGACAAAAAAATCTATAATGT
GAAATTAGAACGCGGTATGAATATTCTAATTAAAACCCCGACCTATTTTACCAATTTTGA
TGATTATAACAATTATCCGAGCACTTGGAGCAATGTGAATACCACCAATCAGGATGGTC
TGCAGGGTAGCGCAAATAAACTGAATGGTGAAACCAAAATCAAAATTCCGATGAGCGAA
CTGAAACCGTATaAACGTTATGTGTTTAGCGGCTATAGCAAAGATCCGCTGACCAGCAA
TAGCATTATTGTGAAAATCAAAGCCAAAGAAGAAAAACCGATTATCTGGTTCCGGAAC
AGGGTTATACCAAATTTAGCTATGAATTTGAAACCACCGAAAAAGATAGCAGTAATATTG
AAATTACCCTGATTGGTAGCGGCACCACCTATCTGGATAATCTGAGTATTACCGAACTG
AATAGCACACCGGAAATTCTGGATGAACCGGAAGTGAAAATTCCGACCGATCAAGAAAT
TATGGATGCCCATAAAATCTATTTTGCCGATCTGAACTTTAATCCGAGCACCGGCAATAC
CTATATTAACGGCATGTATTTTGCACCGACCCAGACCAATAAAGAAGCCCTGGATTATAT
TCAGAAATATCGTGTTGAAGCCACCCTGCAGTATAGCGGTTTTAAAGATATTGGCACCA
AAGATAAAGAAATGCGTAATTATCTGGGCGATCCGAATCAGCCGAAAACCAATTATGTT
AATCTGCGCAGCTATTTTACCGGTGGCGAAAACATTATGACCTACAAAAAACTGCGCAT
TTATGCCATTACACCGGATGATCGTGAACTGCTGGTTCTGAGCGTTGATCA
**SEQ ID NO: 46: Nucleic acid sequence of C166** (KO pore forming mutation E426A-D453A on CdtB without prodomain) 
ATGAGCGATTGGGAAGATGAAGATCTGGATACCGATAATGATAATATTCCGGATAGCTA
TGAACGCAATGGCTATACCATTAAAGATCTGATTGCCGTGAAATGGGAAGATAGCTTTG
CAGAACAGGGCTATAAGAAATATGTGAGCAATTATCTGGAAAGCAATACCGCAGGCGAT
CCGTATACCGATTATGAAAAAGCAAGCGGCAGCTTTGATAAAGCCATTAAAACCGAAGC
ACGTGATCCGCTGGTTGCAGCATATCCGATTGTTGGTGTTGGTATGGAAAAACTGATTA
TTAGCACCAATGAACATGCAAGCACCGATCAGGGTAAAACCGTTAGCCGTGCAACCAC
CAATAGCAAAACCGAAAGCAATACAGCCGGTGTTAGCGTTAATGTTGGTTATCAGAATG
GTTTTACCGCCAATGTGACCACCAATTATAGCCATACCACCGATAATAGCACCGCAGTT
CAGGATAGCAATGGTGAAAGCTGGAATACCGGTCTGAGCATTAACAAAGGTGAAAGCG
CATATATCAATGCCAATGTGCGCTATTATAACACCGGCACCGCACCGATGTATAAAGTT
 
ACCCCGACCACCAATCTGGTTCTGGATGGTGATACCCTGAGTACCATTAAAGCACAAGC
RAATCAGATTGGCAATAATCTGAGTCCGGGTGATACCTATCCGAAAAAGGTCTGAGTC
CGCTGGCACTGAATACCATGGCGCAGTTTAGCAGCCGTCTGATTCCGATTAACTATGAT
CAGCTGAAAAACTGGATGCCGGTAAACAAATCAAACTGGAAACCACCCAGGTTAGCG
GTAATTTTGGCACCAAAAATTCAAGCGGTCAGATTGTTACCGAAGGTAATAGCTGGTCA
GATTATATCAGCCAGATTGATAGCATTAGCGCCAGCATTATTCTGGATACAGAAAATGAA
AGCTATGAACGTCGTGTGACCGCAAAAAATCTGCAGGACCCGGAAGATAAAACACCGG
AACTGACCATTGGTGAAGCAATTGAAAAAGCATTTGGTGCCACCAAAAAAGATGGCCTG
CTGTATTTTAACGATATTCCGATTGATGAAAGCTGCGTGGAACTGATTTTTGATGATAAT
ACCGCCAATAAAATCAAAGATAGCCTGAAAACCCTGAGCGACAAAAAAATCTATAATGT
GAAATTAGAACGCGGTATGAATATTCTAATTAAAACCCCGACCTATTTTACCAATTTTGA
TGATTATAACAATTATCCGAGCACTTGGAGCAATGTGAATACCACCAATCAGGATGGTC
TGCAGGGTAGCGCAAATAAACTGAATGGTGAAACCAAAATCAAAATTCCGATGAGCGAA
CTGAAACCGTATAAACGTTATGTGTTTAGCGGCTATAGCAAAGATCCGCTGACCAGCAA
TAGCATTATTGTGAAAATCAAAGCCAAAGAAGAAAAACCGATTATCTGGTTCCGGAAC
AGGGTTATACCAAATTTAGCTATGAATTTGAAACCACCGAAAAAGATAGCAGTAATATTG
AAATTACCCTGATTGGTAGCGGCACCACCTATCTGGATAATCTGAGTATTACCGAACTG
AATAGCACACCGGAAATTCTGGATGAACCGGAAGTGAAAATTCCGACCGATCAAGAAAT
TATGGATGCCCATAAAATCTATTTTGCCGATCTGAACTTTAATCCGAGCACCGGCAATAC
CTATATTAACGGCATGTATTTTGCACCGACCCAGACCAATAAAGAAGCCCTGGATTATAT
TCAGAAATATCGTGTTGAAGCCACCCTGCAGTATAGCGGTTTTAAAGATATTGGCACCA
AAGATAAAGAAATGCGTAATTATCTGGGCGATCCGAATCAGCCGAAAACCAATTATGTT
AATCTGCGCAGCTATTTTACCGGTGGCGAAAACATTATGACCTACAAAAAACTGCGCAT
TTATGCCATTACACCGGATGATCGTGAACTGCTGGTTCTGAGCGTTGATCAC
**SEQ ID 47 - CDTb C37 construct. CDTb' (minus signal peptide) ligated to Glutathione-S-transferase protein (GST underlined) polypeptide sequence, without histag.** 
MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR
DDVLMQVNTESTISNTLKVNMKKGKEYKVRIELQDKNLGSIDNLSSPNLYWELDGMKKIIPE
ENLFLRDYSNIEKDDPFIPNNNFFDPKLMSDWEDEDLDTDNDNIPDSYERNGYTIKDLIAVK
WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA
VQDSNGESWNTGLSINKGESAYINANVRYYNTGTAPMYKVTPTTNLVLDGDTLSTIKAQEN
 
QIGNNLSPGDTYPKKGLSPLALNTMDQFSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVD
**SEQ ID NO: 48 - amino acid sequence of CDTb with prodomain removed (CDTb", aa212-876) (C55), without histag** 
MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR
YYNTGTAPMYKVTPTTNLVLDGDTLSTIKAQENQIGNNLSPGDTYPKKGLSPLALNTMDQFS
SRLIPINYDQLKKLDAGKQIKLETTQVSGNFGTKNSSGQIVTEGNSWSDYISQIDSISASIILDT
ENESYERRVTAKNLQDPEDKTPELTIGEAIEKAFGATKKDGLLYFNDIPIDESCVELIFDDNTA
NKIKDSLKTLSDKKIYNVKLERGMNILIKTPTYFTNFDDYNNYPSTWSNVNTTNQDGLQGSA
NKLNGETKIKIPMSELKPYKRYVFSGYSKDPLTSNSIIVKIKAKEEKTDYLVPEQGYTKFSYEF
ETTEKDSSNIEITLIGSGTTYLDNLSITELNSTPEILDEPEVKIPTDQEIMDAHKIYFADLNFNPS
TGNTYINGMYFAPTQTNKEALDYIQKYRVEATLQYSGFKDIGTKDKEMRNYLGDPNQPKTN
YVNLRSYFTGGENIMTYKKLRIYAITPDDRELLVLSVD
**SEQ ID NO: 49 C116 without histag** 
MSDWEDEDLDTDNDNIPDSYERNGYTIKDLIAVKWEDSFAEQGYKKYVSNYLESNTAGDPY
TDYEKASGSFDKAIKTEARDPLVAAYPIVGVGMEKLIISTNEHASTDQGKTVSRATTNSKTES
NTAGVSVNVGYQNGFTANVTTNYSHTTDNSTAVQDSNGESWNTGLSINKGESAYINANVR
YYNTGTAPMYKVTPTTNLVLDGDTLSTIKAQENQIGNNLSPGDTYPKKGLSPLALNTMDQFS
SRLIPINYDQLKKLDAGKQIKLETTQVSGNFGTKNSSGQIVTEGNSWSDYISQIDSISASIILDT
ENESYERRVTAKNLQDPEDKTPELTIGEAIEKAFGATKKDGLLYFNDIPIDESCVELIFDDNTA
NKIKDSLKTLSDKKIYNVKLERGMNILIKTPGG
**SEQ ID NO: 50 C117 without histag** 
MAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG
DTLSTIKAQENQIGNNLSPGDTYPKKGLSPLALNTMDQFSSRLIPINYDQLKKLDAGKQIKLE
 
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPTYFTNFDDYNNYPSTWSNVNTTNQDGLQGSANKLNGETKIKIPMSELKPYKRY
VFSGYSKDPLTSNSIIVKIKAKEEKTDYLVPEQGYTKFSYEFETTEKDSSNIEITLIGSGTTYLD
NLSITELNSTPEILDEPEVKIPTDQEIMDAHKIYFADLNFNPSTGNTYINGMYFAPTQTNKEAL
DYIQKYRVEATLQYSGFKDIGTKDKEMRNYLGDPNQPKTNYVNLRSYFTGGENIMTYKKLRI
YAITPDDRELLVLSVD
**SEQ ID NO: 51 C123 CDTb sequence with F455R, without histag.** 
MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR
DDVLMQVNTESTISNTLKVNMKKGKEYKVRIELQDKNLGSIDNLSSPNLYWELDGMKKIIPE
ENLFLRDYSNIEKDDPFIPNNNFFDPKLMSDWEDEDLDTDNDNIPDSYERNGYTIKDLIAVK
WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA
VQDSNGESWNTGLSINKGESAYINANVRYYNTGTAPMYKVTPTTNLVLDGDTLSTIKAQEN
QIGNNLSPGDTYPKKGLSPLALNTMDQRSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVD
**SEQ ID NO: 52 C126 CDTb sequence in which residues 426 (Glutamate) and 453 (Aspartate) were both mutated into alanine, without histag.** 
MSPILGYWKIKGLVQPTRLLLEYLEEKYEEHLYERDEGDKWRNKKFELGLEFPNLPYYIDGD
VKLTQSMAIIRYIADKHNMLGGCPKERAEISMLEGAVLDIRYGVSRIAYSKDFETLKVDFLSKL
PEMLKMFEDRLCHKTYLNGDHVTHPDFMLYDALDVVLYMDPMCLDAFPKLVCFKKRIEAIP
QIDKYLKSSKYIAWPLQGWQATFGGGDHPPKSDLEVLFQGPLGSHMEIVNEDILPNNGLMG
YYFTDEHFKDLKLMAPIKDGNLKFEEKKVDKLLDKDKSDVKSIRWTGRIIPSKDGEYTLSTDR
DDVLMQVNTESTISNTLKVNMKKGKEYKVRIELQDKNLGSIDNLSSPNLYWELDGMKKIIPE
 
ENLFLRDYSNIEKDDPFIPNNNFFDPKLMSDWEDEDLDTDNDNIPDSYERNGYTIKDLIAVK
WEDSFAEQGYKKYVSNYLESNTAGDPYTDYEKASGSFDKAIKTEARDPLVAAYPIVGVGME
KLIISTNEHASTDQGKTVSRATTNSKTESNTAGVSVNVGYQNGFTANVTTNYSHTTDNSTA
VQDSNGESWNTGLSINKGESAYINANVRYYNTGTAPMYKVTPTTNLVLDGDTLSTIKAQAN
QIGNNLSPGDTYPKKGLSPLALNTMAQFSSRLIPINYDQLKKLDAGKQIKLETTQVSGNFGT
KNSSGQIVTEGNSWSDYISQIDSISASIILDTENESYERRVTAKNLQDPEDKTPELTIGEAIEK
AFGATKKDGLLYFNDIPIDESCVELIFDDNTANKIKDSLKTLSDKKIYNVKLERGMNILIKTPTY
FTNFDDYNNYPSTWSNVNTTNQDGLQGSANKLNGETKIKIPMSELKPYKRYVFSGYSKDPL
TSNSIIVKIKAKEEKTDYLVPEQGYTKFSYEFETTEKDSSNIEITLIGSGTTYLDNLSITELNSTP
EILDEPEVKIPTDQEIMDAHKIYFADLNFNPSTGNTYINGMYFAPTQTNKEALDYIQKYRVEA
TLQYSGFKDIGTKDKEMRNYLGDPNQPKTNYVNLRSYFTGGENIMTYKKLRIYAITPDDREL
LVLSVD
**SEQ ID NO: 53 C127 without histag** 
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTAV
TTNYSHTTDNSTAVQDSNGESWNTGLSINKGESAYINANVRYYNTGTAPMYKVTPTTNLVL
DGDTLSTIKAQENQIGNNLSPGDTYPKKGLSPLALNTMDQFSSRLIPINYDQLKKLDAGKQIK
LETTQVSGNFGTKNSSGQIVTEGNSWSDYISQIDSISASIILDTENESYERRVTAgggPELTIG
EAIEKAFGATKKDGLLYFNDIPIDESCVELIFDDNTANKIKDSLKTLSDKKIYNVKLERGMNILI
KTPTYFTNFDDYNNYPSTWSNVNTTNQDGLQGSANKLNGETKIKIPMSELKPYKRYVFSGY
SKDPLTSNSIIVKIKAKEEKTDYLVPEQGYTKFSYEFETTEKDSSNIEITLIGSGTTYLDNLSITE
LNSTPEILDEPEVKIPTDQEIMDAHKIYFADLNFNPSTGNTYINGMYFAPTQTNKEALDYIQKY
RVEATLQYSGFKDIGTKDKEMRNYLGDPNQPKTNYVNLRSYFTGGENIMTYKKLRIYAITPD
DRELLVLSVD
**SEQ ID NO: 54 C128 without histag** 
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
 
VLDGDTLSTIKAQENQIGNNLSPGDTYPKKGLSPLALNTMDQFSSRLIPINYDQLKKLDAGK
QIKLETTQVSGNFGTKNSSGQIVTEGNSWSDYISQIDSISASIILDTENESYERRVTAKNLQgP
gggTPELTIGEAIEKAFGATKKDGLLYFNDIPIDESCVELIFDDNTANKIKDSLKTLSDKKIYNVK
LERGMNILIKTPTYFTNFDDYNNYPSTWSNVNTTNQDGLQGSANKLNGETKIKIPMSELKPY
KRYVFSGYSKDPLTSNSIIVKIKAKEEKTDYLVPEQGYTKFSYEFETTEKDSSNIEITLIGSGTT
YLDNLSITELNSTPEILDEPEVKIPTDQEIMDAHKIYFADLNFNPSTGNTYINGMYFAPTQTNK
EALDYIQKYRVEATLQYSGFKDIGTKDKEMRNYLGDPNQPKTNYVNLRSYFTGGENIMTYK
KLRIYAITPDDRELLVLSVD
**SEQ ID NO: 55 C139 without histag** 
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPSDWEDEDLDTDNDNIPDSYE
RNGYTIKDLIAVKWEDSFAEQGYKKYVSNYLESNTAGDPYTDYEKASGSFDKAIKTEARDPL
VAAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG
DTLSTIKAQENQIGNNLSPGDTYPKKGLSPLALNTMDQFSSRLIPINYDQLKKLDAGKQIKLE
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPTYFTNFDDYNNYPSTWSNVNTTNQDGLQGSANKLNGETKIKIPMSELKPYKRY
VFSGYSKDPLTSNSIIVKIKAKEEKTDYLVPEQGYTKFSYEFETTEKDSSNIEITLIGSGTTYLD
NLSITELNSTPEILDEPEVKIPTDQEIMDAHKIYFADLNFNPSTGNTYINGMYFAPTQTNKEAL
DYIQKYRVEATLQYSGFKDIGTKDKEMRNYLGDPNQPKTNYVNLRSYFTGGENIMTYKKLRI
YAITPDDRELLVLSVDGG
**SEQ ID NO: 56 C145 without histag** 
MVYNTTYKAPIERPEDFLKDKEKAKEWERKEAERIEQKLERSEKEALESYKKDSVEISKYSQ
TRNYFDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPGGGGGGAYPIVGVGMEKLIIS
 
TNEHASTDQGKTVSRATTNSKTESNTAGVSVNVGYQNGFTANVTTNYSHTTDNSTAVQDS
NGESWNTGLSINKGESAYINANVRYYNTGTAPMYKVTPTTNLVLDGDTLSTIKAQENQIGNN
LSPGDTYPKKGLSPLALNTMDQFSSRLIPINYDQLKKLDAGKQIKLETTQVSGNFGTKNSSG
QIVTEGNSWSDYISQIDSISASIILDTENESYERRVTAKNLQDPEDKTPELTIGEAIEKAFGATK
KDGLLYFNDIPIDESCVELIFDDNTANKIKDSLKTLSDKKIYNVKLERGMNILIKTPTYFTNFDD
YNNYPSTWSNVNTTNQDGLQGSANKLNGETKIKIPMSELKPYKRYVFSGYSKDPLTSNSIIV
KIKAKEEKTDYLVPEQGYTKFSYEFETTEKDSSNIEITLIGSGTTYLDNLSITELNSTPEILDEP
EVKIPTDQEIMDAHKIYFADLNFNPSTGNTYINGMYFAPTQTNKEALDYIQKYRVEATLQYSG
FKDIGTKDKEMRNYLGDPNQPKTNYVNLRSYFTGGENIMTYKKLRIYAITPDDRELLVLSVD
GG
**SEQ ID NO: 57 C155 without histag** 
MVYNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPTNFDDYNNYPSTWSNVNTTN
QDGLQGSANKLNGETKIKIPMSELKPYKRYVFSGYSKDPLTSNSIIVKIKAKEEKTDYLVPEQ
GYTKFSYEFETTEKDSSNIEITLIGSGTTYLDNLSITELNSTPEILDEPEVKIPTDQEIMDAHKIY
FADLNFNPSTGNTYINGMYFAPTQTNKEALDYIQKYRVEATLQYSGFKDIGTKDKEMRNYL
GDPNQPKTNYVNLRSYFTGGENIMTYKKLRIYAITPDDRELLVLSVDGG
**SEQ ID NO: 58 C156 without histag** 
MVYNTTYKAPIERPEDFLKDKEKAKEWERKEAERIEQKLERSEKEALESYKKDSVEISKYSQ
TRNYFDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGQYEVLLNHGSKFKINKIDSYKDGTITKLIVDATLIPGGGGGGNTTNQDGLQGSAN
KLNGETKIKIPMSELKPYKRYVFSGYSKDPLTSNSIIVKIKAKEEKTDYLVPEQGYTKFSYEFE
TTEKDSSNIEITLIGSGTTYLDNLSITELNSTPEILDEPEVKIPTDQEIMDAHKIYFADLNFNPST
GNTYINGMYFAPTQTNKEALDYIQKYRVEATLQYSGFKDIGTKDKEMRNYLGDPNQPKTNY
VNLRSYFTGGENIMTYKKLRIYAITPDDRELLVLSVDGG
**SEQ ID NO: 59: C49 without histag** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVS
**SEQ ID NO: 60: C50 without histag** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSS
**SEQ ID NO: 61: CDTa N terminal domain (residue 44 to residue 240) polypeptide sequence without histag.** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKI
**SEQ ID NO: 62: C67 without histag** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGqYqVLLNHGSKFKINKIDSYKDGTITKLIVDATLIP
**SEQ ID NO: 63: C69 without histag** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGQYQVLLNHGSKFKINKIDSYKDGTITKLIVDATLIP
**SEQ ID NO: 64: C107 without histag** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGEYEVLLNHGSKFKINKIDSYKDGTITKLIVDATLIP
**SEQ ID NO: 65: C108 without histag** 
MVCNTTYKAPIERPEDFLKDKEKAKEWERKEAERIEQKLERSEKEALESYKKDSVEISKYSQ
TRNYFDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGEYQVLLNHGSKFKINKIDSYKDGTITKLIVDATLIP
**SEQ ID NO: 66: C149 without histag** 
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQENQIGNNLSPGDTYPKKGLSPLALNTMDQRSSRLIPINYDQLKKLDAGK
QIKLETTQVSGNFGTKNSSGQIVTEGNSWSDYISQIDSISASIILDTENESYERRVTAKNLQD
PEDKTPELTIGEAIEKAFGATKKDGLLYFNDIPIDESCVELIFDDNTANKIKDSLKTLSDKKIYN
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVD
**SEQ ID NO: 67: C152 without histag** 
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
 
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQANQIGNNLSPGDTYPKKGLSPLALNTMAQFSSRLIPINYDQLKKLDAGK
QIKLETTQVSGNFGTKNSSGQIVTEGNSWSDYISQIDSISASIILDTENESYERRVTAKNLQD
PEDKTPELTIGEAIEKAFGATKKDGLLYFNDIPIDESCVELIFDDNTANKIKDSLKTLSDKKIYN
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVD
**SEQ ID NO: 68: C52 without histag** 
MTNFDDYNNYPSTWSNVNTTNQDGLQGSANKLNGETKIKIPMSELKPYKRYVFSGYSKDP
LTSNSIIVKIKAKEEKTDYLVPEQGYTKFSYEFETTEKDSSNIEITLIGSGTTYLDNLSITELNST
PEILDEPEVKIPTDQEIMDAHKIYFADLNFNPSTGNTYINGMYFAPTQTNKEALDYIQKYRVE
ATLQYSGFKDIGTKDKEMRNYLGDPNQPKTNYVNLRSYFTGGENIMTYKKLRIYAITPDDRE
LLVLSVDGG
**SEQ ID NO: 69: C53 without histag** 
MNTTNQDGLQGSANKLNGETKIKIPMSELKPYKRYVFSGYSKDPLTSNSIIVKIKAKEEKTDY
LVPEQGYTKFSYEFETTEKDSSNIEITLIGSGTTYLDNLSITELNSTPEILDEPEVKIPTDQEIM
DAHKIYFADLNFNPSTGNTYINGMYFAPTQTNKEALDYIQKYRVEATLQYSGFKDIGTKDKE
MRNYLGDPNQPKTNYVNLRSYFTGGENIMTYKKLRIYAITPDDRELLVLSVDGG
**SEQ ID NO: 70: CDTa C34 construct polypeptide sequence without histag** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGEYEVLLNHGSKFKINKIDSYKDGTITKLIVDATLIP
**SEQ ID NO: 71: CDTb C118 construct. CDTb without signal peptide, without prodomain, without CDTa-binding domain and without Receptor-binding domain, without histag** 
MAYPIVGVGMEKLIISTNEHASTDQGKTVSRATTNSKTESNTAGVSVNVGYQNGFTANVTT
NYSHTTDNSTAVQDSNGESWNTGLSINKGESAYINANVRYYNTGTAPMYKVTPTTNLVLDG
DTLSTIKAQENQIGNNLSPGDTYPKKGLSPLALNTMDQFSSRLIPINYDQLKKLDAGKQIKLE
TTQVSGNFGTKNSSGQIVTEGNSWSDYISQIDSISASIILDTENESYERRVTAKNLQDPEDKT
PELTIGEAIEKAFGATKKDGLLYFNDIPIDESCVELIFDDNTANKIKDSLKTLSDKKIYNVKLER
GMNILIKTPGG
**SEQ ID NO: 72: CDTb C46 construct. CDTb' (minus signal peptide) polypeptide sequence** without histag 
MEIVNEDILPNNGLMGYYFTDEHFKDLKLMAPIKDGNLKFEEKKVDKLLDKDKSDVKSIRWT
GRIIPSKDGEYTLSTDRDDVLMQVNTESTISNTLKVNMKKGKEYKVRIELQDKNLGSIDNLSS
PNLYWELDGMKKIIPEENLFLRDYSNIEKDDPFIPNNNFFDPKLMSDWEDEDLDTDNDNIPD
SYERNGYTIKDLIAVKWEDSFAEQGYKKYVSNYLESNTAGDPYTDYEKASGSFDKAIKTEAR
DPLVAAYPIVGVGMEKLIISTNEHASTDQGKTVSRATTNSKTESNTAGVSVNVGYQNGFTA
NVTTNYSHTTDNSTAVQDSNGESWNTGLSINKGESAYINANVRYYNTGTAPMYKVTPTTNL
VLDGDTLSTIKAQENQIGNNLSPGDTYPKKGLSPLALNTMDQFSSRLIPINYDQLKKLDAGK
QIKLETTQVSGNFGTKNSSGQIVTEGNSWSDYISQIDSISASIILDTENESYERRVTAKNLQD
PEDKTPELTIGEAIEKAFGATKKDGLLYFNDIPIDESCVELIFDDNTANKIKDSLKTLSDKKIYN
VKLERGMNILIKTPTYFTNFDDYNNYPSTWSNVNTTNQDGLQGSANKLNGETKIKIPMSELK
PYKRYVFSGYSKDPLTSNSIIVKIKAKEEKTDYLVPEQGYTKFSYEFETTEKDSSNIEITLIGS
GTTYLDNLSITELNSTPEILDEPEVKIPTDQEIMDAHKIYFADLNFNPSTGNTYINGMYFAPTQ
TNKEALDYIQKYRVEATLQYSGFKDIGTKDKEMRNYLGDPNQPKTNYVNLRSYFTGGENIM
TYKKLRIYAITPDDRELLVLSVD
**SEQ ID NO: 73: C44 construct. CDTa mutation E428Q polypeptide sequence, without histag.** 
MVCNTTYKAPIERPEDFLKDKEKAKEWERKEAERIEQKLERSEKEALESYKKDSVEISKYSQ
TRNYFDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGqYEVLLNHGSKFKINKIDSYKDGTITKLIVDATLIP
**SEQ ID NO: 74: C54 construct. CDTa mutation E430Q polypeptide sequence, without histag.** 
MVCNTTYKAPIERPEDFLKDKEKAKEWERKEAERIEQKLERSEKEALESYKKDSVEISKYSQ
TRNYFDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYRRSGPQEFGLT
LTSPEYDFNKLENIDAFKSKWEGQALSYPNFISTSIGSVNMSAFAKRKIVLRITIPKGSPGAYL
SAIPGYAGEYqVLLNHGSKFKINKIDSYKDGTITKLIVDATLIP
**SEQ ID NO: 75: C68 without histag** 
MVCNTTYKAPIERPEDFLKDKEKAKEWERKEAERIEQKLERSEKEALESYKKDSVEISKYSQ
TRNYFYDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFISTSIGSVNMSAFAKAKIVLRITIPKGSPGAYL
SAIPGYAGQYQVLLNHGSKFKINKIDSYKDGTITKLIVDATLIP
**SEQ ID NO: 76: Amino acid sequence of CDTa without signal peptide, with six mutations (R345A, Q350A, N385A, R402A, S388F, E428Q, aa 44-463) (C110 without histag)**

MVCNTTYKAPIERPEDFLKDKEKAKEWERKEAERIEQKLERSEKEALESYKKDSVEISKYSQ
TRNYFDYQIEANSREKEYKELRNAISKNKIDKPMYVYYFESPEKFAFNKVIRTENQNEISLE
KFNEFKETIQNKLFKQDGFKDISLYEPGKGDEKPTPLLMHLKLPRNTGMLPYTNTNNVSTLIE
QGYSIKIDKIVRIVIDGKHYIKAEASVVSSLDFKDDVSKGDSWGKANYNDWSNKLTPNELAD
VNDYMRGGYTAINNYLISNGPVNNPNPELDSKITNIENALKREPIPTNLTVYARSGPAEFGLT
LTSPEYDFNKLENIDAFKSKWEGQALSYPAFIFTSIGSVNMSAFAKAKIVLRITIPKGSPGAYL
SAIPGYAGQYEVLLNHGSKFKINKIDSYKDGTITKLIVDATLIP

## Claims

1. An immunogenic composition comprising a fusion protein comprising (i) a full length CDTa protein and (ii) a CDTb protein wherein the CDTb protein is a full length CDTb protein with the prodomain removed.

2. The immunogenic composition of claim 1 wherein the fusion protein comprising a full length CDTa protein and a CDTb protein suitably is or comprises
(i) SEQ ID NO: 14; or
(ii) a variant fusion protein having at least 80%, 85%, 88%, 90%, 92%, 95%, 98%, 99%, 100% sequence identity to SEQ ID NO: 14; or
(iii) a fragment having at least 30, 50, 80, 100, 120, 150, 200, 250, 300, 350 or 400 contiguous amino acids of SEQ ID NO: 14.

3. The immunogenic composition of any preceding claim wherein the composition elicits antibodies that neutralize CDTa or CDTb or both.

4. The immunogenic composition of any preceding claim wherein the composition elicits antibodies that neutralize binary toxin.

5. The immunogenic composition of any preceding claim wherein the immunogenic composition further comprises an isolated *Clostridium difficile* toxin A protein and/or an isolated *C*. *difficile* toxin B protein.

6. The immunogenic composition of claim 5 wherein the immunogenic composition comprises an isolated *Clostridium difficile* toxin A protein and an isolated *C*. *difficile* toxin B protein wherein the isolated *Clostridium difficile* toxin A protein and the isolated *C*. *difficile* toxin B protein form a fusion protein.

7. The immunogenic composition of any preceding claim wherein the immunogenic composition further comprises an adjuvant.

8. A vaccine comprising the immunogenic composition of any one of the preceding claims and a pharmaceutically acceptable excipient.

9. The immunogenic composition of any one of claims 1-7 or the vaccine of claim 8 for use in the treatment or prevention of *C*. *difficile* disease.

## Patentansprüche

1. Immunogene Zusammensetzung, welche ein Fusionsprotein umfasst, umfassend (i) ein CDTa-Protein voller Länge und (ii) ein CDTb-Protein, wobei das CDTb-Protein ein CDTb-Protein voller Länge ist, in dem die Prodomäne entfernt ist.

2. Immunogene Zusammensetzung gemäß Anspruch 1, wobei das Fusionsprotein, welches ein CDTa-Protein voller Länge und ein CDTb-Protein umfasst, zweckmäßigerweise ist oder umfasst:
(i) SEQ ID NO: 14; oder
(ii) ein variantes Fusionsprotein mit wenigstens 80%, 85%, 88%, 90%, 92%, 95%, 98%, 99%, 100% Sequenzidentität zu SEQ ID NO: 14; oder
(iii) ein Fragment mit wenigstens 30, 50, 80, 100, 120, 150, 200, 250, 300, 350 oder 400 zusammenhängenden Aminosäuren von SEQ ID NO: 14.

3. Immunogene Zusammensetzung gemäß irgendeinem vorangegangenen Anspruch, wobei die Zusammensetzung Antikörper auslöst, die CDTa oder CDTb oder beide neutralisieren.

4. Immunogene Zusammensetzung gemäß irgendeinem vorangegangenen Anspruch, wobei die Zusammensetzung Antikörper auslöst, die binäres Toxin neutralisieren.

5. Immunogene Zusammensetzung gemäß irgendeinem vorangegangenen Anspruch, wobei die immunogene Zusammensetzung weiterhin ein isoliertes *Clostridium difficile* Toxin A-Protein und/oder ein isoliertes *C*. *difficile* Toxin B-Protein umfasst.

6. Immunogene Zusammensetzung gemäß Anspruch 5, wobei die immunogene Zusammensetzung ein isoliertes *Clostridium difficile* Toxin A-Protein und ein isoliertes *C*. *difficile* Toxin B-Protein umfasst, wobei das isolierte *Clostridium difficile* Toxin A-Protein und das isolierte *C*. *difficile* Toxin B-Protein ein Fusionsprotein bilden.

7. Immunogene Zusammensetzung gemäß irgendeinem vorangegangenen Anspruch, wobei die immunogene Zusammensetzung weiterhin ein Adjuvans umfasst.

8. Impfstoff, welcher die immunogene Zusammensetzung gemäß irgendeinem der vorangegangenen Ansprüche und einen pharmazeutisch akzeptablen Hilfsstoff umfasst.

9. Immunogene Zusammensetzung gemäß irgendeinem der Ansprüche 1-7 oder Impfstoff gemäß Anspruch 8 zur Verwendung bei der Behandlung oder Prävention von C. *difficile-Erkrankung.*

## Revendications

1. Composition immunogène comprenant une protéine de fusion comprenant (i) une protéine CDTa pleine longueur et (ii) une protéine CDTb, dans laquelle la protéine CDTb est une protéine CDTb pleine longueur avec le prodomaine enlevé.

2. Composition immunogène selon la revendication 1, dans laquelle la protéine de fusion comprenant une protéine CDTa pleine longueur et une protéine CDTb est ou comprend convenablement
(i) SEQ ID N° 14 ; ou
(ii) une protéine de fusion variante ayant au moins 80 %, 85 %, 88 %, 90 %, 92 %, 95 %, 98 %, 99 %, 100 % d'identité de séquence avec SEQ ID N° 14 ; ou
(iii) un fragment ayant au moins 30, 50, 80, 100, 120, 150, 200, 250, 300, 350 ou 400 acides aminés contigus de SEQ ID N° 14.

3. Composition immunogène selon une quelconque revendication précédente, dans laquelle la composition suscite des anticorps qui neutralisent CDTa ou CDTb ou les deux.

4. Composition immunogène selon une quelconque revendication précédente, dans laquelle la composition suscite des anticorps qui neutralisent une toxine binaire.

5. Composition immunogène selon une quelconque revendication précédente, dans laquelle la composition immunogène comprend en outre une protéine de toxine A de *Clostridium difficile* isolée et/ou une protéine de toxine B de *C*. *difficile* isolée.

6. Composition immunogène selon la revendication 5, dans laquelle la composition immunogène comprend une protéine de toxine A de *Clostridium difficile* isolée et une protéine de toxine B de *C*. *difficile* isolée, dans laquelle la protéine de toxine A de *Clostridium difficile* isolée et la protéine de toxine B de *C*. *difficile* isolée forment une protéine de fusion.

7. Composition immunogène selon une quelconque revendication précédente, dans laquelle la composition immunogène comprend en outre un adjuvant.

8. Vaccin comprenant la composition immunogène de l'une quelconque des revendications précédentes et un excipient pharmaceutiquement acceptable.

9. Composition immunogène selon l'une quelconque des revendications 1 à 7 ou vaccin selon la revendication 8, pour une utilisation dans le traitement ou la prévention d'une maladie à *C*. *difficile.*
